(19) Europäisches Patentamt
European Patent Office
Office européen des brevets

(11) EP 4 431 521 A1

(12) EUROPEAN PATENT APPLICATION
published in accordance with Art. 153(4) EPC

(43) Date of publication:
**18.09.2024 Bulletin 2024/38**

(21) Application number: **22900495.7**

(22) Date of filing: **30.11.2022**

(51) International Patent Classification (IPC):
***C07K 7/02*** *(2006.01)* ***A61K 47/68*** *(2017.01)*
***A61P 35/00*** *(2006.01)*

(52) Cooperative Patent Classification (CPC):
**A61K 47/65; A61K 47/68; A61P 35/00; C07K 7/02; C07K 7/06**

(86) International application number:
**PCT/CN2022/135216**

(87) International publication number:
**WO 2023/098691 (08.06.2023 Gazette 2023/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.12.2021 CN 202111452890**

(71) Applicant: **Shanghai Institute of Biological Products Co., Ltd.**
**Shanghai 200051 (CN)**

(72) Inventors:
- **QU, Aidong**
  **Shanghai (CN)**
- **LIANG, Hongyuan**
  **Shanghai (CN)**
- **SHEN, Wenyan**
  **Shanghai (CN)**
- **ZHU, Jingye**
  **Shanghai (CN)**
- **QIU, Jianhua**
  **Shanghai (CN)**
- **ZHOU, Xu**
  **Shanghai (CN)**

(74) Representative: **HGF**
**HGF Limited**
**1 City Walk**
**Leeds LS11 9DX (GB)**

Remarks:
The complete document including Reference Table(s) and the Sequence Listing(s) can be downloaded from the EPO website

# (54) ANTIBODY-DRUG CONJUGATE AND USE THEREOF

(57) A linker, comprising an amino acid unit (RL). The RL comprises -Val-Ala-Q-Gly-Phe-Gly-, wherein Q is a bond, a single amino acid or a dipeptide. By optimizing a polypeptide sequence, the purpose of more effectively releasing small molecule drugs in tumor cells by ADC drugs is achieved. In addition, the present invention further relates to linker drug containing the above-mentioned linker, a conjugate, and the use of the linker drug and the conjugate.

EP 4 431 521 A1

## Description

TECHNICAL FIELD

The present application relates to the field of biopharmaceuticals, in particular to a linker and an antibody-drug conjugate (ADC) comprising the linker.

BACKGROUND

Many cytotoxic drug molecules cannot be used in cancer therapy because they cannot selectively kill cancer cells. Therefore, antibody-drug conjugates (ADCs) have been developed as novel cancer therapeutic drugs. An ADC generally consists of three parts: an antibody, a linker, and a drug, wherein the antibody is conjugated to the drug through the linker. The action of mechanism of an antibody-drug complex is that the antibody firstly recognizes an antigen specifically expressed or over-expressed on tumor cells by the high recognition capability of the antibody to the corresponding antigen and binds to the antigen, and when the formed complex enters tumor cells by endocytosis, the conjugated cytotoxic drug molecule is also brought into the tumor cells. In the tumor cells, the antibody is degraded or the linker is broken down to release the cytotoxic drug molecule, thereby killing the target tumor cells and achieving a selective toxic killing effect.

The linker used in ADC needs to meet several requirements. While circulating in human plasma, the linker needs to be stable to prevent early release of drug. Upon internalization in tumor cells, a cleavable linker can be cleaved under certain condition to release a drug, and for a non-cleavable linker, the drug moiety is released in an active form comprising the drug, the linker, and amino acid residues derived from a proteolytic degradation ligand.

The main action of mechanism of a cleavable antibody-drug conjugate is that the antibody targets and binds to a specific cell surface antigen, and the cleavable antibody-drug conjugate enters cell endoplasmic reticulum system by endocytosis after the binding, and releases a small molecule drug via the cleavage by protease in endosome or lysosome, thereby achieving targeted killing of tumor cells. For solid tumors, some tumors do not express or underexpress target antigens due to tumor heterogeneity, and small molecules with membrane-penetrating activity can kill neighboring tumor cells after being released, which is called bystander effect.

Cathepsin B is one of the important components in a series of proteases within endosomes or lysosomes. Cathepsin B is a member of the papain-like family of cysteine proteases. Cathepsin B is homologous to papain and has an acidic pH optimum. Since cathepsin B participates in dissolution of extracellular matrix and further influences infiltration and metastasis of tumors, it is found to have relatively high expression in cells of various tumors, such as gastric cancer, lung cancer, intestinal cancer, breast cancer, prostate cancer, and kidney cancer. Therefore, ADCs entering tumor cells can be quickly cleaved by very abundant intracellular cathepsin B, and small molecules are quickly released to kill cells.

Non-optimal properties may lead to a decrease in ADC potency, insufficient immunospecificity of conjugates, and increased toxicity due to non-specific release of drugs from conjugates. Therefore, it is necessary to develop new linker technologies and conjugates that can be used in targeted therapies.

SUMMARY

The present application provides a linker comprising a self-degradation spacer, namely an amino acid unit, wherein the amino acid unit comprises GFG as a preferred substrate component for cathepsin B. A VA sequence is added at the N-terminus of the GFG fragment to enhance the binding to a cleavage active region of cathepsin B, and meanwhile, considering the influence of polypeptide length on the activity of an enzyme on a substrate, one S and two S can be added separately between VA and GF, so that three polypeptide sequences, namely VAGFG, VASGFG or VASSGFG, are formed. Through polypeptide sequence optimization, a more effective release of a small molecule drug from an ADC in tumor cells can be achieved.

The present application also provides a linker-drug or conjugate comprising the linker described above, and use thereof.

In one aspect, the present application provides a linker comprising an amino acid unit (RL), wherein RL comprises: -Val-Ala-Q-Gly-Phe-Gly-, wherein Q is a bond, a single amino acid, or a dipeptide.

In certain embodiments, RL is cleavable by an enzyme.

In certain embodiments, RL is cleavable by cathepsin B.

In certain embodiments, Q is a bond.

In certain embodiments, Q is -Ser- or -Ser-Ser-.

In certain embodiments, RL comprises -Val-Ala-Gly-Phe-Gly- (VAGFG), -Val-Ala-Ser-Gly-Phe-Gly- (VASGFG), or -Val-Ala-Ser-Ser-Gly-Phe-Gly- (VASSGFG).

In certain embodiments, the linker is selected from the following structures: -Z-A-S*-RL- and -Z-AS*-RL-Y-;

wherein, Z is an extender unit, the A is a bond or an adapter unit; S* is a bond or a partitioning agent; Y is a spacer unit.

In certain embodiments, Z comprises the following structure: -(succinimid-3-yl-N)-, $-CH_2-C(=O)-NH-$ or -C(=O)-; wherein, -(succinimid-3-yl-N)- has the following structure:

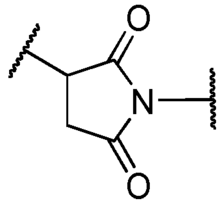
,

wherein the wavy line at position 3 of the structure represents an attachment position to a ligand unit, the wavy line at the N atom of position 1 represents an attachment position to A.

In certain embodiments, A is selected from the following structures:

$-C_1-C_{10}$ alkylene-, $-C_1-C_{10}$ heteroalkylene-, $-C_3-C_8$ carbocyclyl-, $-O-(C_1-C_8$ alkylene)-, -arylene-, $-C_1-C_{10}$ alkylene-arylene-, -arylene-$C_1-C_{10}$ alkylene-, $-C_1-C_{10}$ alkylene-($C_3-C_8$ carbocyclyl)-, -($C_3-C_8$ carbocyclyl)-$C_1-C_{10}$ alkylene-, $-C_3-C_8$ heterocyclyl-, $-C_1-C_{10}$ alkylene-($C_3-C_8$ heterocyclyl)-, -($C_3-C_8$ heterocyclyl)-$C_1-C_{10}$ alkylene-, $-C_1-C_{10}$ alkylene-C(=O)-, $-C_1-C_{10}$ heteroalkylene-C(=O)-, $-C_3-C_8$ carbocyclyl-C(=O)-, $-O-(C_1-C_8$ alkylene)-C(=O)-, -arylene-C(=O)-, $-C_1-C_{10}$ alkylene-arylene-C(=O)-, -arylene-$C_1-C_{10}$ alkylene-C(=O)-, $-C_1-C_{10}$ alkylene-($C_3-C_8$ carbocyclyl)-C(=O)-, -($C_3-C_8$ carbocyclyl)-$C_1-C_{10}$ alkylene-C(=O)-, $-C_3-C_8$ heterocyclyl-C(=O)-, $-C_1-C_{10}$ alkylene-($C_3-C_8$ heterocyclyl)-C(=O)-, -($C_3-C_8$ heterocyclyl)-$C_1-C_{10}$ alkylene-C(=O)-, $-C_1-C_{10}$ alkylene-NH-, $-C_1-C_{10}$ heteroalkylene-NH-, $-C_3-C_8$ carbocyclyl-NH-, $-O-(C_1-C_8$ alkylene)-NH-, -arylene-NH-, $-C_1-C_{10}$ alkylene-arylene-NH-, -arylene-$C_1-C_{10}$ alkylene-NH-, $-C_1-C_{10}$ alkylene-($C_3-C_8$ carbocyclyl)-NH-, - ($C_3-C_8$ carbocyclyl)-$C_1-C_{10}$ alkylene-NH-, $-C_3-C_8$ heterocyclyl-NH-, $-C_1-C_{10}$ alkylene-($C_3-C_8$ heterocyclyl)-NH-, -($C_3-C_8$ heterocyclyl)-$C_1-C_{10}$ alkylene-NH-, $-C_1-C_{10}$ alkylene-S-, $-C_1-C_{10}$ heteroalkylene-S-, $-C_3-C_8$ carbocyclyl-S-, $-O-(C_1-C_8$ alkylene)-S-, -arylene-S-, $-C_1-C_{10}$ alkylene-arylene-S-, -arylene-$C_1-C_{10}$ alkylene-S-, $-C_1-C_{10}$ alkylene-($C_3-C_8$ carbocyclyl)-S-, -($C_3-C_8$ carbocyclyl)-$C_1-C_{10}$ alkylene-S-, $-C_3-C_8$ heterocyclyl-S-, $-C_1-C_{10}$ alkylene-($C_3-C_8$ heterocyclyl)-S-, or -($C_3-C_8$ heterocyclyl)-$C_1-C_{10}$ alkylene-S-;

wherein, A is optionally substituted with a basic unit (BU), wherein the basic unit is $-(CH_2)_xNH_2$, - $(CH_2)_xNHR^a$, or $-(CH_2)_xNR^a{}_2$; wherein x is any integer from 1 to 4; each $R^a$ is independently selected from $C_1$-$C_6$ alkyl and $C_1$-$C_6$ haloalkyl, or two $R^a$ groups, together with nitrogen attached thereto, form a 4- to 6-membered heterocycloalkyl ring, or an azetidinyl, pyrrolidinyl or piperidinyl group.

In certain embodiments, A is selected from the following structure: $-(C_1-C_{10})$alkylene-C(=O)-, wherein the alkylene moiety of A is optionally substituted with the basic unit (BU).

In certain embodiments, the linker may comprise the following structure:

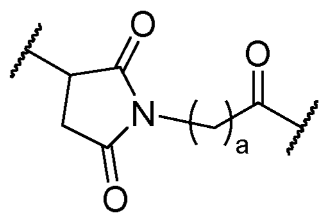
,

wherein a is any integer from 1 to 8.

In certain embodiments, a is any integer from 2 to 5.

In certain embodiments, Y comprises a structure represented by $-NH-(CH_2)n^1$-La-Lb-Lc-, wherein La represents -C(=O)-NH-, $-NR^1-(CH_2)n^2-$, -O-, or a single bond, $R^1$ represents H, $C_1-C_6$ alkyl, $C_1-C_6$ alkylene-carboxyl, or $C_1-C_6$ alkylene-hydroxyl;

Lb represents $-CR^2(-R^3)-$ or a single bond, wherein $R^2$ and $R^3$ each independently represents H, $-NH_2$, $C_1-C_6$ alkylene, $C_1-C_6$ alkylene-amino, $C_1-C_6$ alkylene-carboxyl, or $C_1-C_6$ alkylene-hydroxyl, and $R^2$ and $R^3$ are not both $-NH_2$;

$n^1$ represents an integer from 0 to 6;

Lc represents $-CH_2-$ or -C(=O)-.

In certain embodiments, Y comprises NH-$(CH_2)n^1$-O-$(CH_2)n^2$-C(=O)-, wherein $n^1$ or $n^2$ is each independently any integer from 0 to 5.
In certain embodiments, Y comprises -NH-$CH_2$-C(=O)-, -NH-$CH_2CH_2$-C(=O)-, -NH-$(CH_2)_3$-C(=O)-, -NH-$(CH_2)_4$-C(=O)-, -NH-$(CH_2)_5$-C(=O)-, -NH-$CH_2$-O-$CH_2$-C(=O)-, or -NH-$(CH_2)_2$-O-$CH_2$-C(=O)-.
In certain embodiments, S* is a bond, and the linker is -Z-A-RL- or -Z-A-RL-Y-.
In certain embodiments, S* is a partitioning agent, and the linker is -Z-A-S*-RL- or -Z-A-S*-RL-Y-.
In certain embodiments, S* comprises a PEG unit.
In certain embodiments, S* has the following formula:

-NH-$(CH_2CH_2O)_b$-$CH_2CH_2$C(=O)-;
-NH-$(CH_2CH_2O)_b$-$CH_2CH_2$C(=O)NH-$(CH_2CH_2O)$-$CH_2CH_2$C(=O)-; or
-NH-$(CH_2CH_2O)_b$-$CH_2CH_2$NH-$(CH_2CH_2O)$-$CH_2CH_2$C(=O)-;

wherein S* is connected to A on the left side and connected to RL on the right side, and b is any integer from 2 to 20.
In certain embodiments, b is 2, 4, 8, or 12.
In certain embodiments, the linker is selected from the following structures:

-(succinimid-3-yl-N)-$CH_2CH_2$-C(=O)-RL-NH-$CH_2CH_2$-C(=O)-;
-(succinimid-3-yl-N)-$CH_2CH_2$-C(=O)-RL-NH-$CH_2CH_2CH_2$-C(=O)-;
-(succinimid-3-yl-N)-$CH_2CH_2CH_2CH_2CH_2$-C(=O)-RL-NH-$CH_2CH_2$-C(=O)-;
-(succinimid-3-yl-N)-$CH_2CH_2CH_2CH_2CH_2$-C(=O)-RL-NH-$CH_2CH_2CH_2$-C(=O)-;
-(succinimid-3-yl-N)-$CH_2CH_2CH_2CH_2CH_2$-C(=O)-RL-NH-$CH_2CH_2CH_2CH_2CH_2$-C(=O)-;
-(succinimid-3-yl-N)-$CH_2CH_2CH_2CH_2CH_2$-C(=O)-RL-NH-$CH_2$-O-$CH_2$-C(=O)-;
-(succinimid-3-yl-N)-$CH_2CH_2CH_2CH_2CH_2$-C(=O)-RL-NH-$CH_2CH_2$-O-$CH_2$-C(=O)-;
-(succinimid-3-yl-N)-$CH_2CH_2$-C(=O)-NH-$CH_2CH_2O$-$CH_2CH_2O$-$CH_2CH_2$-C(=O)-RL-NH-$CH_2CH_2CH_2$-C(=O)-;
-(succinimid-3-yl-N)-$CH_2CH_2$-C(=O)-NH-$CH_2CH_2O$-$CH_2CH_2O$-$CH_2CH_2$-C(=O)-RL-NH-$CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2$-C(=O)-;
-$CH_2$-C(=O)-NH-$CH_2CH_2$-C(=O)-RL-NH-$CH_2CH_2CH_2$-C(=O)-;
-C(=O)-$CH_2CH_2CH_2CH_2CH_2CH_2$-C(=O)-RL-NH-$CH_2CH_2CH_2$-C(=O)-;
wherein,
-(succinimid-3-yl-N)- has the following structure:

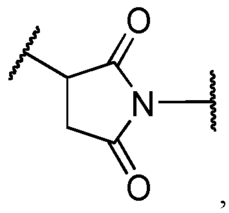
,

wherein the wavy line at position 3 of the structure represents an attachment position to a ligand unit, the wavy line at the N atom of position 1 represents an attachment position to A;
-RL- represents the following structure: -Val-Ala-Gly-Phe-Gly- (VAGFG), -Val-Ala-Ser-Gly-Phe-Gly- (VASGFG), or -Val-Ala-Ser-Ser-Gly-Phe-Gly- (VASSGFG).

In certain embodiments, the linker-drug is selected from the following structures:

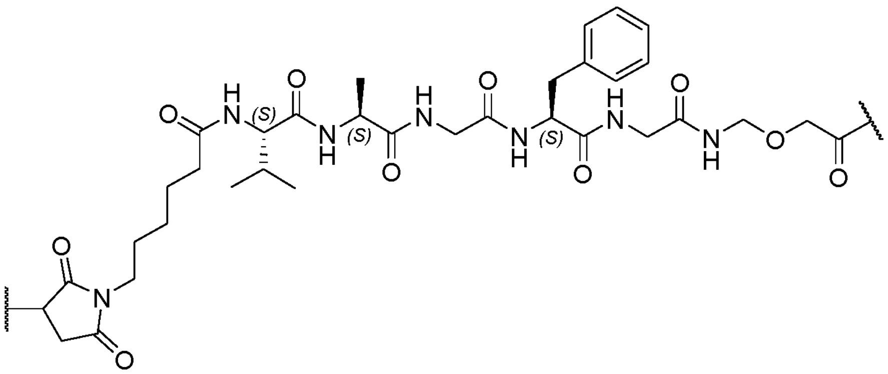

,

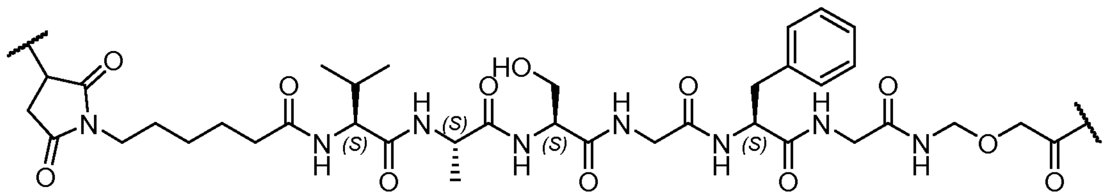

,

and

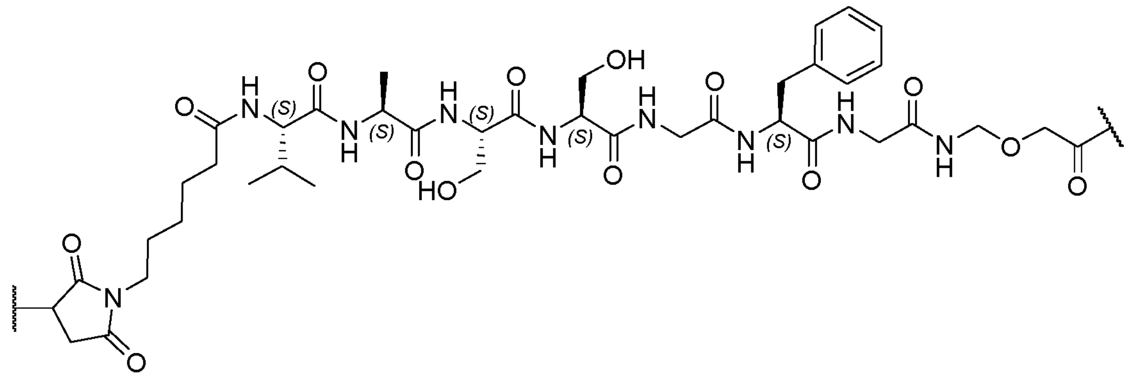

.

In another aspect, the present application provides a linker-drug comprising the linker as described herein.

In certain embodiments, the linker-drug is selected from the following structures: Z'-A-S*-RL-D and -Z'-A-S*-RL-Y-D; wherein, Z' is an extender unit precursor; A is a bond or an adapter unit; S* is a bond or a partitioning agent; Y is a spacer unit; D is a drug unit.

In certain embodiments, the extender unit precursor Z' is selected from (maleimid-N-yl)-, (pyrrolidine-2,5-dione-N-yl)-, M-$CH_2$-C(=O)-NH-, and HS-$(CH_2)_{1-5}$-C(=O)-, wherein M is halogen. In certain embodiments, the drug unit comprises a cytotoxic agent.

In certain embodiments, the drug unit comprises: amanitins, anthracyclines, auristatins, baccatins, calicheamicins, camptothecins, cemadotins, colchicines, colcimids, combretastatins, cryptophycins, discodermolides, duocarmycins, docetaxel, doxorubicin, duocarmycins, echinomycins, eleutherobins, epothilones, estramustines, lexitropsins, maytansines, maytansinoids, methotrexate, netropsins, pyrrolo[2,1-c][1,4]benzodiazepines (PBDs), puromycins, rhizoxins, SN-38, taxanes, tubulysins, vinca alkaloids, or derivatives thereof.

In certain embodiments, the drug unit comprises a DNA topoisomerase I inhibitor.

In certain embodiments, the drug unit comprises camptothecin or a derivative thereof.

In certain embodiments, the drug unit has the following structure:

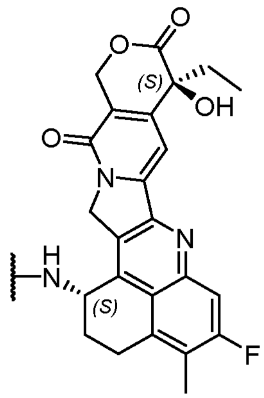

Dxd

wherein the nitrogen atom of the amino group at position 1 is a group at an attachment position.

In certain embodiments, the linker-drug unit has the following structure:

(maleimid-N-yl)-$CH_2CH_2$-C(=O)-RL-NH-$CH_2CH_2$-C(=O)-Dxd;
(maleimid-N-yl)-$CH_2CH_2$-C(=O)-RL-NH-$CH_2CH_2CH_2$-C(=O)-Dxd;
(maleimid-N-yl)-$CH_2CH_2CH_2CH_2CH_2$-C(=O)-RL-NH-$CH_2CH_2$-C(=O)-Dxd;
(maleimid-N-yl)-$CH_2CH_2CH_2CH_2CH_2$-C(=O)-RL-NH-$CH_2CH_2CH_2$-C(=O)-Dxd;
(maleimid-N-yl)-$CH_2CH_2CH_2CH_2CH_2$-C(=O)-RL-NH-$CH_2CH_2CH_2CH_2CH_2$-C(=O)-Dxd;
(maleimid-N-yl)-$CH_2CH_2CH_2CH_2CH_2$-C(=O)-RL-NH-$CH_2$-O-$CH_2$-C(=O)-Dxd;
(maleimid-N-yl)-$CH_2CH_2CH_2CH_2CH_2$-C(=O)-RL-NH-$CH_2CH_2$-O-$CH_2$-C(=O)-Dxd;
(maleimid-N-yl)-$CH_2CH_2$-C(=O)-NH-$CH_2CH_2O$-$CH_2CH_2O$-$CH_2CH_2$-C(=O)-RL-NH-$CH_2CH_2CH_2$-C(=O)-Dxd;
(maleimid-N-yl)-$CH_2CH_2$-C(=O)-NH-$CH_2CH_2O$-$CH_2CH_2O$-$CH_2CH_2$-C(=O)-RL-NH-$CH_2CH_2$-C(=O)-Dxd;
(maleimid-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-Dxd;
(maleimid-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2$-C(=O)-Dxd;
(pyrrolidine-2,5-dione-N-yl)-$CH_2CH_2$-C(=O)-RL-NH-$CH_2CH_2$-C(=O)-Dxd;
(pyrrolidine-2,5-dione-N-yl)-$CH_2CH_2$-C(=O)-RL-NH-$CH_2CH_2CH_2$-C(=O)-Dxd;
(pyrrolidine-2,5-dione-N-yl)-$CH_2CH_2CH_2CH_2CH_2$-C(=O)-RL-NH-$CH_2CH_2$-C(=O)-Dxd;
(pyrrolidine-2,5-dione-N-yl)-$CH_2CH_2CH_2CH_2CH_2$-C(=O)-RL-NH-$CH_2CH_2CH_2$-C(=O)-Dxd;
(pyrrolidine-2,5-dione-N-yl)-$CH_2CH_2CH_2CH_2CH_2$-C(=O)-RL-NH-$CH_2CH_2CH_2CH_2CH_2$-C(=O)-Dxd;
(pyrrolidine-2,5-dione-N-yl)-$CH_2CH_2CH_2CH_2CH_2$-C(=O)-RL-NH-$CH_2$-O-$CH_2$-C(=O)-Dxd;
(pyrrolidine-2,5-dione-N-yl)-$CH_2CH_2CH_2CH_2CH_2$-C(=O)-RL-NH-$CH_2CH_2$-O-$CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2$-C(=O)-RL-NH-$CH_2CH_2CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2$-C(=O)-RL-NH-$CH_2CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2$-C(=O)-Dxd;
M-$CH_2$-C(=O)-NH-$CH_2CH_2$-C(=O)-RL-NH-$CH_2CH_2$-C(=O)-Dxd;
M-$CH_2$-C(=O)-NH-$CH_2CH_2$-C(=O)-RL-NH-$CH_2CH_2CH_2$-C(=O)-Dxd;
M-$CH_2$-C(=O)-NH-$CH_2CH_2$-C(=O)-RL-NH-$CH_2$-O-$CH_2$-C(=O)-Dxd;
M-$CH_2$-C(=O)-NH-$CH_2CH_2$-C(=O)-RL-NH-$CH_2CH_2$-O-$CH_2$-C(=O)-Dxd;
M-$CH_2$-C(=O)-NH-$CH_2CH_2CH_2$-C(=O)-RL-NH-$CH_2CH_2$-C(=O)-Dxd;
M-$CH_2$-C(=O)-NH-$CH_2CH_2CH_2$-C(=O)-RL-NH-$CH_2CH_2CH_2$-C(=O)-Dxd;
M-$CH_2$-C(=O)-NH-$CH_2CH_2CH_2$-C(=O)-RL-NH-$CH_2$-O-$CH_2$-C(=O)-Dxd;
M-$CH_2$-C(=O)-NH-$CH_2CH_2CH_2$-C(=O)-RL-NH-$CH_2CH_2$-O-$CH_2$-C(=O)-Dxd;
M-$CH_2$-C(=O)-NH-$CH_2CH_2CH_2CH_2$-C(=O)-RL-NH-$CH_2CH_2$-C(=O)-Dxd;
M-$CH_2$-C(=O)-NH-$CH_2CH_2CH_2CH_2$-C(=O)-RL-NH-$CH_2CH_2CH_2$-C(=O)-Dxd;
M-$CH_2$-C(=O)-NH-$CH_2CH_2CH_2CH_2$-C(=O)-RL-NH-$CH_2$-O-$CH_2$-C(=O)-Dxd;

$M-CH_2-C(=O)-NH-CH_2CH_2CH_2CH_2-C(=O)-RL-NH-CH_2CH_2-O-CH_2-C(=O)-Dxd$;
$M-CH_2-C(=O)-NH-CH_2CH_2CH_2CH_2CH_2-C(=O)-RL-NH-CH_2CH_2-C(=O)-Dxd$;
$M-CH_2-C(=O)-NH-CH_2CH_2CH_2CH_2CH_2-C(=O)-RL-NH-CH_2CH_2CH_2-C(=O)-Dxd$;
$M-CH_2-C(=O)-NH-CH_2CH_2CH_2CH_2CH_2-C(=O)-RL-NH-CH_2-O-CH_2-C(=O)-Dxd$;
$M-CH_2-C(=O)-NH-CH_2CH_2CH_2CH_2CH_2-C(=O)-RL-NH-CH_2CH_2-O-CH_2-C(=O)-Dxd$;
$HS-CH_2CH_2-C(=O)-RL-NH-CH_2CH_2-C(=O)-Dxd$;
$HS-CH_2CH_2CH_2-C(=O)-RL-NH-CH_2CH_2-C(=O)-Dxd$;
$HS-CH_2CH_2CH_2CH_2-C(=O)-RL-NH-CH_2CH_2-C(=O)-Dxd$;
$HS-CH_2CH_2CH_2CH_2CH_2-C(=O)-RL-NH-CH_2CH_2-C(=O)-Dxd$;
$HS-CH_2CH_2-C(=O)-RL-NH-CH_2CH_2CH_2-C(=O)-Dxd$;
$HS-CH_2CH_2CH_2-C(=O)-RL-NH-CH_2CH_2CH_2-C(=O)-Dxd$;
$HS-CH_2CH_2CH_2CH_2-C(=O)-RL-NH-CH_2CH_2CH_2-C(=O)-Dxd$;
$HS-CH_2CH_2CH_2CH_2CH_2-C(=O)-RL-NH-CH_2CH_2CH_2-C(=O)-Dxd$;
$HS-CH_2CH_2-C(=O)-RL-NH-CH_2-O-CH_2-C(=O)-Dxd$;
$HS-CH_2CH_2CH_2-C(=O)-RL-NH-CH_2-O-CH_2-C(=O)-Dxd$;
$HS-CH_2CH_2CH_2CH_2-C(=O)-RL-NH-CH_2-O-CH_2-C(=O)-Dxd$;
$HS-CH_2CH_2CH_2CH_2CH_2-C(=O)-RL-NH-CH_2-O-CH_2-C(=O)-Dxd$;
$HS-CH_2CH_2-C(=O)-RL-NH-CH_2CH_2-O-CH_2-C(=O)-Dxd$;
$HS-CH_2CH_2CH_2-C(=O)-RL-NH-CH_2CH_2-O-CH_2-C(=O)-Dxd$;
$HS-CH_2CH_2CH_2CH_2-C(=O)-RL-NH-CH_2CH_2-O-CH_2-C(=O)-Dxd$;
$HS-CH_2CH_2CH_2CH_2CH_2-C(=O)-RL-NH-CH_2CH_2-O-CH_2-C(=O)-Dxd$;
$HS-CH_2CH_2-C(=O)-NH-CH_2CH_2O-CH_2CH_2O-CH_2CH_2-C(=O)-RL-NH-CH_2CH_2CH_2-C(=O)-Dxd$;
$HS-CH_2CH_2-C(=O)-NH-CH_2CH_2O-CH_2CH_2O-CH_2CH_2O-CH_2CH_2-C(=O)-RL-NH-CH_2CH_2CH_2-C(=O)-Dxd$; or
$HS-CH_2CH_2-C(=O)-NH-CH_2CH_2O-CH_2CH_2O-CH_2CH_2O-CH_2CH_2O-CH_2CH_2-C(=O)-RL-NH-CH_2CH_2CH_2-C(=O)-Dxd$;

wherein,

(maleimid-N-yl)- has the following structure:

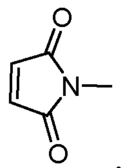
,

wherein the nitrogen atom is a group at an attachment position;
(pyrrolidine-2,5-dione-N-yl)- has the following structure:

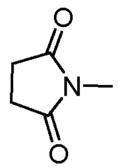
,

wherein the nitrogen atom is a group at an attachment position;
-RL- represents the following structure: -Val-Ala-Gly-Phe-Gly- (VAGFG), -Val-Ala-Ser-Gly-Phe-Gly- (VASGFG), or -Val-Ala-Ser-Ser-Gly-Phe-Gly- (VASSGFG);
Dxd has the following structure:

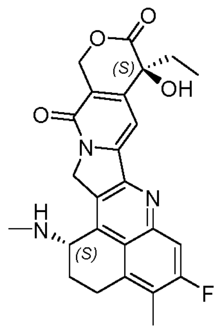,

wherein the nitrogen atom of the amino group at position 1 is a group at an attachment position;
M represents a halogen atom.

In certain embodiments, the linker-drug unit is selected from the following structures:

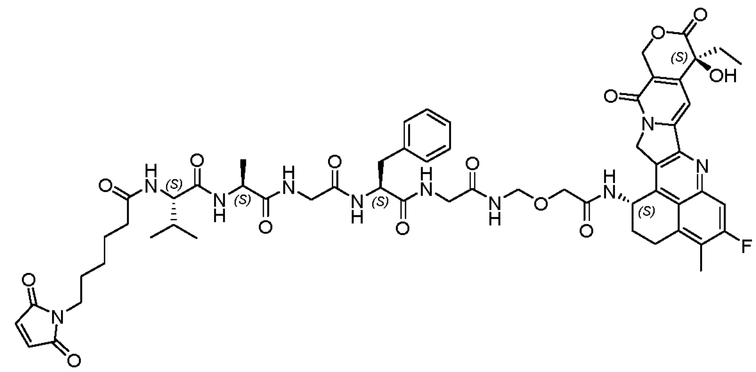,

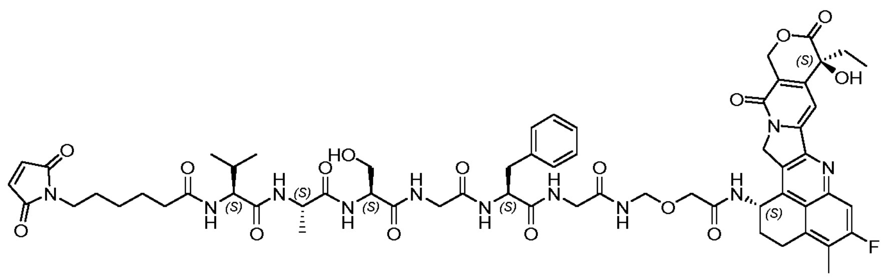,

and

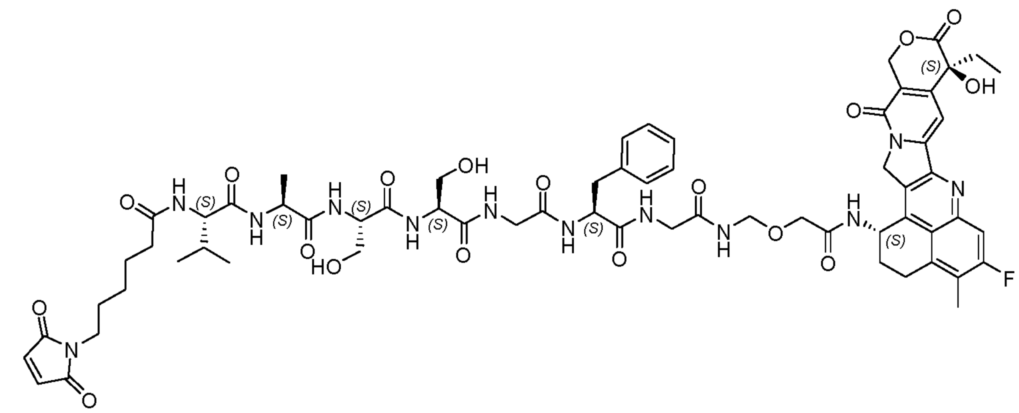.

In another aspect, the present application provides a conjugate comprising the linker as described herein.

In certain embodiments, the conjugate comprises a ligand.

In certain embodiments, the ligand targets a cell surface receptor or a tumor-associated antigen.

In certain embodiments, the cell surface receptor or tumor-associated antigen comprises: HER2, HER3, claudin18.2, folate receptor alpha (FR$\alpha$), BCMA, PSMA, TROP-2, CD19, CD20, CD22, CD30, CD79b, EGFR, c-Met, or CEACAM5.

In certain embodiments, the ligand comprises an antibody or an antigen-binding fragment thereof.

In certain embodiments, the antibody comprises a monoclonal antibody, a polyclonal antibody, a dimer, a multimer, a multispecific antibody, an intact antibody, an antibody fragment, a human antibody, a humanized antibody, or a chimeric antibody.

In certain embodiments, the antigen-binding fragment comprises a Fab, a Fab', an Fv fragment, an F(ab')2, an scFv, a di-scFv, and/or a dAb.

In certain embodiments, the antibody is a monoclonal antibody.

In certain embodiments, the antibody is a human antibody, a humanized antibody, or a chimeric antibody.

In certain embodiments, the conjugate comprises a ligand-drug conjugate, and has the following structure: C-$(L-D)_m$, wherein C represents a ligand unit, L represents a linker, D represents a drug unit, m represents any number from 1 to 10.

In certain embodiments, the ligand-drug conjugate is an antibody-drug conjugate (ADC), and has the following structure: Ab-$(L-D)_m$, wherein, Ab represents an antibody or an antigen-binding fragment thereof, L represents a linker, D represents a drug unit, m represents any number from 1 to 10.

In certain embodiments, the drug unit comprises a cytotoxic agent.

In certain embodiments, the drug unit comprises: amanitins, anthracyclines, auristatins, baccatins, calicheamicins, camptothecins, cemadotins, colchicines, colcimids, combretastatins, cryptophycins, discodermolides, duocarmycins, docetaxel, doxorubicin, duocarmycins, echinomycins, eleutherobins, epothilones, estramustines, lexitropsins, maytansines, maytansinoids, methotrexate, netropsins, pyrrolo[2,1-*c*][1,4]benzodiazepines (PBDs), puromycins, rhizoxins, SN-38, taxanes, tubulysins, vinca alkaloids, or derivatives thereof.

In certain embodiments, the drug unit comprises a DNA topoisomerase I inhibitor.

In certain embodiments, the drug unit comprises camptothecin or a derivative thereof.

In certain embodiments, the drug unit has the following structure:

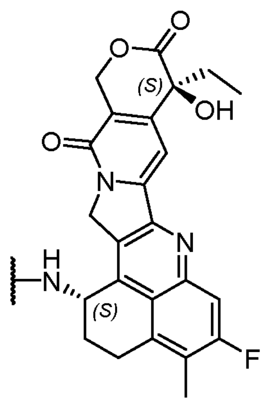

Dxd

wherein the nitrogen atom of the amino group at position 1 is a group at an attachment position.

In certain embodiments, the antibody-drug conjugate is selected from the following structures:

Ab-[-(succinimid-3-yl-N)-$CH_2CH_2$-C(=O)-RL-NH-$CH_2CH_2$-C(=O)-Dxd$]_m$;
Ab-[-(succinimid-3-yl-N)-$CH_2CH_2$-C(=O)-RL-NH-$CH_2CH_2CH_2$-C(=O)-Dxd$]_m$;
Ab-[-(succinimid-3-yl-N)-$CH_2CH_2CH_2CH_2CH_2$-C(=O)-RL-NH-$CH_2CH_2$-C(=O)-Dxd$]_m$;
Ab-[-(succinimid-3-yl-N)-$CH_2CH_2CH_2CH_2CH_2$-C(=O)-RL-NH-$CH_2CH_2CH_2$-C(=O)-Dxd$]_m$;
Ab-[-(succinimid-3-yl-N)-$CH_2CH_2CH_2CH_2CH_2$-C(=O)-RL-NH-$CH_2CH_2CH_2CH_2CH_2$-C(=O)-Dxd$]_m$;
Ab-[-(succinimid-3-yl-N)-$CH_2CH_2CH_2CH_2CH_2$-C(=O)-RL-NH-$CH_2$-O-$CH_2$-C(=O)-Dxd$]_m$;
Ab-[-(succinimid-3-yl-N)-$CH_2CH_2CH_2CH_2CH_2$-C(=O)-RL-NH-$CH_2CH_2$-O-$CH_2$-C(=O)-Dxd$]_m$;
Ab-[-(succinimid-3-yl-N)-$CH_2CH_2$-C(=O)-NH-$CH_2CH_2O$-$CH_2CH_2O$-$CH_2CH_2$-C(=O)-RL-NH-$CH_2CH_2CH_2$-C(=O)-Dxd$]_m$;

Ab-[-(succinimid-3-yl-N)-$CH_2CH_2$-C(=O)-NH-$CH_2CH_2O$-$CH_2CH_2O$-$CH_2CH_2$-C(=O)-RL-NH-$CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2$-C(=O)-Dxd]$_m$;
Ab-[-(succinimid-3-yl-N)-$CH_2CH_2$-C(=O)-VASGFG-NH-$CH_2CH_2$-C(=O)-Dxd]$_m$;
Ab-[-(succinimid-3-yl-N)-$CH_2CH_2$-C(=O)-VASGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2CH_2CH_2CH_2$-C(=O)-Dxd]m;
Ab-[-(succinimid-3-yl-N)-$CH_2CH_2CH_2CH_2CH_2$-C(=O)-VASGFG-NH-$CH_2$-O-$CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2$-O-$CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-VASGFG-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-VASGFG-NH-$CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VAS-GFG-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VAS-GFG-NH-$CH_2CH_2$-C(=O)-Dxd]$_m$;
Ab-[-(succinimid-3-yl-N)-$CH_2CH_2$-C(=O)-VASSGFG-NH-$CH_2CH_2$-C(=O)-Dxd]$_m$;
Ab-[-(succinimid-3-yl-N)-$CH_2CH_2$-C(=O)-VASSGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2CH_2CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2$-O-$CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2$-O-$CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-VASSGFG-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-VASSGFG-NH-$CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VASS-GFG-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VASS-GFG-NH-$CH_2CH_2$-C(=O)-Dxd]$_m$;
Ab-[-$CH_2$-C(=O)-NH-$CH_2CH_2$-C(=O)-RL-NH-$CH_2CH_2$-C(=O)-Dxd]$_m$;
Ab-[-$CH_2$-C(=O)-NH-$CH_2CH_2$-C(=O)-RL-NH-$CH_2CH_2CH_2$-C(=O)-Dxd]$_m$;
Ab-[-$CH_2$-C(=O)-NH-$CH_2CH_2$-C(=O)-RL-NH-$CH_2$-O-$CH_2$-C(=O)-Dxd]$_m$;
Ab-[-$CH_2$-C(=O)-NH-$CH_2CH_2$-C(=O)-RL-NH-$CH_2CH_2$-O-$CH_2$-C(=O)-Dxd]$_m$;
Ab-[-$CH_2$-C(=O)-NH-$CH_2CH_2CH_2$-C(=O)-RL-NH-$CH_2CH_2$-C(=O)-Dxd]$_m$;
Ab-[-$CH_2$-C(=O)-NH-$CH_2CH_2CH_2$-C(=O)-RL-NH-$CH_2CH_2CH_2$-C(=O)-Dxd]$_m$;
Ab-[-$CH_2$-C(=O)-NH-$CH_2CH_2CH_2$-C(=O)-RL-NH-$CH_2$-O-$CH_2$-C(=O)-Dxd]$_m$;
Ab-[-$CH_2$-C(=O)-NH-$CH_2CH_2CH_2$-C(=O)-RL-NH-$CH_2CH_2$-O-$CH_2$-C(=O)-Dxd]$_m$;
Ab-[-$CH_2$-C(=O)-NH-$CH_2CH_2CH_2CH_2$-C(=O)-RL-NH-$CH_2CH_2$-C(=O)-Dxd]$_m$;
Ab-[-$CH_2$-C(=O)-NH-$CH_2CH_2CH_2CH_2$-C(=O)-RL-NH-$CH_2CH_2CH_2$-C(=O)-Dxd]$_m$;
Ab-[-$CH_2$-C(=O)-NH-$CH_2CH_2CH_2CH_2$-C(=O)-RL-NH-$CH_2$-O-$CH_2$-C(=O)-Dxd]$_m$;
Ab-[-$CH_2$-C(=O)-NH-$CH_2CH_2CH_2CH_2$-C(=O)-RL-NH-$CH_2CH_2$-O-$CH_2$-C(=O)-Dxd]$_m$;
Ab-[-$CH_2$-C(=O)-NH-$CH_2CH_2CH_2CH_2CH_2$-C(=O)-RL-NH-$CH_2CH_2$-C(=O)-Dxd]$_m$;
Ab-[-$CH_2$-C(=O)-NH-$CH_2CH_2CH_2CH_2CH_2$-C(=O)-RL-NH-$CH_2CH_2CH_2$-C(=O)-Dxd]$_m$;
Ab-[-$CH_2$-C(=O)-NH-$CH_2CH_2CH_2CH_2CH_2$-C(=O)-RL-NH-$CH_2$-O-$CH_2$-C(=O)-Dxd]$_m$;
Ab-[-$CH_2$-C(=O)-NH-$CH_2CH_2CH_2CH_2CH_2$-C(=O)-RL-NH-$CH_2CH_2$-O-$CH_2$-C(=O)-Dxd]$_m$;
Ab-[-S-$CH_2CH_2$-C(=O)-RL-NH-$CH_2CH_2$-C(=O)-Dxd]$_m$;
Ab-[-S-$CH_2CH_2CH_2$-C(=O)-RL-NH-$CH_2CH_2$-C(=O)-Dxd]$_m$;
Ab-[-S-$CH_2CH_2CH_2CH_2$-C(=O)-RL-NH-$CH_2CH_2$-C(=O)-Dxd]$_m$;
Ab-[-S-$CH_2CH_2CH_2CH_2CH_2$-C(=O)-RL-NH-$CH_2CH_2$-C(=O)-Dxd]$_m$;
Ab-[-S-$CH_2CH_2$-C(=O)-RL-NH-$CH_2CH_2CH_2$-C(=O)-Dxd]$_m$;
Ab-[-S-$CH_2CH_2CH_2$-C(=O)-RL-NH-$CH_2CH_2CH_2$-C(=O)-Dxd]$_m$;

$Ab-[-S-CH_2CH_2CH_2CH_2-C(=O)-RL-NH-CH_2CH_2CH_2-C(=O)-Dxd]_m$;
$Ab-[-S-CH_2CH_2CH_2CH_2CH_2-C(=O)-RL-NH-CH_2CH_2CH_2-C(=O)-Dxd]_m$;
$Ab-[-S-CH_2CH_2-C(=O)-RL-NH-CH_2-O-CH_2-C(=O)-Dxd]_m$;
$Ab-[-S-CH_2CH_2CH_2-C(=O)-RL-NH-CH_2-O-CH_2-C(=O)-Dxd]_m$;
$Ab-[-S-CH_2CH_2CH_2CH_2-C(=O)-RL-NH-CH_2-O-CH_2-C(=O)-Dxd]_m$;
$Ab-[-S-CH_2CH_2CH_2CH_2CH_2-C(=O)-RL-NH-CH_2-O-CH_2-C(=O)-Dxd]_m$;
$Ab-[-S-CH_2CH_2-C(=O)-RL-NH-CH_2CH_2-O-CH_2-C(=O)-Dxd]_m$;
$Ab-[-S-CH_2CH_2CH_2-C(=O)-RL-NH-CH_2CH_2-O-CH_2-C(=O)-Dxd]_m$;
$Ab-[-S-CH_2CH_2CH_2CH_2-C(=O)-RL-NH-CH_2CH_2-O-CH_2-C(=O)-Dxd]_m$;
$Ab-[-S-CH_2CH_2CH_2CH_2CH_2-C(=O)-RL-NH-CH_2CH_2-O-CH_2-C(=O)-Dxd]_m$;
$Ab-[-S-CH_2CH_2-C(=O)-NH-CH_2CH_2O-CH_2CH_2O-CH_2CH_2-C(=O)-RL-NH-CH_2CH_2CH_2-C(=O)-Dxd]_m$;
$Ab-[-S-CH_2CH_2-C(=O)-NH-CH_2CH_2O-CH_2CH_2O-CH_2CH_2O-CH_2CH_2-C(=O)-RL-NH-CH_2CH_2CH_2-C(=O)-Dxd]_m$;
and
$Ab-[-S-CH_2CH_2-C(=O)-NH-CH_2CH_2O-CH_2CH_2O-CH_2CH_2O-CH_2CH_2O-CH_2CH_2-C(=O)-RL-NH-CH_2CH_2CH_2-C(=O)-Dxd]_m$;

wherein,

-(succinimid-3-yl-N)- has the following structure:

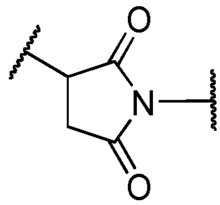
,

wherein the wavy line at position 3 of the structure represents an attachment position to a ligand unit, the wavy line at the N atom of position 1 represents an attachment position to A;
-RL- represents the following structure: -Val-Ala-Gly-Phe-Gly- (VAGFG), -Val-Ala-Ser-Gly-Phe-Gly- (VASGFG), or -Val-Ala-Ser-Ser-Gly-Phe-Gly- (VASSGFG);
Dxd has the following structure:

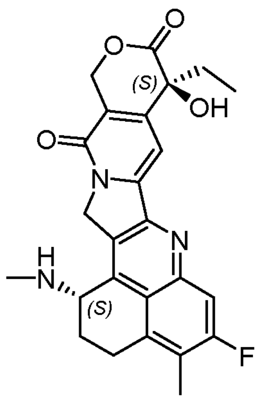
,

wherein the nitrogen atom of the amino group at position 1 is a group at an attachment position;
Ab represents an antibody or an antigen-binding fragment thereof, m represents any number from 1 to 10.

In certain embodiments, the antibody-drug conjugate is selected from the following structures:

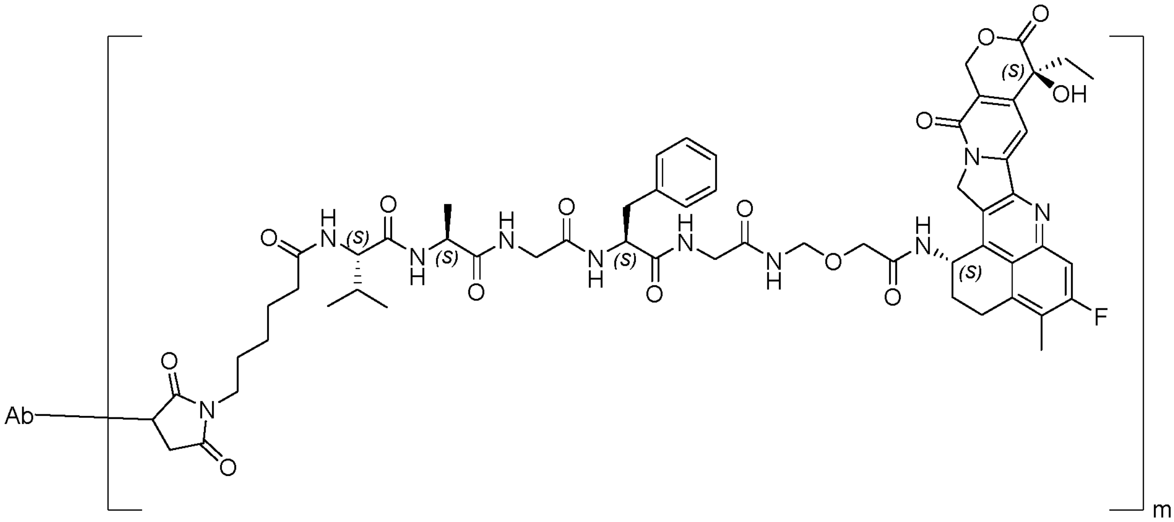

,

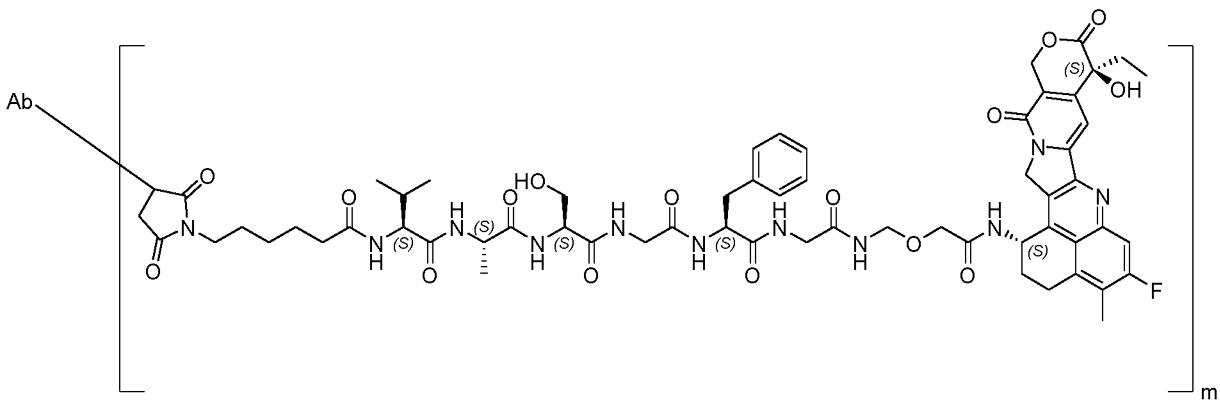

,

and

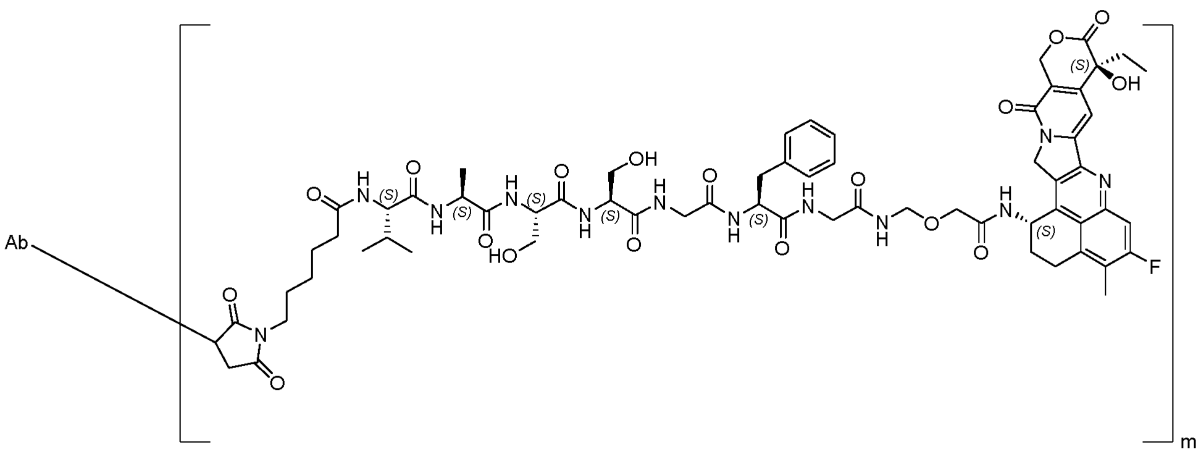

;

wherein, Ab represents an antibody or an antigen-binding fragment thereof, m is any number from 1 to 10.

In certain embodiments, the antibody or the antigen-binding fragment thereof comprises: an anti-HER2 antibody, an anti-HER3 antibody, an anti-claudin18.2 antibody, an anti-FRα antibody, or an antigen-binding fragment thereof.

In another aspect, the present application provides a pharmaceutical composition comprising the conjugate as described herein and optionally a pharmaceutically acceptable carrier.

In another aspect, the present application provides use of the conjugate as described herein or the pharmaceutical composition as described herein in preparing a medicament for treating a disease or a condition.

In certain embodiments, the disease or condition comprises a tumor.

In certain embodiments, the disease or condition comprises a solid tumor and a hematologic tumor.

In certain embodiments, the solid tumor comprises: gastric cancer, gastric cancer with peritoneal metastasis, liver cancer, leukemia, kidney tumor, lung cancer, small intestine cancer, bone cancer, prostate cancer, colorectal cancer, breast cancer, large intestine cancer, cervical cancer, ovarian cancer, lymph cancer, nasopharyngeal cancer, adrenal tumor, bladder tumor, non-small cell lung cancer (NSCLC), brain glioma, endometrial cancer, or a combination thereof.

In certain embodiments, the hematologic tumor comprises: acute myeloid leukemia (AML), multiple myeloma (MM), chronic lymphocytic leukemia (CLL), acute lymphocytic leukemia (ALL), diffuse large B-cell lymphoma (DLBCL), Hodgkin lymphoma, or a combination thereof.

In another aspect, the present application provides a method for treating a disease or a condition in a subject in need thereof, comprising administering to the subject an effective amount of the conjugate as described herein.

In certain embodiments, the disease or condition comprises a tumor.

In certain embodiments, the disease or condition comprises a solid tumor and a hematologic tumor.

In certain embodiments, the solid tumor comprises: gastric cancer, gastric cancer with peritoneal metastasis, liver cancer, leukemia, kidney tumor, lung cancer, small intestine cancer, bone cancer, prostate cancer, colorectal cancer, breast cancer, large intestine cancer, cervical cancer, ovarian cancer, lymph cancer, nasopharyngeal cancer, adrenal tumor, bladder tumor, non-small cell lung cancer (NSCLC), brain glioma, endometrial cancer, or a combination thereof.

In certain embodiments, the hematologic tumor comprises: acute myeloid leukemia (AML), multiple myeloma (MM), chronic lymphocytic leukemia (CLL), acute lymphocytic leukemia (ALL), diffuse large B-cell lymphoma (DLBCL), Hodgkin lymphoma, or a combination thereof.

Other aspects and advantages of the present application will be readily apparent to those skilled in the art from the following detailed description. Only exemplary embodiments of the present application have been shown and described in the following detailed description. As those skilled in the art will recognize, the content of the present application enables those skilled in the art to make changes in the specific embodiments disclosed without departing from the spirit and scope of the invention to which the present application relates. Accordingly, the drawings and description of the present application are provided only for purpose of illustration rather than limitation.

BRIEF DESCRIPTION OF THE DRAWINGS

The specific features of the invention to which the present application relates are set forth in the appended claims. The features and advantages of the invention to which the present application relates may be more fully understood with reference to the exemplary embodiments and drawings described in detail below. The drawings are briefly described as follows:

FIG. 1A shows an HIC-HPLC chromatogram of HER3-ADC04 according to Example 1 of the present application.

FIG. 1B shows an HIC-HPLC chromatogram of HER3-ADC07 according to Example 1 of the present application.

FIG. 1C shows an HIC-HPLC chromatogram of HER3-ADC08 according to Example 1 of the present application.

FIG. 1D shows an HIC-HPLC chromatogram of HER3-ADC09 according to Example 1 of the present application.

FIG. 1E shows an HIC-HPLC chromatogram of Claudin18.2-ADC08 according to Example 1 of the present application.

FIG. 1F shows an HIC-HPLC chromatogram of Claudin18.2-ADC09 according to Example 1 of the present application.

FIG. 1G shows an HIC-HPLC chromatogram of FRα-ADC08 according to Example 1 of the present application.

FIG. 1H shows an HIC-HPLC chromatogram of FRα-ADC09 according to Example 1 of the present application.

FIG. 2A shows an *in vitro* killing effect of HER3-ADC04 according to the present application on human colon cancer SW620 cells.

FIG. 2B shows an *in vitro* killing effect of different linker-Dxd drugs according to the present application on human colon cancer SW620 cells.

FIG. 3 shows an *in vitro* killing effect of anti-HER3 ADCs comprising different linkers according to the present application on human colon cancer SW620 cells.

FIG. 4 shows an *in vitro* killing effect of anti-HER3 ADCs comprising different linkers according to the present application on CHO-K1 non-target cells.

FIG. 5 shows an *in vitro* killing effect of anti-HER3 ADCs according to the present application on SW620 target cells after plasma incubation;

FIG. 6 shows a therapeutic effect of anti-HER3 ADCs according to the present application on a SW620 xenograft model.

FIG. 7 shows changes in the body weight of mice during treatment of a SW620 xenograft model with anti-HER3 ADCs according to the present application.

FIG. 8 shows an *in vitro* killing effect of anti-Claudin18.2 ADCs according to the present application on human gastric cancer NUGC4 cells.

FIG. 9 shows an *in vitro* killing effect of anti-Claudin18.2 ADCs according to the present application on recombinant human gastric cancer NUGC4 cells overexpressing Claudin18.2 molecules.

FIG. 10 shows an *in vitro* killing effect of anti-Claudin18.2 ADCs according to the present application on recombinant HEK293 cells overexpressing Claudin18.2 molecules.

FIG. 11 shows an *in vitro* killing effect of an anti-Claudin18.2 ADC according to the present application on HEK293 cells overexpressing Claudin18.2 molecules after plasma incubation.

FIG. 12 shows a growth inhibitory effect of an anti-claudin18.2 ADC according to the present application on an NUGC4 gastric cancer xenograft model.

FIG. 13 shows an *in vitro* killing effect of anti-FRα ADCs according to the present application on human ovarian cancer OVCAR3 cells.

FIG. 14 shows an *in vitro* killing effect of an anti-FRα ADC according to the present application on human ovarian cancer OVCAR3 cells after plasma incubation.

FIG. 15 shows a growth inhibitory effect of an anti-FRα ADC according to the present application on an OVCAR3 subcutaneous xenograft mouse model.

DETAILED DESCRIPTION

The implementation of the invention to which the present application relates is described below with reference to specific embodiments, and other advantages and effects of the invention to which the present application relates will be readily apparent to those skilled in the art from the disclosure of the present specification.

Definitions

In the present application, the terms "antibody drug conjugate (antibody-drug conjugate)" and "ADC" are used interchangeably, and generally refer to one or more therapeutic compounds (e.g., Dxd) conjugated to one or more antibodies or antigen-binding fragments, which are defined by the following general formula: $Ab\text{-}(L\text{-}D)_m$, wherein Ab = antibody or antigen-binding fragment, L = linker, D = drug unit, and m = the number of drug moieties per antibody or antigen-binding fragment, and may be any number from 1 to 10. In some embodiments, the linker L may comprise the cleavable moiety between the antibody or antigen-binding fragment and the drug unit. Exemplary cleavable adapters are described and exemplified herein.

In the present application, the term "antibody" is usually used in the broadest sense and specifically encompasses monoclonal antibodies, polyclonal antibodies, dimers, multimers, multispecific antibodies (e.g., bispecific antibodies), and antibody fragments, so long as they exhibit the desired biological activity (Miller et al., (2003), Jour. of Immunology, 170:4854-4861). Antibodies may be murine, human, humanized, chimeric antibodies, or derived from other species. Without limitation, "antibody" may typically comprise a protein having at least two heavy chains (HCs) and two light chains (LCs) interconnected by disulfide bonds, or an antigen-binding fragment thereof. Each of the heavy chains comprises a heavy chain variable region (VH) and a heavy chain constant region. In certain naturally occurring IgG, IgD, and IgA antibodies, the heavy chain constant region comprise three domains, i.e., CH1, CH2, and CH3. In certain naturally occurring antibodies, each of the light chains comprises a light chain variable region (VL) and a light chain constant region. The light chain constant region comprises one domain CL. VH and VL regions can be further subdivided into hypervariable regions called complementarity-determining regions (CDRs), which are interspersed with more conserved regions called framework regions (FRs). VH and VL each comprises three CDRs and four FRs, arranged from amino-terminus to carboxyl-terminus in the following order: FR1, CDR1, FR2, CDR2, FR3, CDR3, and FR4. The variable domains of natural heavy chains and light chains individually comprise four FRs (HFR1, HFR2, HFR3, HFR4, LFR1, LFR2, LFR3, and LFR4), mostly adopting a β-folding configuration, connected by three CDRs to form a loop linkage, and in some cases, part of a β-folding structure. The CDRs in each of the chains are held together in close proximity by the FRs and, together with the CDRs from the other chain, contribute to the formation of the antigen-binding site of antibodies. The constant region of antibodies can mediate the binding of immunoglobulins to host tissues or factors, including various cells (such as effector cells) of immune system and the first component (Clq) of classical complement system.

In the present application, the term "antigen-binding fragment" or "functional fragment of antibody" (also referred to herein as "targeting portion" or "antigen-binding portion") generally refers to a portion of antibody molecule comprising amino acids responsible for specific binding of antibody to antigen. The portion of the antigen specifically recognized and bound by the antibody is referred to as the "epitope" as described above. An antigen-binding fragment may typically comprise an antibody light chain variable region (VL) and an antibody heavy chain variable region (VH); however, it does not necessarily comprise both. Examples of antigen-binding fragments of antibodies include: (1) a Fab fragment,

a monovalent fragment having a VL, a VH, a light chain constant region (CL), and a CH1 domain; (2) a F(ab')$_2$ fragment, a bivalent fragment having two Fab fragments linked by a disulfide bridge in the hinge region; (3) an Fd fragment, having two VH and CH1 domains; (4) an Fv fragment, having VL and VH domains of a single arm of an antibody; (5) a dAb fragment (Ward et al., "Binding Activities of a Repertoire of Single Immunoglobulin Variable Domains Secreted From Escherichia coli," Nature 341:544-546(1989), which is incorporated herein by reference in its entirety), having a VH domain; (6) an isolated complementarity-determining region (CDR); (7) a single-chain Fv (scFv), e.g., derived from a scFV library, although the two domains VL and VH of the Fv fragment are encoded by independent genes, they may be joined using a recombinant method via a synthetic linker, and the synthetic linker enables the VL and VH regions to be prepared as a single protein chain (called single-chain Fv (scFv)), in which the VL and VH regions pair to form a monovalent molecule (see, for example, Huston et al., "Protein Engineering of Antibody Binding Sites: Recovery of Specific Activity in an Anti-Digoxin Single-Chain Fv Analogue Produced in Escherichia coli," Proc. Natl. Acad. Sci. USA 85:5879-5883(1988)); and (8) VHH, relating to a variable antigen-binding domain of a heavy chain antibody derived from Camelidae (camel, dromedary, llama, alpaca, etc.) (see Nguyen VK. et al., 2000, The EMBO Journal, 19, 921-930; Muyldermans S., 2001, J Biotechnol., 74, 277-302, and review Vanlandschoot P. et al., 2011, Antiviral Research 92, 389-407). The antibody fragments are obtained using conventional techniques known to those skilled in the art, and the functions of the fragments can be assessed in the same manner as for intact antibodies.

In the present application, the term "variable" generally refers to the fact that certain portions of sequences of variable domains of antibodies differ extensively, resulting in the binding and specificity of various specific antibodies to their specific antigens. However, the variability is not evenly distributed throughout the variable region of the antibody. It is concentrated in three segments, called complementarity-determining regions (CDRs) or hypervariable regions (HVRs), in each of the light chain and heavy chain variable regions. The more highly conserved portions of variable domains are called framework regions (FRs). In the art, the CDRs of antibodies may be defined by a variety of methods, such as Kabat definition rules based on sequence variability (see Kabat et al., Sequences of Proteins of Immunological Interest, fifth edition, National Institutes of Health, Bethesda, Md. (1991)), Chothia definition rules based on the positions of structural loop regions (see A1-Lazikani et al., J Mol Biol 273:927-48, 1997), and KABAT definition rules based on IMGT-ONTOLOGY concepts and IMGT Scientific chart rules.

In the present application, the term "monoclonal antibody" generally refers to an antibody obtained from a population of substantially homogeneous antibodies, that is, the individual antibodies in the population are identical except for a small number of natural mutations that may be present. The monoclonal antibodies are typically highly specific for a single antigenic site. Moreover, unlike conventional polyclonal antibody formulations (which typically have different antibodies directed against different determinants), each monoclonal antibody is directed against a single determinant on the antigen. In addition to their specificity, the monoclonal antibodies are advantageous in that they can be synthesized by hybridoma culture without contamination by other immunoglobulins. The modifier "monoclonal" indicates the characteristic of the antibody obtained from a population of substantially homogeneous antibodies, and is not to be construed as requiring production of the antibody by any particular method. For example, the monoclonal antibodies used in the present application may be prepared in hybridoma cells, or may be prepared by the recombinant DNA method.

In the present application, the term "chimeric antibody" generally refers to an antibody in which the variable region is derived from one species and the constant region is derived from another species. Typically, the variable region is derived from an antibody ("parent antibody") of a laboratory animal such as a rodent, and the constant region is derived from a human antibody, such that the resulting chimeric antibody is less likely to induce an adverse immune response in a human individual as compared to the parent (e.g., mouse-derived) antibody.

In the present application, the term "humanized antibody" generally refers to an antibody in which some or all of the amino acids other than the CDRs of a non-human antibody (e.g., a mouse antibody) are substituted with corresponding amino acids derived from human immunoglobulins. In the CDRs, small additions, deletions, insertions, substitutions, or modifications of amino acids may also be permissible, so long as they retain the ability of the antibody to bind to a specific antigen. A humanized antibody may optionally comprise at least a portion of human immunoglobulin constant regions. "Humanized antibodies" retain similar antigenic specificity to original antibodies. "Humanized" forms of non-human (e.g., murine) antibodies are chimeric antibodies minimally comprising sequences derived from non-human immunoglobulins. In certain cases, residues in CDR regions of a human immunoglobulin (acceptor antibody) may be replaced with residues in CDRs from a non-human species (donor antibody) (such as mouse, rat, rabbit, or nonhuman primate) having the desired properties, affinity, and/or capacity. In certain cases, residues in FR regions of a human immunoglobulin may be replaced with corresponding non-human residues. In addition, humanized antibodies may comprise amino acid modifications that are not present in the acceptor antibody or in the donor antibody. These modifications may be made to further improve the properties of the antibodies, such as binding affinity.

In the present application, the term "fully human antibody" or "human antibody" generally refers to the antibody produced by transferring a human antibody-encoding gene to a genetically engineered animal with antibody gene deletion and expressing the antibody in the animal. All portions of the antibody (including the variable regions and constant regions of the antibody) are encoded by genes of human origin. The fully human antibodies can greatly reduce the

adverse immune responses of heterologous antibodies to human body. The methods for obtaining the fully human antibodies in the art include phage display technology, transgenic mouse technology, ribosome display technology, RNA-polypeptide technology, and the like.

In the present application, the term "specific binding" when referring to the interaction of a binding molecule (e.g., an antibody) with its binding partner (e.g., an antigen) generally means that the interaction is dependent on the presence of a specific structure (e.g. an antigenic determinant or epitope) on the binding partner. In other words, the antibody will preferentially bind to or recognize the binding partner even when the binding partner is present in a mixture with other molecules or organisms. The binding may be mediated by covalent or non-covalent interaction or a combination of both. In other words, the term "specific binding" generally refers to the immunospecific binding to an antigenic determinant or epitope without immunospecific binding to other antigenic determinants or epitopes. The binding molecule with immunospecific binding to an antigen can bind to other peptides or polypeptides with lower affinity, as determined by, for example, radioimmunoassay (RIA), enzyme-linked immunosorbent assay (ELISA), BIACORE, or other assays known in the art. The binding molecule or a fragment thereof with immunospecific binding to an antigen can cross-react with a related antigen with the same epitope. In certain cases, the binding molecule or a fragment thereof with immunospecific binding to an antigen does not cross-react with other antigens.

In the present application, the term "linker", "adapter", or "L" generally refers to any chemical moiety capable of covalently conjugating a compound (typically a drug unit) to another moiety (such as an antibody or an antigen-binding fragment). An adapter may be susceptible to or substantially resistant to acid-induced cleavage, peptidase-induced cleavage, light-based cleavage, esterase-induced cleavage, and/or disulfide bond cleavage under the conditions of maintaining the compound or antibody activity.

In the present application, the term "drug unit" or "D" generally refers to any compound having the desired biological activity and reactive functional group, which can be used to incorporate a drug into a conjugate of the present application. In some embodiments, the drug unit represents a cytotoxic drug for cancer therapy; proteins or polypeptides having a desired biological activity, for example, toxins such as abrin, ricin A, pseudomonas exotoxin, and diphtheria toxin; and other suitable proteins, including tumor necrosis factors, interferon-alpha, interferon-beta, nerve growth factors, platelet-derived growth factors, tissue plasminogen activators, and biological response modifiers, such as lymphokines, interleukin-1 (IL-1), interleukin-2 (IL-2), interleukin-6 (IL-6), granulocyte-macrophage colony-stimulating factor (GM-CSF), granulocyte colony-stimulating factor (G-CSF), or other growth factors.

In the present application, the term "cytotoxic agent" generally refers to a substance that inhibits or prevents cell functions and/or causes cell death or destruction. The cytotoxic agent includes, but is not limited to, radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$, $Pb^{212}$, and radioactive isotopes of Lu); chemotherapeutic agents or drugs (e.g., methotrexate, doxorubicin, vinca alkaloids (vincristine, vinblastine, etoposide), doxorubicin, melphalan, mitomycin C, chlorambucil, daunomycin, or other intercalating agents; growth inhibitors; enzymes and fragments thereof, such as nucleolytic enzymes; antibiotics; toxins, such as small molecule toxins or enzymatically active toxins, of bacterial, fungal, plant or animal origins, including fragments and/or variants thereof; and various antitumor or anticancer agents disclosed below.

In the present application, the term "pharmaceutically acceptable form" generally refers to a form of the disclosed compound, including, but not limited to, pharmaceutically acceptable salts, esters, hydrates, solvates, polymorphs, isomers, prodrugs, and isotopically labeled derivatives thereof. In one embodiment, "pharmaceutically acceptable form" includes, but is not limited to, pharmaceutically acceptable salts, esters, prodrugs, and isotopically labeled derivatives thereof. In some embodiments, "pharmaceutically acceptable form" includes, but is not limited to, pharmaceutically acceptable isomers and stereoisomers, prodrugs, and isotopically labeled derivatives thereof.

The linker, linker-drug, and conjugate of the present application may comprise any pharmaceutically acceptable form thereof, such as pharmaceutically acceptable salts, esters, hydrates, solvates, polymorphs, isomers, prodrugs, or isotopically labeled derivatives. The pharmaceutically acceptable forms described above are also within the scope of the present application.

In the present application, the term "pharmaceutically acceptable salt" generally refers to a pharmaceutically acceptable organic or inorganic salt of a compound (e.g., a drug, a linker-drug, or a conjugate). In some aspects, the compound may contain at least one amino group, and thus may form acid addition salts with the amino groups. Exemplary salts include, but are not limited to, sulfate, trifluoroacetate, citrate, acetate, oxalate, chloride, bromide, iodide, nitrate, bisulfate, phosphate, acid phosphate, isonicotinate, lactate, salicylate, citrate, tartrate, oleate, tannate, pantothenate, bitartrate, ascorbate, succinate, maleate, gentisate, fumarate, gluconate, glucuronate, saccharate, formate, benzoate, glutamate, methanesulfonate, ethanesulfonate, benzenesulfonate, *p*-toluenesulfonate, and pamoate (i.e., 1,1'-methylenebis(2-hydroxy-3-naphthoate)). The pharmaceutically acceptable salt may involve the introduction of another molecule, such as acetate ion, succinate ion, or other counterions. The counterion can be any organic or inorganic moiety to stabilize the charge of the parent compound. In addition, the pharmaceutically acceptable salt may have more than one charged atom in its structure. In the case that a plurality of charged atoms are part of the pharmaceutically acceptable salt, the salt may have a plurality of counterions. Therefore, the pharmaceutically acceptable salt may have one or more

charged atoms and/or one or more counterions.

In the present application, the term "derivative" generally refers to a chemical compound or molecule made from a parent compound by one or more chemical reactions.

In the present application, the term "prodrug" generally refers to a less biologically active or inactive compound, which is converted into a more biologically active compound *in vivo* via a chemical or biological process (i.e., a chemical reaction or enzymatic bioconversion).

In the present application, the term "isotopically labeled compound" or "isotopically labeled derivative" generally refers to a presently disclosed compound in which one or more atoms are replaced by an atom having the atomic mass or mass number different from the atomic mass or mass number usually found in nature, including pharmaceutically acceptable salts and prodrugs each as described herein. Examples of isotopes that can be incorporated into the presently disclosed compounds include isotopes of hydrogen, carbon, nitrogen, oxygen, phosphorus, fluorine, and chlorine, such as $^{2}H$, $^{3}H$, $^{13}C$, $^{14}C$, $^{15}N$, $^{18}O$, $^{17}O$, $^{31}P$, $^{32}P$, $^{35}S$, $^{18}F$ and $^{36}Cl$, respectively.

In the present application, the term "pharmaceutically acceptable carrier" generally refers to one or more non-toxic substances that do not interfere with the effectiveness of the biological activity of an active ingredient. Such formulations may typically comprise a salt, a buffer, a preservative, a compatible carrier, an adjuvant, and optionally other therapeutic agents. Such pharmaceutically acceptable formulations may also typically comprise a compatible solid or liquid filler, a diluent, or an encapsulating material, suitable for administration to humans. Without limitation, the pharmaceutically acceptable carriers may comprise a liquid, such as water, saline, glycerol, and ethanol. In these carriers, an auxiliary substance may be present, such as a wetting agent or emulsifying agent, a pH buffering substance, etc.

In the present application, the term "alkyl" or "alkyl group" generally refers to a fully saturated straight, branched, or cyclic hydrocarbon chain. In certain embodiments, the alkyl may contain 1-8 carbon atoms ("$C_1$-$C_8$ alkyl"). In certain embodiments, the alkyl may contain 1-6 carbon atoms ("$C_1$-$C_6$ alkyl"). In certain embodiments, the alkyl contains 1-3 carbon atoms. In still other embodiments, the alkyl contains 2-3 carbon atoms, and in still other embodiments, the alkyl contains 1-2 carbon atoms.

In the present application, the term "alkylalkoxy" refers to alkyl substituted with alkoxy.

In the present application, the term "alkoxy" refers to the alkyl as previously defined attached to a main carbon chain via an oxygen atom ("alkoxy").

In the present application, the term "alkylhydroxyl" refers to alkyl substituted with hydroxyl.

In the present application, the term "hydroxyl (or hydroxy)" refers to -OH.

In the present application, the term "carbocycle" or "carbocyclyl" includes both aromatic groups (e.g., aryl) and non-aromatic groups (e.g., cycloalkyl). In certain embodiments, the carbocycle group contains 3-10 carbon atoms ("3- to 10-membered carbocycle"). In certain embodiments, the carbocycle group contains 3-8 carbon atoms ("3- to 8-membered carbocycle"). In certain embodiments, the carbocycle group contains 3-6 carbon atoms ("3- to 6-membered carbocycle"). In certain embodiments, the carbocycle group contains 3-5 carbon atoms ("3- to 5-membered carbocycle").

In the present application, the term "haloalkyl" refers to alkyl substituted with one or more halogen atoms.

In the present application, the term "halogen" refers to any halogen group, such as -F, -Cl, -Br, or -I. In the present application, the terms "heterocycle (or heterocyclyl or heterocyclic)" and "heterocyclyl group" refer to monocyclic heterocycle, bicyclic heterocycle, or tricyclic heterocycle containing at least one heteroatom in the ring. The terms "heterocycle (or heterocyclyl or heterocyclic)" and "heterocyclyl group" encompass heteroaryl. "Heteroaryl" refers to a cyclic moiety having one or more closed rings and one or more heteroatoms (oxygen, nitrogen, or sulfur) in at least one of such rings, wherein at least one of such rings is an aromatic ring, and wherein the ring or such rings may be independently fused and/or bridged. Examples include, but are not limited to, phenyl, thienyl, triazolyl, pyridinyl, pyrimidinyl, pyridazinyl, and pyrazinyl.

In the present application, the term "PEG unit" is generally an organic moiety consisting of repeating ethylene-oxy subunits (PEG or PEG subunits), and may be polydisperse, monodisperse, or discrete (i.e., having a discrete number of ethylene-oxy subunits). Polydisperse PEG is a heterogeneous mixture in size and molecular weight, whereas monodisperse PEG is typically purified from a heterogeneous mixture and thus has single chain length and molecular weight. The PEG unit of the present application may comprise one or more polyethylene glycol chains, and each of the polyethylene glycol chains consists of one or more ethylene-oxy subunits covalently attached to each other. The polyethylene glycol chains may be linked together, for example, in a linear, branched or star configuration.

In the present application, the term "substituted", whether preceded by the term "optionally" or not, generally refers to the replacement of one or more hydrogen of the designated moiety with appropriate substituents. Unless otherwise indicated, an "optionally substituted" group may have a suitable substituent at each substitutable position of the group, and when more than one position in any given structure may be substituted with more than one substituent selected from a specified group, the substituents at each position may be the same or not. Combinations of substituents contemplated in accordance with the present disclosure are preferably those resulting in the formation of stable or chemically feasible compounds.

It will be understood by those of ordinary skill in the art that "substitution", "substituted", or "absent" includes

the following implicit limitations: the substitution or absence is in accordance with the permitted valence of the substituted atom and substituent, and the substitution or absence results in a stable compound, for example, the stable compound does not spontaneously undergo transformation, e.g. by rearrangement, cyclization, elimination, and the like. For the purposes of the present disclosure, a heteroatom such as nitrogen may have a hydrogen substituent and/or any permissible substituent of organic compounds described herein satisfying the valence of the heteroatom.

In the present application, the term "preventing and/or treating" includes not only preventing and/or treating a disease, but also generally includes preventing the onset of a disease, slowing or reversing the progression of a disease, preventing or slowing the onset of one or more symptoms associated with a disease, reducing and/or alleviating one or more symptoms associated with a disease, reducing the severity and/or duration of a disease and/or any symptom associated therewith, and/or preventing further increase in the severity of a disease and/or any symptom associated therewith, preventing, reducing or reversing any physiological damage caused by a disease, and any pharmacological effect which would normally be beneficial to the patient being treated. The viable therapeutic agent formed of the composition of the present application is not required to realize complete cure or eradication of any symptom or manifestation of a disease. As recognized in the art, a drug used as a therapeutic agent may reduce the severity of a given disease state, and does not need to eliminate every manifestation of the disease to be considered a useful therapeutic agent. Similarly, a viable prophylactic agent by applying prophylactic treatment is not required to entirely effective in preventing the onset of a condition. It is enough to simply reduce the impact of a disease (e.g., by reducing the number or severity of its symptoms, or by increasing the effectiveness of another treatment, or by producing another beneficial effect), or reduce the likelihood of the disease occurrence or worsening, in a subject.

In the present application, the term "administering" generally refers to the delivery of a protein (including an immunoglobulin) to a human or animal in need thereof by any route known in the art. Pharmaceutically acceptable carriers and formulations or compositions are also well known in the art. Routes of administration may include: intravenous, intramuscular, intradermal, subcutaneous, transdermal, mucosal, intratumoral, or mucosal administrations.

In the present application, the term "contact" generally means that two or more different types of substances come into contact in any order, in any manner, and for any length of time. When being applied to cells, the term "contact" generally refers to a method by which the antibody or the antigen-binding fragment thereof, the drug, the linker-drug, the conjugate, and/or the pharmaceutical composition of the present application is delivered to or placed in direct proximity to target cells, wherein the delivery may be *in vitro* or *in vivo* and may relate to the use of a recombinant vector system. The "contact" may occur *in vivo, ex vivo,* or *in vitro.*

In the present application, the term "effective amount" or "effective dose" generally refers to an amount sufficient to achieve, or at least partially achieve, a desired effect. A "therapeutically effective amount" or "therapeutically effective dose" of a drug or therapeutic agent is generally any amount of the drug that promotes the regression of a disease (as evidenced by a decrease in the severity of disease symptom, an increase in the frequency and duration of the asymptomatic phase of the disease, or the prevention of damage or disability due to the suffering of the disease) when being used alone or in combination with another therapeutic agent. A "prophylactically effective amount" or "prophylactically effective dose" of a drug generally refers to an amount of the drug that inhibits the progression or recurrence of a disease when being administered, alone or in combination with another therapeutic agent, to a subject at risk of disease progression or disease recurrence. The capacity of a therapeutic agent or prophylactic agent to promote disease regression or inhibit disease progression or recurrence can be assessed using a variety of methods known to those skilled in the art, such as by predicting efficacy in humans in a human subject during clinical trial and in animal model systems, or by measuring the activity of the agent in an *in vitro* assay.

In the present application, the term "tumor" generally refers to all neoplastic cell growth and proliferation, whether malignant or benign, and all pre-cancerous and cancerous cells and tissues. The terms "cancer", "carcinoma", "cell proliferative disorder", "proliferative disorder", and "tumor" are not mutually exclusive when referred to herein. In the present application, a tumor may be a solid tumor or a non-solid tumor.

In the present application, the term "subject" generally refers to a human or a non-human animal (including mammals) in need of diagnosis, prognosis, amelioration, prevention and/or treatment of a disease, such as human, non-human primates (apes, gibbons, gorillas, chimpanzees, orangutans, and macaques), livestock (dogs and cats), farm animals (poultry such as chickens and ducks, horses, cattle, goats, sheep, and pigs), and laboratory animals (mice, rats, rabbits, and guinea pigs). Human subjects include fetal, neonatal, infant, juvenile, and adult subjects. The subject may include an animal disease model.

In the present application, the terms "comprising", "including", "having", "may", "containing" and variations thereof are generally intended to be open-ended transitional phrases, terms, or words, which do not exclude the possibility of additional acts or structures. The term "consisting of ..." generally means that no other components (or likewise, features, integers, steps, etc.) can be present. The singular forms "a", "an", and "the" include plural referents unless the content clearly dictates otherwise.

In the present application, the term "about" generally refers to approximately, in the region of, roughly, or around. When the term "about" is used in reference to a range of values, the cutoff or specified value is used to indicate that the

value set forth can differ from the listed value by as much as 10%. Therefore, the term "about" can be used to encompass variations of ± 10% or less, variations of ± 5% or less, variations of ± 1% or less, variations of ± 0.5% or less, or variations of ± 0.1% or less from a specified value.

Detailed description

Linker

The present application provides a novel linker (conjugating linker) structure comprising an amino acid unit (RL), wherein RL comprises: -Val-Ala-Q-Gly-Phe-Gly-, wherein Q is a bond, a single amino acid, or a dipeptide.
In certain embodiments, RL is cleavable by an enzyme.
In certain embodiments, RL is cleavable by cathepsin B.
In certain embodiments, Q is a bond.
In certain embodiments, Q is -Ser- or -Ser-Ser-.
In certain embodiments, RL comprises -Val-Ala-Gly-Phe-Gly- (VAGFG), -Val-Ala-Ser-Gly-Phe-Gly- (VASGFG), or -Val-Ala-Ser-Ser-Gly-Phe-Gly- (VASSGFG).
In certain embodiments, the linker is selected from the following structures: -Z-A-S*-RL- and -Z-AS*-RL-Y-; wherein, Z is an extender unit, the A is a bond or an adapter unit; S* is a bond or a partitioning agent; Y is a spacer unit.
In certain embodiments, Z comprises the following structure: -(succinimid-3-yl-N)-, $-CH_2-C(=O)-NH-$ or -C(=O)-; wherein, -(succinimid-3-yl-N)- has the following structure:

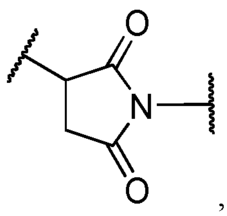
,

wherein the wavy line at position 3 of the structure represents an attachment position to a ligand unit, the wavy line at the N atom of position 1 represents an attachment position to A.
In certain embodiments, A is selected from the following structures:

$-C_1-C_{10}$ alkylene-, $-C_1-C_{10}$ heteroalkylene-, $-C_3-C_8$ carbocyclyl-, $-O-(C_1-C_8$ alkylene)-, -arylene-, $-C_1-C_{10}$ alkylene-arylene-, -arylene-$C_1-C_{10}$ alkylene-, $-C_1-C_{10}$ alkylene-($C_3-C_8$ carbocyclyl)-, -($C_3-C_8$ carbocyclyl)-$C_1-C_{10}$ alkylene-, $-C_3-C_8$ heterocyclyl-, $-C_1-C_{10}$ alkylene-($C_3-C_8$ heterocyclyl)-, -($C_3-C_8$ heterocyclyl)-$C_1-C_{10}$ alkylene-, $-C_1-C_{10}$ alkylene-C(=O)-, $-C_1-C_{10}$ heteroalkylene-C(=O)-, $-C_3-C_8$ carbocyclyl-C(=O)-, -O-($C_1-C_8$ alkylene)-C(=O)-, -arylene-C(=O)-, $-C_1-C_{10}$ alkylene-arylene-C(=O)-, -arylene-$C_1-C_{10}$ alkylene-C(=O)-, $-C_1-C_{10}$ alkylene-($C_3-C_8$ carbocyclyl)-C(=O)-, -($C_3-C_8$ carbocyclyl)-$C_1-C_{10}$ alkylene-C(=O)-, $-C_3-C_8$ heterocyclyl-C(=O)-, $-C_1-C_{10}$ alkylene-($C_3-C_8$ heterocyclyl)-C(=O)-, -($C_3-C_8$ heterocyclyl)-$C_1-C_{10}$ alkylene-C(=O)-, $-C_1-C_{10}$ alkylene-NH-, $-C_1-C_{10}$ heteroalkylene-NH-, $-C_3-C_8$ carbocyclyl-NH-, -O-($C_1-C_8$ alkylene)-NH-, -arylene-NH-, $-C_1-C_{10}$ alkylene-arylene-NH-, -arylene-$C_1-C_{10}$ alkylene-NH-, $-C_1-C_{10}$ alkylene-($C_3-C_8$ carbocyclyl)-NH-, - ($C_3-C_8$ carbocyclyl)-$C_1-C_{10}$ alkylene-NH-, $-C_3-C_8$ heterocyclyl-NH-, $-C_1-C_{10}$ alkylene-($C_3-C_8$ heterocyclyl)-NH-, -($C_3-C_8$ heterocyclyl)-$C_1-C_{10}$ alkylene-NH-, $-C_1-C_{10}$ alkylene-S-, $-C_1-C_{10}$ heteroalkylene-S-, $-C_3-C_8$ carbocyclyl-S-, -O-($C_1-C_8$ alkylene)-S-, -arylene-S-, $-C_1-C_{10}$ alkylene-arylene-S-, -arylene-$C_1-C_{10}$ alkylene-S-, $-C_1-C_{10}$ alkylene-($C_3-C_8$ carbocyclyl)-S-, -($C_3-C_8$ carbocyclyl)-$C_1-C_{10}$ alkylene-S-, $-C_3-C_8$ heterocyclyl-S-, $-C_1-C_{10}$ alkylene-($C_3-C_8$ heterocyclyl)-S-, or -($C_3-C_8$ heterocyclyl)-$C_1-C_{10}$ alkylene-S-;
wherein, A is optionally substituted with a basic unit (BU), wherein the basic unit is $-(CH_2)_xNH_2$, - $(CH_2)_xNHR^a$, or $-(CH_2)_xNR^a{}_2$; wherein x is any integer from 1 to 4; each $R^a$ is independently selected from $C_{1-}C_6$ alkyl and $C_{1-}C_6$ haloalkyl, or two $R^a$ groups, together with nitrogen attached thereto, form a 4- to 6-membered heterocycloalkyl ring, or an azetidinyl, pyrrolidinyl or piperidinyl group.

In certain embodiments, A is selected from the following structure: $-(C_1-C_5)$alkylene-C(=O)-, wherein the alkylene moiety of A is optionally substituted with the basic unit (BU).
For example, Z may be -(succinimid-3-yl-N)-, A may be $-(C_1-C_{10})$alkylene-C(=O)-, Z-A may comprise the following structure:

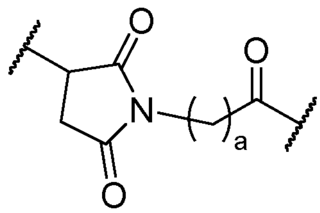

,

wherein a is any integer from 1 to 8. For example, a may be any integer from 2 to 5.

For another example, Z-A- may have the following structure:

-(succinimid-3-yl-N)-$CH_2CH_2$-C(=O)-,
-(succinimid-3-yl-N)-$CH_2CH_2CH_2$-C(=O)-,
-(succinimid-3-yl-N)-$CH_2CH_2CH_2CH_2$-C(=O)-,
-(succinimid-3-yl-N)-$CH_2CH_2CH_2CH_2CH_2$-C(=O)-,
-$CH_2$C(=O)NH-$CH_2$-C(=O)-,
-$CH_2$C(=O)NH-$CH_2CH_2$-C(=O)-,
-$CH_2$C(=O)NH-$CH_2CH_2CH_2$-C(=O)-,
-$CH_2$C(=O)NH-$CH_2CH_2CH_2CH_2$-C(=O)-,
-$CH_2$C(=O)NH-$CH_2CH_2CH_2CH_2CH_2$-C(=O)-,
-C(=O)-cyc.Hex(1,4)-$CH_2$-(N-ly-3-diminiccuS)-,
-C(=O)-Aryl-$CH_2$-(N-ly-3-diminiccuS)-,
-C(=O)-cyc.Het-$CH_2$-(N-ly-3-diminiccuS)-,
-C(=O)-$CH_2CH_2$-C(=O)-,
-C(=O)-$CH_2CH_2CH_2$-C(=O)-,
-C(=O)-$CH_2CH_2CH_2CH_2$-C(=O)-,
-C(=O)-$CH_2CH_2CH_2CH_2CH_2$-C(=O)-,
-C(=O)-$CH_2CH_2CH_2CH_2CH_2CH_2$-C(=O)-, or the like,

(Aryl represents a divalent aromatic hydrocarbyl, cyc.Het represents a divalent cyclic heterocyclyl). In certain embodiments, Y comprises a structure represented by -NH-$(CH_2)n^1$-La-Lb-Lc-, wherein La represents -C(=O)-NH-, -$NR^1$-$(CH_2)n^2$-, -O-, or a single bond, $R^1$ represents H, $C_1$-$C_6$ alkyl, $C_1$-$C_6$ alkylene-carboxyl, or $C_1$-$C_6$ alkylene-hydroxyl;

Lb represents -$CR^2(-R^3)$- or a single bond, wherein $R^2$ and $R^3$ each independently represents H, -$NH_2$, $C_1$-$C_6$ alkylene, $C_1$-$C_6$ alkylene-amino, $C_1$-$C_6$ alkylene-carboxyl, or $C_1$-$C_6$ alkylene-hydroxyl, and $R^2$ and $R^3$ are not both -$NH_2$;

$n^1$ represents an integer from 0 to 6;

Lc represents -$CH_2$- or -C(=O)-.

In certain embodiments, Y comprises NH-$(CH_2)n^1$-O-$(CH_2)n^2$-C(=O)-, wherein $n^1$ or $n^2$ is each independently any integer from 0 to 5. The spacer may be a functional group that promotes the attachment of RL to a drug unit, or can provide an additional structural component (e.g., a self-immolating p-aminobenzyl (PAB) component) to further promote the release of the drug unit from the remainder of the conjugate.

In certain embodiments, Y comprises -NH-$CH_2$-C(=O)-, -NH-$CH_2CH_2$-C(=O)-, -NH-$(CH_2)_3$-C(=O)-, -NH-$(CH_2)_4$-C(=O)-, -NH-$(CH_2)_5$-C(=O)-, -NH-$CH_2$-O-$CH_2$-C(=O)-, or -NH-$(CH_2)_2$-O-$CH_2$-C(=O)-.

For example, Y may comprise the following structure:

-NH-$CH_2$-,
-NH-CH(-Me)-,
-NH-C$(-Me)_2$-,
-NH-$CH_2$-CHMe-,
-NH-CH(-$CH_2$OH)-,
-NH-CH(-$CH_2$COOH)-,
-NH-CH(-$CH_2CH_2$COOH)-,
-NH-CH(-$CH_2CH_2CH_2CH_2NH_2$)-,
-NH-$CH_2CH_2$-,
-NH-$CH_2$-O-$CH_2$-,
-NH-$CH_2CH_2$-O-,

$-NH-CH_2CH_2-O-CH_2-$,
$-NH-CH_2CH_2C(-Me)_2-$,
$-NH-CH_2CH_2NH-$,
$-NH-CH_2CH_2NH-CH_2-$,
$-NH-CH_2CH_2NMe-CH_2-$,
$-NH-CH_2CH_2NH-CH_2CH_2-$,
$-NH-CH_2CH_2NMe-CH_2CH_2-$,
$-NH-CH_2CH_2N(-CH_2COOH)-CH_2-$,
$-NH-CH_2CH_2N(-CH_2CH_2OH)-CH_2-$,
$-NH-CH_2CH_2N(-CH_2CH_2OH)-CH_2CH_2-$,
$-NH-CH_2CH_2CH_2C(=O)-NHCH(-CH_2OH)-$,
$-NH-CH_2CH_2CH_2C(=O)-NHCH(-CH_2COOH)-$,
$-NH-CH_2CH_2CH_2C(=O)-NHCH(-CH_2CH_2CH_2CH_2NH_2)-$,
$-NH-CH_2CH_2CH_2-$,
$-NH-CH_2CH_2CH_2CH_2-$,
$-NH-CH_2CH_2CH_2CH_2CH_2-$,
$-NH-CH_2CH_2CH_2CH_2CH(NH_2)-$, or the like.

In certain embodiments, S* is a bond, and the linker is -Z-A-RL- or -Z-A-RL-Y-.
In certain embodiments, S* is a partitioning agent, and the linker is -Z-A-S*-RL- or -Z-A-S*-RL-Y-. The partitioning agent moiety may be used, for example, to mask the hydrophobicity of a drug unit or other linking unit components.
An exemplary partitioning agent may comprise a polyethylene glycol (PEG) unit, a cyclodextrin unit, polyamide, a hydrophilic peptide, polysaccharide, and dendrimer. When the linker comprises a polyethylene glycol (PEG) unit, a cyclodextrin unit, polyamide, a hydrophilic peptide, polysaccharide, or dendrimer, the groups may be present as an "in line" component or as a side chain or branched component.
In certain embodiments, S* comprises a PEG unit.
In certain embodiments, S* has the following formula:

$-NH-(CH_2CH_2O)_b-CH_2CH_2C(=O)-$;
$-NH-(CH_2CH_2O)_b-CH_2CH_2C(=O)NH-(CH_2CH_2O)-CH_2CH_2C(=O)-$; or
$-NH-(CH_2CH_2O)_b-CH_2CH_2NH-(CH_2CH_2O)-CH_2CH_2C(=O)-$;

wherein S* is connected to A on the left side and connected to RL on the right side, and b is any integer from 2 to 20. For example, b may be 2, 4, 8, or 12. In some embodiments, subscript b is 2. In some embodiments, subscript b is 4. In some embodiments, subscript b is 8. In some embodiments, subscript b is 12.
For example, the linker may be selected from the following structures:

-(succinimid-3-yl-N)-$CH_2CH_2-C(=O)$-RL-$NH-CH_2CH_2-C(=O)-$;
-(succinimid-3-yl-N)-$CH_2CH_2-C(=O)$-RL-$NH-CH_2CH_2CH_2-C(=O)-$;
-(succinimid-3-yl-N)-$CH_2CH_2CH_2CH_2CH_2-C(=O)$-RL-$NH-CH_2CH_2-C(=O)-$;
-(succinimid-3-yl-N)-$CH_2CH_2CH_2CH_2CH_2-C(=O)$-RL-$NH-CH_2CH_2CH_2-C(=O)-$;
-(succinimid-3-yl-N)-$CH_2CH_2CH_2CH_2CH_2-C(=O)$-RL-$NH-CH_2CH_2CH_2CH_2CH_2-C(=O)-$;
-(succinimid-3-yl-N)-$CH_2CH_2CH_2CH_2CH_2-C(=O)$-RL-$NH-CH_2-O-CH_2-C(=O)-$;
-(succinimid-3-yl-N)-$CH_2CH_2CH_2CH_2CH_2-C(=O)$-RL-$NH-CH_2CH_2-O-CH_2-C(=O)-$;
-(succinimid-3-yl-N)-$CH_2CH_2-C(=O)-NH-CH_2CH_2O-CH_2CH_2O-CH_2CH_2-C(=O)$-RL-$NH-CH_2CH_2CH_2-C(=O)-$;
-(succinimid-3-yl-N)-$CH_2CH_2-C(=O)-NH-CH_2CH_2O-CH_2CH_2O-CH_2CH_2-C(=O)$-RL-$NH-CH_2CH_2-C(=O)-$;
-(succinimid-3-yl-N)-$CH_2CH_2-C(=O)-NH-CH_2CH_2O-CH_2CH_2O-CH_2CH_2O-CH_2CH_2O-CH_2CH_2-C(=O)$-RL-$NH-CH_2CH_2CH_2-C(=O)-$;
-(succinimid-3-yl-N)-$CH_2CH_2-C(=O)-NH-CH_2CH_2O-CH_2CH_2O-CH_2CH_2O-CH_2CH_2O-CH_2CH_2-C(=O)$-RL-$NH-CH_2CH_2-C(=O)-$;
$-CH_2-C(=O)-NH-CH_2CH_2-C(=O)$-RL-$NH-CH_2CH_2CH_2-C(=O)-$; or
$-C(=O)-CH_2CH_2CH_2CH_2CH_2CH_2-C(=O)$-RL-$NH-CH_2CH_2CH_2-C(=O)-$;

wherein,

-(succinimid-3-yl-N)- has the following structure:

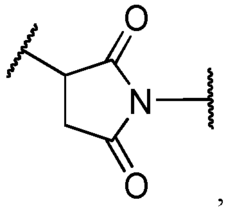

,

wherein the wavy line at position 3 of the structure represents an attachment position to a ligand unit, the wavy line at the N atom of position 1 represents an attachment position to A;
-RL- represents the following structure: -Val-Ala-Gly-Phe-Gly- (VAGFG), -Val-Ala-Ser-Gly-Phe-Gly- (VASGFG), or -Val-Ala-Ser-Ser-Gly-Phe-Gly- (VASSGFG).

For another example, the linker may have the following structure:

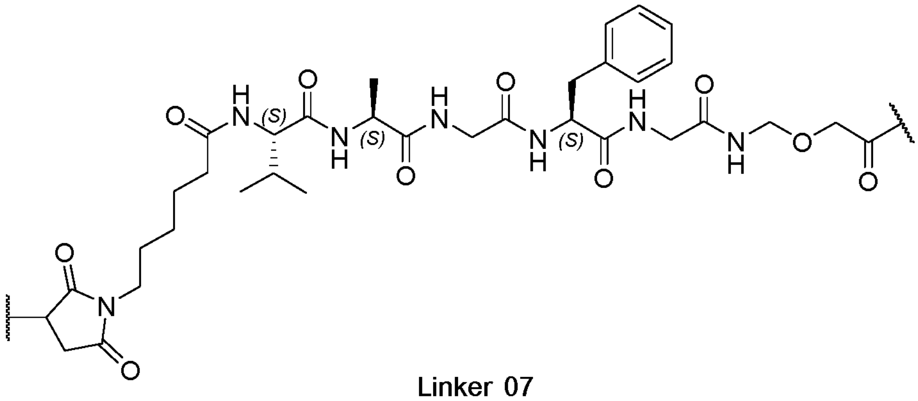

Linker 07

,

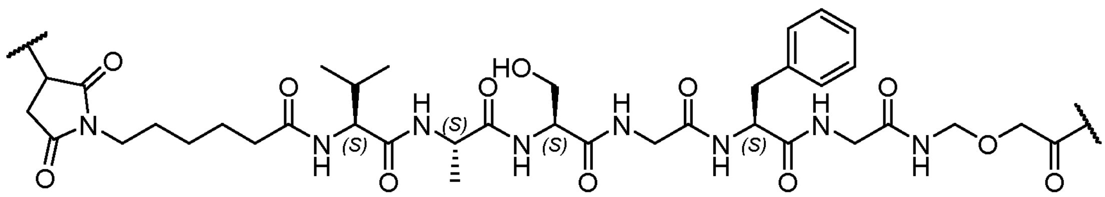

Linker 8

,

or

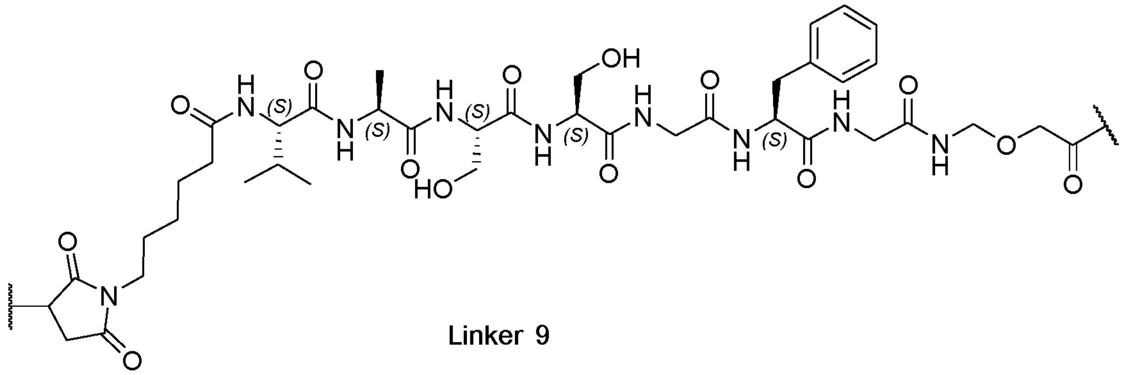

Linker 9

.

Linker-drug (linker-payload)

In another aspect, the present application provides a linker-drug comprising the linker as described herein.

In certain embodiments, the linker-drug is selected from the following structures: Z'-A-S*-RL-D and -Z'-A-S*-RL-Y-D; wherein, Z' is an extender unit precursor; A is a bond or an adapter unit; S* is a bond or a partitioning agent; Y is a spacer unit; D is a drug unit.

In certain embodiments, the extender unit precursor Z' is selected from (maleimid-N-yl)-, (pyrrolidine-2,5-dione-N-yl)-, M-$CH_2$-C(=O)-NH-, and HS-$(CH_2)_{1-5}$-C(=O)-, wherein M is halogen. In certain embodiments, the drug unit comprises a cytotoxic agent.

In certain embodiments, the drug unit comprises: amanitins, anthracyclines, auristatins, baccatins, calicheamicins, camptothecins, cemadotins, colchicines, colcimids, combretastatins, cryptophycins, discodermolides, duocarmycins, docetaxel, doxorubicin, duocarmycins, echinomycins, eleutherobins, epothilones, estramustines, lexitropsins, maytansines, maytansinoids, methotrexate, netropsins, pyrrolo[2,1-c][1,4]benzodiazepines (PBDs), puromycins, rhizoxins, SN-38, taxanes, tubulysins, vinca alkaloids, or derivatives thereof.

In certain embodiments, the drug unit comprises a DNA topoisomerase I inhibitor.

In certain embodiments, the drug unit comprises camptothecin or a derivative thereof.

In certain embodiments, the drug unit has the following structure:

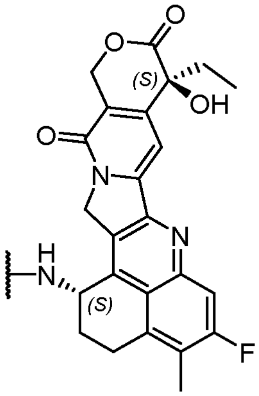

Dxd

wherein the nitrogen atom of the amino group at position 1 is a group at an attachment position.

In certain embodiments, the linker-drug unit has the following structure:

(maleimid-N-yl)-$CH_2CH_2$-C(=O)-RL-NH-$CH_2CH_2$-C(=O)-Dxd;
(maleimid-N-yl)-$CH_2CH_2$-C(=O)-RL-NH-$CH_2CH_2CH_2$-C(=O)-Dxd;
(maleimid-N-yl)-$CH_2CH_2CH_2CH_2CH_2$-C(=O)-RL-NH-$CH_2CH_2$-C(=O)-Dxd;
(maleimid-N-yl)-$CH_2CH_2CH_2CH_2CH_2$-C(=O)-RL-NH-$CH_2CH_2CH_2$-C(=O)-Dxd;
(maleimid-N-yl)-$CH_2CH_2CH_2CH_2CH_2$-C(=O)-RL-NH-$CH_2CH_2CH_2CH_2CH_2$-C(=O)-Dxd;
(maleimid-N-yl)-$CH_2CH_2CH_2CH_2CH_2$-C(=O)-RL-NH-$CH_2$-O-$CH_2$-C(=O)-Dxd;
(maleimid-N-yl)-$CH_2CH_2CH_2CH_2CH_2$-C(=O)-RL-NH-$CH_2CH_2$-O-$CH_2$-C(=O)-Dxd;
(maleimid-N-yl)-$CH_2CH_2$-C(=O)-NH-$CH_2CH_2O$-$CH_2CH_2O$-$CH_2CH_2$-C(=O)-RL-NH-$CH_2CH_2CH_2$-C(=O)-Dxd;
(maleimid-N-yl)-$CH_2CH_2$-C(=O)-NH-$CH_2CH_2O$-$CH_2CH_2O$-$CH_2CH_2$-C(=O)-RL-NH-$CH_2CH_2$-C(=O)-Dxd;
(maleimid-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-Dxd;
(maleimid-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2$-C(=O)-Dxd;
(pyrrolidine-2,5-dione-N-yl)-$CH_2CH_2$-C(=O)-RL-NH-$CH_2CH_2$-C(=O)-Dxd;
(pyrrolidine-2,5-dione-N-yl)-$CH_2CH_2$-C(=O)-RL-NH-$CH_2CH_2CH_2$-C(=O)-Dxd;
(pyrrolidine-2,5-dione-N-yl)-$CH_2CH_2CH_2CH_2CH_2$-C(=O)-RL-NH-$CH_2CH_2$-C(=O)-Dxd;
(pyrrolidine-2,5-dione-N-yl)-$CH_2CH_2CH_2CH_2CH_2$-C(=O)-RL-NH-$CH_2CH_2CH_2$-C(=O)-Dxd;
(pyrrolidine-2,5-dione-N-yl)-$CH_2CH_2CH_2CH_2CH_2$-C(=O)-RL-NH-$CH_2CH_2CH_2CH_2CH_2$-C(=O)-Dxd;
(pyrrolidine-2,5-dione-N-yl)-$CH_2CH_2CH_2CH_2CH_2$-C(=O)-RL-NH-$CH_2$-O-$CH_2$-C(=O)-Dxd;
(pyrrolidine-2,5-dione-N-yl)-$CH_2CH_2CH_2CH_2CH_2$-C(=O)-RL-NH-$CH_2CH_2$-O-$CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2$-C(=O)-RL-NH-

$CH_2CH_2CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2$-C(=O)-RL-NH-$CH_2CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2$-C(=O)-Dxd;
M-$CH_2$-C(=O)-NH-$CH_2CH_2$-C(=O)-RL-NH-$CH_2CH_2$-C(=O)-Dxd;
M-$CH_2$-C(=O)-NH-$CH_2CH_2$-C(=O)-RL-NH-$CH_2CH_2CH_2$-C(=O)-Dxd;
M-$CH_2$-C(=O)-NH-$CH_2CH_2$-C(=O)-RL-NH-$CH_2$-O-$CH_2$-C(=O)-Dxd;
M-$CH_2$-C(=O)-NH-$CH_2CH_2$-C(=O)-RL-NH-$CH_2CH_2$-O-$CH_2$-C(=O)-Dxd;
M-$CH_2$-C(=O)-NH-$CH_2CH_2CH_2$-C(=O)-RL-NH-$CH_2CH_2$-C(=O)-Dxd;
M-$CH_2$-C(=O)-NH-$CH_2CH_2CH_2$-C(=O)-RL-NH-$CH_2CH_2CH_2$-C(=O)-Dxd;
M-$CH_2$-C(=O)-NH-$CH_2CH_2CH_2$-C(=O)-RL-NH-$CH_2$-O-$CH_2$-C(=O)-Dxd;
M-$CH_2$-C(=O)-NH-$CH_2CH_2CH_2$-C(=O)-RL-NH-$CH_2CH_2$-O-$CH_2$-C(=O)-Dxd;
M-$CH_2$-C(=O)-NH-$CH_2CH_2CH_2CH_2$-C(=O)-RL-NH-$CH_2CH_2$-C(=O)-Dxd;
M-$CH_2$-C(=O)-NH-$CH_2CH_2CH_2CH_2$-C(=O)-RL-NH-$CH_2CH_2CH_2$-C(=O)-Dxd;
M-$CH_2$-C(=O)-NH-$CH_2CH_2CH_2CH_2$-C(=O)-RL-NH-$CH_2$-O-$CH_2$-C(=O)-Dxd;
M-$CH_2$-C(=O)-NH-$CH_2CH_2CH_2CH_2$-C(=O)-RL-NH-$CH_2CH_2$-O-$CH_2$-C(=O)-Dxd;
M-$CH_2$-C(=O)-NH-$CH_2CH_2CH_2CH_2CH_2$-C(=O)-RL-NH-$CH_2CH_2$-C(=O)-Dxd;
M-$CH_2$-C(=O)-NH-$CH_2CH_2CH_2CH_2CH_2$-C(=O)-RL-NH-$CH_2CH_2CH_2$-C(=O)-Dxd;
M-$CH_2$-C(=O)-NH-$CH_2CH_2CH_2CH_2CH_2$-C(=O)-RL-NH-$CH_2$-O-$CH_2$-C(=O)-Dxd;
M-$CH_2$-C(=O)-NH-$CH_2CH_2CH_2CH_2CH_2$-C(=O)-RL-NH-$CH_2CH_2$-O-$CH_2$-C(=O)-Dxd;
HS-$CH_2CH_2$-C(=O)-RL-NH-$CH_2CH_2$-C(=O)-Dxd;
HS-$CH_2CH_2CH_2$-C(=O)-RL-NH-$CH_2CH_2$-C(=O)-Dxd;
HS-$CH_2CH_2CH_2CH_2$-C(=O)-RL-NH-$CH_2CH_2$-C(=O)-Dxd;
HS-$CH_2CH_2CH_2CH_2CH_2$-C(=O)-RL-NH-$CH_2CH_2$-C(=O)-Dxd;
HS-$CH_2CH_2$-C(=O)-RL-NH-$CH_2CH_2CH_2$-C(=O)-Dxd;
HS-$CH_2CH_2CH_2$-C(=O)-RL-NH-$CH_2CH_2CH_2$-C(=O)-Dxd;
HS-$CH_2CH_2CH_2CH_2$-C(=O)-RL-NH-$CH_2CH_2CH_2$-C(=O)-Dxd;
HS-$CH_2CH_2CH_2CH_2CH_2$-C(=O)-RL-NH-$CH_2CH_2CH_2$-C(=O)-Dxd;
HS-$CH_2CH_2$-C(=O)-RL-NH-$CH_2$-O-$CH_2$-C(=O)-Dxd;
HS-$CH_2CH_2CH_2$-C(=O)-RL-NH-$CH_2$-O-$CH_2$-C(=O)-Dxd;
HS-$CH_2CH_2CH_2CH_2$-C(=O)-RL-NH-$CH_2$-O-$CH_2$-C(=O)-Dxd;
HS-$CH_2CH_2CH_2CH_2CH_2$-C(=O)-RL-NH-$CH_2$-O-$CH_2$-C(=O)-Dxd;
HS-$CH_2CH_2$-C(=O)-RL-NH-$CH_2CH_2$-O-$CH_2$-C(=O)-Dxd;
HS-$CH_2CH_2CH_2$-C(=O)-RL-NH-$CH_2CH_2$-O-$CH_2$-C(=O)-Dxd;
HS-$CH_2CH_2CH_2CH_2$-C(=O)-RL-NH-$CH_2CH_2$-O-$CH_2$-C(=O)-Dxd;
HS-$CH_2CH_2CH_2CH_2CH_2$-C(=O)-RL-NH-$CH_2CH_2$-O-$CH_2$-C(=O)-Dxd;
HS-$CH_2CH_2$-C(=O)-NH-$CH_2CH_2O$-$CH_2CH_2O$-$CH_2CH_2$-C(=O)-RL-NH-$CH_2CH_2CH_2$-C(=O)-Dxd;
HS-$CH_2CH_2$-C(=O)-NH-$CH_2CH_2O$-$CH_2CH_2O$-$CH_2CH_2O$-$CH_2CH_2$-C(=O)-RL-NH-$CH_2CH_2CH_2$-C(=O)-Dxd; or
HS-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-Dxd;

wherein,

(maleimid-N-yl)- has the following structure: attachment position;

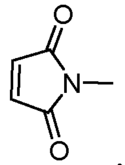
,

wherein the nitrogen atom is a group at an
(pyrrolidine-2,5-dione-N-yl)- has the following structure:

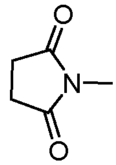,

wherein the nitrogen atom is a group at an attachment position;
-RL- represents the following structure: -Val-Ala-Gly-Phe-Gly- (VAGFG), -Val-Ala-Ser-Gly-Phe-Gly- (VASGFG), or -Val-Ala-Ser-Ser-Gly-Phe-Gly- (VASSGFG);
Dxd has the following structure:

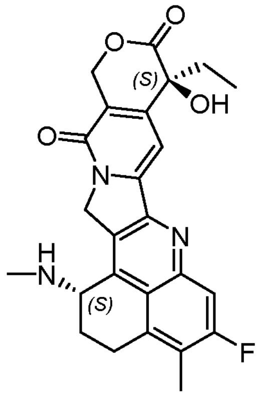,

wherein the nitrogen atom of the amino group at position 1 is a group at an attachment position;
M represents a halogen atom.

In certain embodiments, the linker-drug unit is selected from the following structures:

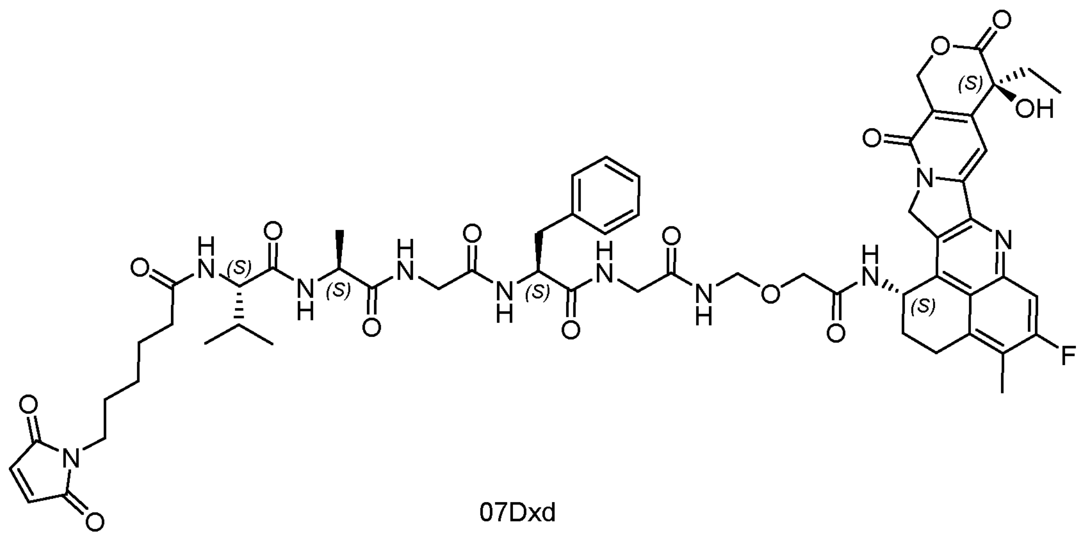

07Dxd

;

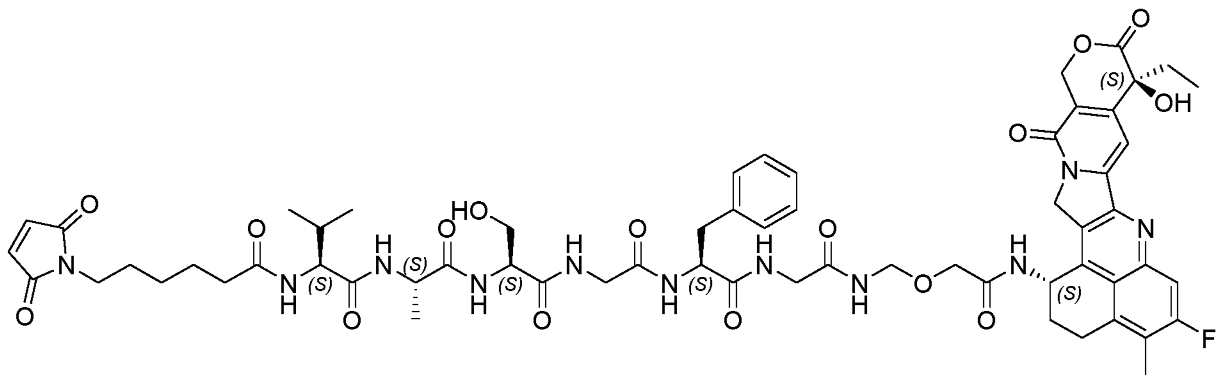

08Dxd

;

and

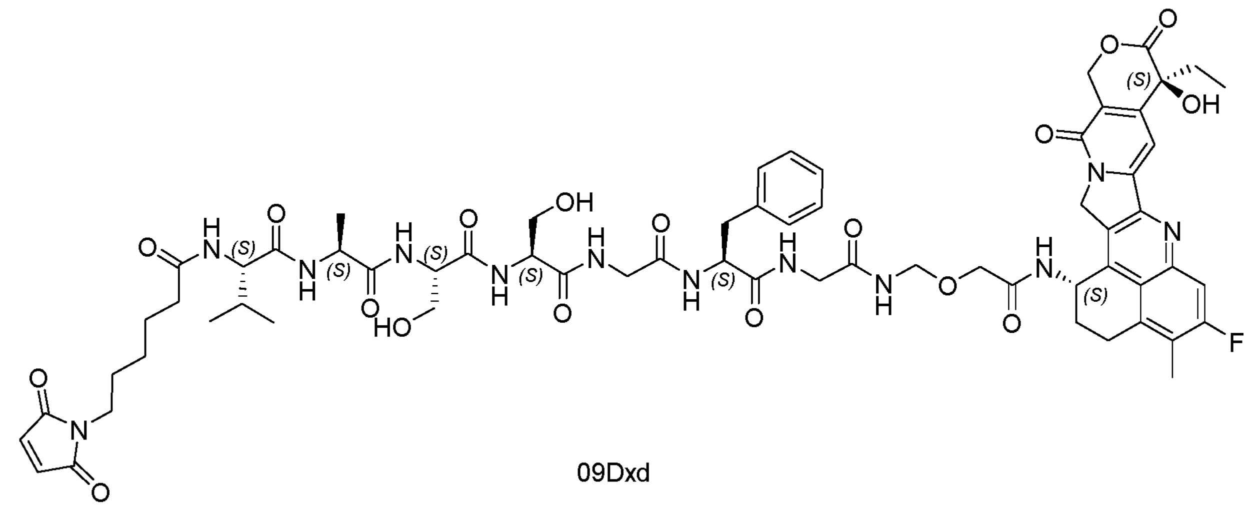

09Dxd

.

Ligand unit

In some embodiments of the present application, a ligand unit is present. The ligand unit (C) is a targeting agent that specifically binds to a target moiety. In some embodiments, the ligand unit specifically and selectively binds to a cellular component (a cell engager) or other target molecule of interest. The role of the ligand unit is to target and delivery a drug unit (e.g., Dxd) to a specific target cell population that interacts with the ligand unit due to the presence of the target component or molecule thereof and allows for subsequent release of the free drug within (i.e., intracellularly) or nearby (i.e., extracellularly) the target cells. The ligand unit C includes, but is not limited to, proteins, polypeptides, and peptides.

Suitable ligand units include, for example, antibodies, e.g., full-length antibodies and antigen-binding fragments thereof, interferons, lymphokines, hormones, growth factors and colony-stimulating factors, vitamins, nutrient transporters (such as, but not limited to, transferrin), or any other cell-binding molecule or substance. In some embodiments, the ligand unit (C) is an antibody or a non-antibody protein-targeting agent.

In some embodiments, the ligand unit is bonded to L (linker unit) comprising a releasable peptide linker. As described above, in the conjugate described herein, other linking components may be present to provide additional space between the drug unit and the ligand unit (e.g., an extender unit and optionally an adapter unit A) or to provide composition properties so as to increase solubility (e.g., a partitioning agent S*). In some of the embodiments, the ligand unit is bonded to Z of the linker unit via a heteroatom of the ligand unit. Heteroatoms that may be present in the ligand unit for this bonding include sulfur (in one embodiment, from a sulfydryl group of the targeting ligand), oxygen (in one embodiment, from a carboxyl or hydroxyl group of the targeting ligand), and nitrogen (in one embodiment, from a primary or secondary amine functional group of the targeting ligand, or in another embodiment, from amide nitrogen optionally substituted), optionally substituted. These heteroatoms may be present in the targeting ligand in the natural state of the

ligand (e.g., in a naturally occurring antibody), or may be introduced into the targeting ligand via chemical modification or bioengineering.

In one embodiment, the ligand unit has a sulfydryl functional group, such that the ligand unit is bonded to the linker unit via the sulfur atom of the sulfydryl functional group.

In another embodiment, the ligand unit has one or more lysine residues, which can react with an activated ester of the extender unit precursor of a camptothecin-linker compound intermediate (such ester includes, but is not limited to, *N*-hydroxysuccinimide ester, pentafluorophenyl ester, and *p*-nitrophenyl ester) and thereby provide an amide bond consisting of the nitrogen atom from the ligand unit and the C=O group from the extender unit of the linker unit.

In some aspects, the ligand unit can form a covalent bond between the extender unit (Z) and the ligand unit corresponding to the targeting ligand by interacting with a reactive functional group of the extender unit precursor (Z'). The functional group of Z' that can interact with the targeting ligand depends on the nature of the ligand unit. In some embodiments, the reactive group is maleimide that is present in the extender unit (i.e., the maleimide moiety of the extender unit precursor) prior to the attachment to form the ligand unit. The covalent attachment of the ligand unit to the extender unit is achieved by the interaction of the sulfydryl functional group of the ligand unit with the maleimide functional group of Z' to form sulfur-substituted succinimide. The sulfydryl functional group may be present in the ligand unit in the natural state of the ligand (e.g., in a naturally occurring residue), or may be introduced into the ligand unit via chemical modification or bioengineering.

In yet another embodiment, the ligand unit is an antibody, and the sulfydryl group is formed by the reduction of an inter-chain disulfide of the antibody. Correspondingly, in some embodiments, the linker unit is conjugated to a cysteine residue from the reduced inter-chain disulfide.

In yet another embodiment, the ligand unit is an antibody, and the sulfydryl functional group is chemically introduced into the antibody, for example, by the introduction of a cysteine residue. Correspondingly, in some embodiments, the linker unit (with or without an attached drug unit) is conjugated to the ligand unit via the introduced cysteine residue of the ligand unit.

In some embodiments, the ligand targets a cell surface receptor or a tumor-associated antigen.

In some embodiments, the cell surface receptor or tumor-associated antigen may comprise HER2, HER3, claudin18.2, folate receptor alpha (FR$\alpha$), BCMA, PSMA, TROP-2, CD19, CD20, CD22, CD30, CD79b, EGFR, c-Met, or CEACAM5.

## Antibody-drug conjugate (ADC)

In another aspect, the present application provides an antibody-drug conjugate (ADC) having the following structure: Ab-(L-D)$_m$, wherein, Ab represents an antibody or an antigen-binding fragment thereof, L represents a linker, D represents a drug unit, m represents any number from 1 to 10.

In certain embodiments, the antibody comprises a monoclonal antibody, a polyclonal antibody, a dimer, a multimer, a multispecific antibody, an intact antibody, an antibody fragment, a human antibody, a humanized antibody, or a chimeric antibody.

In certain embodiments, the antigen-binding fragment comprises a Fab, a Fab', an Fv fragment, an F(ab')2, an scFv, a di-scFv, and/or a dAb.

In certain embodiments, the antibody is a monoclonal antibody.

In certain embodiments, the antibody is a human antibody, a humanized antibody, or a chimeric antibody.

In certain embodiments, the drug unit comprises a cytotoxic agent.

For example, the drug unit may be a microtubule-interfering drug, such as auristatins, like monomethyl auristatin E (MMAE), monomethyl auristatin F (MMAF), and auristatin F (AF). For another example, the drug unit may be a microtubule-disrupting drug, such as maytansinoids, like DM1, DM3, and DM4. For another example, the drug unit may be a DNA-damaging agent, such as calicheamicins, duocarmycins, SN-38, and pyrrolo[2,1-c][1,4]benzodiazepines (PBDs). For another example, the drug unit may be amanitins, anthracyclines, baccatins, camptothecins, cemadotins, colchicines, colcimids, combretastatins, cryptophycins, discodermolides, docetaxel, doxorubicin, echinomycins, eleutherobins, epothilones, estramustines, lexitropsins, maytansines, methotrexate, netropsins, puromycins, rhizoxins, taxanes, tubulysins, or vinca alkaloids.

In certain embodiments, the drug unit is a topoisomerase I inhibitor, such as camptothecin derivative Dxd.

In certain embodiments, the antibody-drug conjugate is selected from the following structures:

Ab-[-(succinimid-3-yl-N)-$CH_2CH_2$-C(=O)-RL-NH-$CH_2CH_2$-C(=O)-Dxd]$_m$;
Ab-[-(succinimid-3-yl-N)-$CH_2CH_2$-C(=O)-RL-NH-$CH_2CH_2CH_2$-C(=O)-Dxd]$_m$;
Ab-[-(succinimid-3-yl-N)-$CH_2CH_2CH_2CH_2CH_2$-C(=O)-RL-NH-$CH_2CH_2$-C(=O)-Dxd]$_m$;
Ab-[-(succinimid-3-yl-N)-$CH_2CH_2CH_2CH_2CH_2$-C(=O)-RL-NH-$CH_2CH_2CH_2$-C(=O)-Dxd]$_m$;
Ab-[-(succinimid-3-yl-N)-$CH_2CH_2CH_2CH_2CH_2$-C(=O)-RL-NH-$CH_2CH_2CH_2CH_2CH_2$-C(=O)-Dxd]$_m$;

Ab-[-(succinimid-3-yl-N)-$CH_2CH_2CH_2CH_2CH_2$-C(=O)-RL-NH-$CH_2$-O-$CH_2$-C(=O)-Dxd]$_m$;
Ab-[-(succinimid-3-yl-N)-$CH_2CH_2CH_2CH_2CH_2$-C(=O)-RL-NH-$CH_2CH_2$-O-$CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-RL-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-RL-NH-$CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2$-C(=O)-Dxd]$_m$;
Ab-[-(succinimid-3-yl-N)-$CH_2CH_2$-C(=O)-VASGFG-NH-$CH_2CH_2$-C(=O)-Dxd]$_m$;
Ab-[-(succinimid-3-yl-N)-$CH_2CH_2$-C(=O)-VASGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2CH_2CH_2CH_2$-C(=O)-Dxd]$_m$;
Ab-[-(succinimid-3-yl-N)-$CH_2CH_2CH_2CH_2CH_2$-C(=O)-VASGFG-NH-$CH_2$-O-$CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2$-O-$CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-VASGFG-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-VASGFG-NH-$CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VASGFG-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VASGFG-NH-$CH_2CH_2$-C(=O)-Dxd]$_m$;
Ab-[-(succinimid-3-yl-N)-$CH_2CH_2$-C(=O)-VASSGFG-NH-$CH_2CH_2$-C(=O)-Dxd]$_m$;
Ab-[-(succinimid-3-yl-N)-$CH_2CH_2$-C(=O)-VASSGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2CH_2CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2$-O-$CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2$-O-$CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-VASSGFG-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-VASSGFG-NH-$CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VASSGFG-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VASSGFG-NH-$CH_2CH_2$-C(=O)-Dxd]$_m$;
Ab-[-$CH_2$-C(=O)-NH-$CH_2CH_2$-C(=O)-RL-NH-$CH_2CH_2$-C(=O)-Dxd]$_m$;
Ab-[-$CH_2$-C(=O)-NH-$CH_2CH_2$-C(=O)-RL-NH-$CH_2CH_2CH_2$-C(=O)-Dxd]$_m$;
Ab-[-$CH_2$-C(=O)-NH-$CH_2CH_2$-C(=O)-RL-NH-$CH_2$-O-$CH_2$-C(=O)-Dxd]$_m$;
Ab-[-$CH_2$-C(=O)-NH-$CH_2CH_2$-C(=O)-RL-NH-$CH_2CH_2$-O-$CH_2$-C(=O)-Dxd]$_m$;
Ab-[-$CH_2$-C(=O)-NH-$CH_2CH_2CH_2$-C(=O)-RL-NH-$CH_2CH_2$-C(=O)-Dxd]$_m$;
Ab-[-$CH_2$-C(=O)-NH-$CH_2CH_2CH_2$-C(=O)-RL-NH-$CH_2CH_2CH_2$-C(=O)-Dxd]$_m$;
Ab-[-$CH_2$-C(=O)-NH-$CH_2CH_2CH_2$-C(=O)-RL-NH-$CH_2$-O-$CH_2$-C(=O)-Dxd]$_m$;
Ab-[-$CH_2$-C(=O)-NH-$CH_2CH_2CH_2$-C(=O)-RL-NH-$CH_2CH_2$-O-$CH_2$-C(=O)-Dxd]$_m$;
Ab-[-$CH_2$-C(=O)-NH-$CH_2CH_2CH_2CH_2$-C(=O)-RL-NH-$CH_2CH_2$-C(=O)-Dxd]$_m$;
Ab-[-$CH_2$-C(=O)-NH-$CH_2CH_2CH_2CH_2$-C(=O)-RL-NH-$CH_2CH_2CH_2$-C(=O)-Dxd]$_m$;
Ab-[-$CH_2$-C(=O)-NH-$CH_2CH_2CH_2CH_2$-C(=O)-RL-NH-$CH_2$-O-$CH_2$-C(=O)-Dxd]$_m$;
Ab-[-$CH_2$-C(=O)-NH-$CH_2CH_2CH_2CH_2$-C(=O)-RL-NH-$CH_2CH_2$-O-$CH_2$-C(=O)-Dxd]$_m$;
Ab-[-$CH_2$-C(=O)-NH-$CH_2CH_2CH_2CH_2CH_2$-C(=O)-RL-NH-$CH_2CH_2$-C(=O)-Dxd]$_m$;
Ab-[-$CH_2$-C(=O)-NH-$CH_2CH_2CH_2CH_2CH_2$-C(=O)-RL-NH-$CH_2CH_2CH_2$-C(=O)-Dxd]$_m$;
Ab-[-$CH_2$-C(=O)-NH-$CH_2CH_2CH_2CH_2CH_2$-C(=O)-RL-NH-$CH_2$-O-$CH_2$-C(=O)-Dxd]$_m$;
Ab-[-$CH_2$-C(=O)-NH-$CH_2CH_2CH_2CH_2CH_2$-C(=O)-RL-NH-$CH_2CH_2$-O-$CH_2$-C(=O)-Dxd]$_m$;
Ab-[-S-$CH_2CH_2$-C(=O)-RL-NH-$CH_2CH_2$-C(=O)-Dxd]$_m$;
Ab-[-S-$CH_2CH_2CH_2$-C(=O)-RL-NH-$CH_2CH_2$-C(=O)-Dxd]$_m$;

Ab-[-S-$CH_2CH_2CH_2CH_2$-C(=O)-RL-NH-$CH_2CH_2$-C(=O)-Dxd$]_m$;
Ab-[-S-$CH_2CH_2CH_2CH_2CH_2$-C(=O)-RL-NH-$CH_2CH_2$-C(=O)-Dxd$]_m$;
Ab-[-S-$CH_2CH_2$-C(=O)-RL-NH-$CH_2CH_2CH_2$-C(=O)-Dxd$]_m$;
Ab-[-S-$CH_2CH_2CH_2$-C(=O)-RL-NH-$CH_2CH_2CH_2$-C(=O)-Dxd$]_m$;
Ab-[-S-$CH_2CH_2CH_2CH_2$-C(=O)-RL-NH-$CH_2CH_2CH_2$-C(=O)-Dxd$]_m$;
Ab-[-S-$CH_2CH_2CH_2CH_2CH_2$-C(=O)-RL-NH-$CH_2CH_2CH_2$-C(=O)-Dxd$]_m$;
Ab-[-S-$CH_2CH_2$-C(=O)-RL-NH-$CH_2$-O-$CH_2$-C(=O)-Dxd$]_m$;
Ab-[-S-$CH_2CH_2CH_2$-C(=O)-RL-NH-$CH_2$-O-$CH_2$-C(=O)-Dxd$]_m$;
Ab-[-S-$CH_2CH_2CH_2CH_2$-C(=O)-RL-NH-$CH_2$-O-$CH_2$-C(=O)-Dxd$]_m$;
Ab-[-S-$CH_2CH_2CH_2CH_2CH_2$-C(=O)-RL-NH-$CH_2$-O-$CH_2$-C(=O)-Dxd$]_m$;
Ab-[-S-$CH_2CH_2$-C(=O)-RL-NH-$CH_2CH_2$-O-$CH_2$-C(=O)-Dxd$]_m$;
Ab-[-S-$CH_2CH_2CH_2$-C(=O)-RL-NH-$CH_2CH_2$-O-$CH_2$-C(=O)-Dxd$]_m$;
Ab-[-S-$CH_2CH_2CH_2CH_2$-C(=O)-RL-NH-$CH_2CH_2$-O-$CH_2$-C(=O)-Dxd$]_m$;
Ab-[-S-$CH_2CH_2CH_2CH_2CH_2$-C(=O)-RL-NH-$CH_2CH_2$-O-$CH_2$-C(=O)-Dxd$]_m$;
Ab-[-S-$CH_2CH_2$-C(=O)-NH-$CH_2CH_2$O-$CH_2CH_2$O-$CH_2CH_2$-C(=O)-RL-NH-$CH_2CH_2CH_2$-C(=O)-Dxd$]_m$;
Ab-[-S-$CH_2CH_2$-C(=O)-NH-$CH_2CH_2$O-$CH_2CH_2$O-$CH_2CH_2$O-$CH_2CH_2$-C(=O)-RL-NH-$CH_2CH_2CH_2$-C(=O)-Dxd$]_m$;
and
Ab-[-S-$CH_2CH_2$-C(=O)-NH-$CH_2CH_2$O-$CH_2CH_2$O-$CH_2CH_2$O-$CH_2CH_2$O-$CH_2CH_2$-C(=O)-RL-NH-$CH_2CH_2CH_2$-C(=O)-Dxd$]_m$;

wherein,

-(succinimid-3-yl-N)- has the following structure:

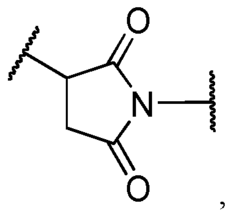,

wherein the wavy line at position 3 of the structure represents an attachment position to a ligand unit, the wavy line at the N atom of position 1 represents an attachment position to A;
-RL- represents the following structure: -Val-Ala-Gly-Phe-Gly- (VAGFG), -Val-Ala-Ser-Gly-Phe-Gly- (VASGFG), or -Val-Ala-Ser-Ser--Gly-Phe-Gly- (VASSGFG);
Dxd has the following structure:

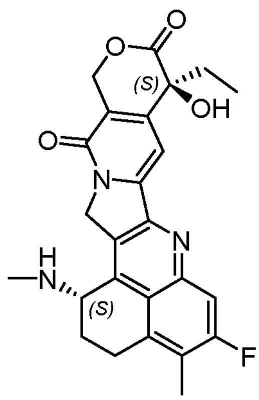,

wherein the nitrogen atom of the amino group at position 1 is a group at an attachment position;
Ab represents an antibody or an antigen-binding fragment thereof, m represents any number from 1 to 10.

In certain embodiments, the antibody-drug conjugate is selected from the following structures:

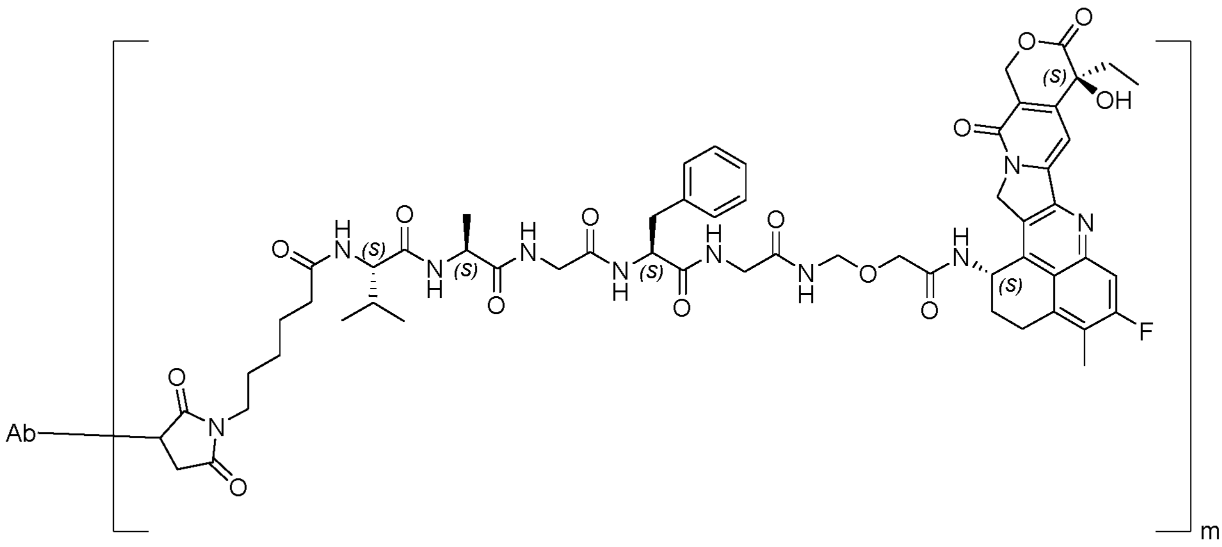

ADC07

;

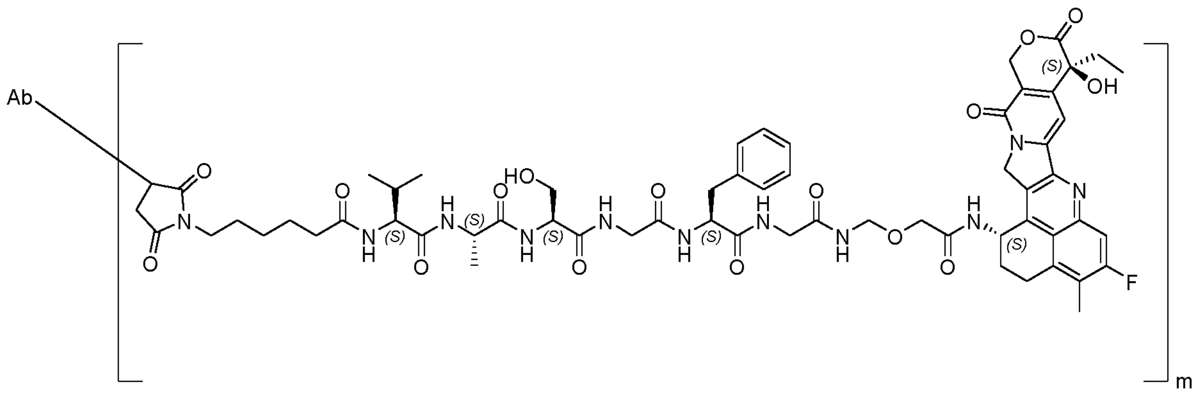

ADC08

;

and

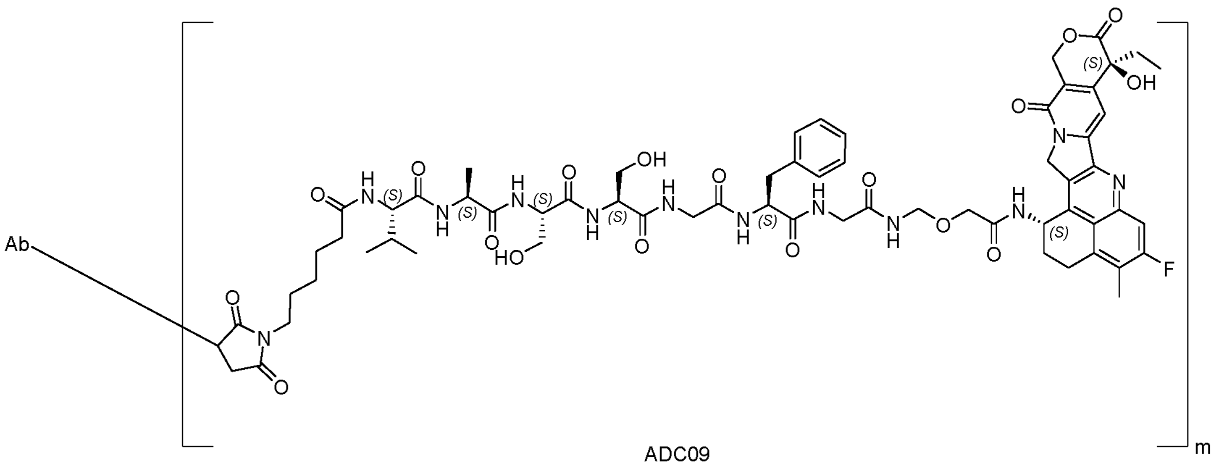

ADC09

;

wherein, Ab represents an antibody or an antigen-binding fragment thereof, m is any number from 1 to 10.

In certain embodiments, the antibody or the antigen-binding fragment thereof comprises: an anti-HER2 antibody,

an anti-HER3 antibody, an anti-claudin18.2 antibody, an anti-FRα antibody, or an antigen-binding fragment thereof.

The antibody-drug conjugate (ADC) of the present disclosure comprises ADCs having an anti-cancer activity. Specifically, an ADC comprises an antibody or an antigen-binding fragment (including an antigen-binding fragment thereof) binding (i.e., covalently conjugated via an adapter) to a drug unit (e.g., a cytotoxic agent), e.g., wherein, the cytotoxic agent has cytotoxicity or cytostatic effect when it does not bind to the antibody or antigen-binding fragment.

The ADCs of the present application can selectively deliver effective doses of cytotoxic agents or cytostatic agents to cancer cells or tumor tissues. The disclosed ADCs have been found to have potent cytotoxicity and/or cytostatic activity against cells expressing respective target antigens (e.g. HER2, HER3, claudin18.2, folate receptor alpha (FRα), CD138, EPHA2, MSLN, FOLH1, CDH6, CEACAM5, CFC1B, ENPP3, FOLR1, HAVCR1, KIT, MET, MUC16, SLC39A6, SLC44A4 or STEAP1). In some embodiments, the cytotoxicity and/or cytostatic activity of the ADC is dependent on the target antigen expression in the cells. In some embodiments, the disclosed ADCs are particularly effective in killing cancer cells expressing the target antigen while minimizing off-target killing. In some embodiments, the disclosed ADCs do not exhibit cytotoxicity and/or cytostatic effect on cancer cells not expressing the target antigen.

Examples of the types of cancers to which the anti-HER3 antibody-drug conjugate of the present invention may be applied include lung cancer, kidney cancer, urothelial cancer, colorectal cancer, prostate cancer, glioblastoma multiforme, ovarian cancer, pancreatic cancer, breast cancer, metastatic breast cancer, luminal breast cancer, melanoma, liver cancer, bladder cancer, gastric cancer (stomach cancer), gastrointestinal stromal tumor, cervical cancer, head and neck cancer, esophageal cancer, epidermoid cancer, peritoneal cancer, adult glioblastoma multiforme, hepatic cancer, hepatocellular cancer, colon cancer, rectal cancer, colon and rectal cancer, endometrial cancer, uterine cancer, salivary gland cancer, renal cancer, vulval cancer, thyroid cancer, hepatic carcinoma, anal cancer, or penile cancer. The treatment with the anti-HER3 antibody-drug conjugate of the present invention can target cancer cells expressing HER3 protein recognizable by the antibody of the antibody-drug conjugate in the cancer cells of a subject. In the present description, "cancer expressing HER3 protein" refers to the cancer having cells with HER3 protein on the surfaces or the cancer secreting HER3 protein into blood.

Examples of the types of cancers to which the anti-claudin18.2 antibody-drug conjugate of the present invention may be applied include gastrointestinal cancer, pancreatic cancer, esophageal cancer, non-small cell lung cancer, liver cancer, ovarian cancer, lung cancer, gallbladder cancer, or head and neck cancer.

Examples of the types of cancers to which the anti-FRα antibody-drug conjugate of the present invention may be applied include ovarian cancer, breast cancer, lung cancer, colorectal cancer, or kidney cancer.

Examples of the types of cancers to which the anti-HER2 antibody-drug conjugate of the present invention may be applied include, but are not limited to, breast cancer, gastric cancer, bladder cancer, urothelial cell carcinoma, esophageal cancer, lung cancer (e.g., lung adenocarcinoma), uterine cancer (e.g., uterine serous endometrial cancer), salivary gland cancer, cervical cancer, endometrial cancer, and ovarian cancer (English et al., (2013) Mol Diagn Ther. [Molecular Diagnosis & Therapy] 17: 85-99).

In the present application, the antibody may target a cell surface receptor or a tumor-associated antigen. In addition, the antibody may comprise a full-length antibody or an antibody fragment that binds to, reactively associates with, or complexes with receptors, antigens, or other target cell-associated moieties. The antibody may be any protein, proteinoid, or polypeptide that binds to, complexes with, or reacts with a portion of a cell population seeking therapeutic modification. In some embodiments, the antibody may be a chimeric antibody or a functionally active fragment thereof, a humanized antibody or a functionally active fragment thereof, or a human antibody or a functionally active fragment thereof.

In some embodiments, the antibody may also be a functionally active fragment, or a derivative or an analog of the antibody, wherein the antibody can immunospecifically bind to a target antigen (e.g., a cancer antigen, a viral antigen, a microbial antigen, or other antigen that can bind to a cell or matrix). Herein, "functionally active" means that the fragments, derivatives or analogs can recognize the same antigen, and recognize the antigen-derived fragments, derivatives or analogs. Other useful antibodies include fragments of antibodies, such as, but not limited to: F(ab')2 fragments, Fab fragments, Fab', Fv fragments, and heavy chain dimers and light chain dimers of antibodies, or Fv fragments or single chain antibodies (scFv). In other embodiments, the antibody may be a fusion protein of an antibody, or a functionally active fragment thereof.

The antibodies of the present application may include, but are not limited to, antibodies against the following antigens (exemplary cancer is indicated in parentheses): CA125 (ovarian cancer), CA15-3 (carcinomas), CA19-9 (carcinomas), L6 (carcinomas), Lewis Y (carcinomas), Lewis X (carcinomas), alpha-fetoprotein (carcinomas), CA 242 (colorectal cancer), placental alkaline phosphatase (carcinomas), prostate-specific membrane antigen (prostate cancer), prostatic acid phosphatase (prostate cancer), epidermal growth factor (carcinomas), MAGE-1 (carcinomas), MAGE-2 (carcinomas), MAGE-3 (carcinomas), MAGE-4 (carcinomas), anti-transferrin receptor (carcinomas), p97 (melanoma), MUC1-KLH (breast cancer), CEA (colorectal cancer), gp100 (melanoma), MART1 (melanoma), prostate-specific antigen (PSA) (prostate cancer), IL-2 receptor (T-cell leukemia and lymphoma), CD20 (non-Hodgkin lymphoma), CD52 (leukemia), CD33 (leukemia), CD22 (lymphoma), human chorionic gonadotropin (carcinomas), CD38 (multiple myeloma),

CD40 (lymphoma), mucin (carcinomas), P21 (carcinomas), MPG (melanoma), and Neu oncogene product (carcinomas).

The antibodies of the present application may also include analogs and derivatives modified or unmodified (i.e., by covalent linking to any molecule), so long as such covalent linking allows the antibody to retain its antigen-binding immunospecificity, including, but not limited to: antibody analogs and derivatives, including those that have been further modified, for example: by glycosylation, acetylation, PEGylation, phosphorylation, amidation, derivatization with known protecting/blocking groups, protease cleavage, linking to cellular antibody units or other proteins, and the like. Any of numerous chemical modifications can be achieved using known techniques, including but not limited to specific chemical cleavage, acetylation, formylation, metabolic synthesis in the presence of tunicamycin, and the like. In addition, the analogs or derivatives may comprise one or more unnatural amino acids. In some embodiments, the antibody may have a modification (e.g., substitution, deletion, or addition) in an amino acid residue that interacts with an Fc receptor.

In certain embodiments, the antibody or the antigen-binding fragment thereof includes: an anti-HER2 antibody, an anti-claudin18.2 antibody, an anti-FRα antibody, an anti-HER3 antibody, or an antigen-binding fragment thereof.

In one embodiment, the antibody is an anti-HER3 antibody comprising VH, wherein the VH comprises HCDR1, HCDR2, and HCDR3, wherein the HCDR1 comprises an amino acid sequence set forth in SEQ ID NO: 1, the HCDR2 comprises an amino acid sequence set forth in SEQ ID NO: 2, and the HCDR3 comprises an amino acid sequence set forth in SEQ ID NO: 3.

In certain embodiments, the anti-HER3 antibody comprises VH, wherein the VH comprises an amino acid sequence set forth in SEQ ID NO: 15.

In certain embodiments, the anti-HER3 antibody comprises an antibody heavy chain constant region. For example, the antibody heavy chain constant region may comprise a constant region derived from human IgG. For another example, the antibody heavy chain constant region may comprise a constant region derived from human IgG1, IgG2, IgG3, or IgG4.

In certain embodiments, the anti-HER3 antibody comprises an antibody heavy chain, wherein the antibody heavy chain comprises an amino acid sequence set forth in SEQ ID NO: 17.

In certain embodiments, the anti-HER3 antibody comprises VL, wherein the VL comprises LCDR1, LCDR2 and LCDR3, wherein the LCDR1 comprises an amino acid sequence set forth in SEQ ID NO: 4, the LCDR2 comprises an amino acid sequence set forth in SEQ ID NO: 5, and the LCDR3 comprises an amino acid sequence set forth in SEQ ID NO: 6.

In certain embodiments, the anti-HER3 antibody comprises VH and VL, wherein the VH comprises HCDR1, HCDR2, and HCDR3, and the VL comprises LCDR1, LCDR2, and LCDR3; wherein the HCDR1 comprises an amino acid sequence set forth in SEQ ID NO: 1, the HCDR2 comprises an amino acid sequence set forth in SEQ ID NO: 2, the HCDR3 comprises an amino acid sequence set forth in SEQ ID NO: 3, the LCDR1 comprises an amino acid sequence set forth in SEQ ID NO: 4, the LCDR2 comprises an amino acid sequence set forth in SEQ ID NO: 5, and the LCDR3 comprises an amino acid sequence set forth in SEQ ID NO: 6.

In certain embodiments, the anti-HER3 antibody comprises VL, wherein the VL comprises an amino acid sequence set forth in SEQ ID NO: 16.

In certain embodiments, the anti-HER3 antibody comprises VH and VL, wherein the VH comprises an amino acid sequence set forth in SEQ ID NO: 15, and the VL comprises an amino acid sequence set forth in SEQ ID NO: 16.

In certain embodiments, the anti-HER3 antibody comprises an antibody light chain constant region. For example, the antibody light chain constant region may comprise a human Igκ constant region or a human Igλ constant region.

In certain embodiments, the anti-HER3 antibody comprises an antibody light chain, wherein the antibody light chain comprises an amino acid sequence set forth in SEQ ID NO: 18.

In certain embodiments, the anti-HER3 antibody comprises an antibody heavy chain and an antibody light chain, wherein the antibody heavy chain comprises an amino acid sequence set forth in SEQ ID NO: 17, and the antibody light chain comprises an amino acid sequence set forth in SEQ ID NO: 18. In one embodiment, the antibody is an anti-Claudin18.2 antibody comprising VH, wherein the VH comprises HCDR1, HCDR2, and HCDR3, wherein the HCDR1 comprises an amino acid sequence set forth in SEQ ID NO: 19, the HCDR2 comprises an amino acid sequence set forth in SEQ ID NO: 20, and the HCDR3 comprises an amino acid sequence set forth in SEQ ID NO: 21.

In certain embodiments, the anti-Claudin18.2 antibody comprises VH, wherein the VH comprises an amino acid sequence set forth in SEQ ID NO: 32.

In certain embodiments, the anti-Claudin18.2 antibody comprises an antibody heavy chain constant region. For example, the antibody heavy chain constant region may comprise a constant region derived from human IgG. For another example, the antibody heavy chain constant region may comprise a constant region derived from human IgG1, IgG2, IgG3, or IgG4.

In certain embodiments, the anti-Claudin18.2 antibody comprises an antibody heavy chain, wherein the antibody heavy chain comprises an amino acid sequence set forth in SEQ ID NO: 34.

In certain embodiments, the anti-Claudin18.2 antibody comprises VL, wherein the VL comprises LCDR1, LCDR2 and LCDR3, wherein the LCDR1 comprises an amino acid sequence set forth in SEQ ID NO: 22, the LCDR2 comprises

an amino acid sequence set forth in SEQ ID NO: 23, and the LCDR3 comprises an amino acid sequence set forth in SEQ ID NO: 24.

In certain embodiments, the anti-Claudin18.2 antibody comprises VH and VL, wherein the VH comprises HCDR1, HCDR2, and HCDR3, and the VL comprises LCDR1, LCDR2, and LCDR3; wherein the HCDR1 comprises an amino acid sequence set forth in SEQ ID NO: 19, the HCDR2 comprises an amino acid sequence set forth in SEQ ID NO: 20, the HCDR3 comprises an amino acid sequence set forth in SEQ ID NO: 21, the LCDR1 comprises an amino acid sequence set forth in SEQ ID NO: 22, the LCDR2 comprises an amino acid sequence set forth in SEQ ID NO: 23, and the LCDR3 comprises an amino acid sequence set forth in SEQ ID NO: 24.

In certain embodiments, the anti-Claudin18.2 antibody comprises VL, wherein the VL comprises an amino acid sequence set forth in SEQ ID NO: 33.

In certain embodiments, the anti-Claudin18.2 antibody comprises VH and VL, wherein the VH comprises an amino acid sequence set forth in SEQ ID NO: 32, and the VL comprises an amino acid sequence set forth in SEQ ID NO: 33.

In certain embodiments, the anti-Claudin18.2 antibody comprises an antibody light chain constant region. For example, the antibody light chain constant region may comprise a human Igκ constant region or a human Igλ. constant region.

In certain embodiments, the anti-Claudin18.2 antibody comprises an antibody light chain, wherein the antibody light chain comprises an amino acid sequence set forth in SEQ ID NO: 35.

In certain embodiments, the anti-Claudin18.2 antibody comprises an antibody heavy chain and an antibody light chain, wherein the antibody heavy chain comprises an amino acid sequence set forth in SEQ ID NO: 34, and the antibody light chain comprises an amino acid sequence set forth in SEQ ID NO: 35.

In one embodiment, the antibody is an anti-FRα antibody comprising VH, wherein the VH comprises HCDR1, HCDR2 and HCDR3, wherein the HCDR1 comprises an amino acid sequence set forth in SEQ ID NO: 36, the HCDR2 comprises an amino acid sequence set forth in SEQ ID NO: 37, and the HCDR3 comprises an amino acid sequence set forth in SEQ ID NO: 38.

In certain embodiments, the anti-FRα antibody comprises VH, wherein the VH comprises an amino acid sequence set forth in SEQ ID NO: 49.

In certain embodiments, the anti-FRα antibody comprises an antibody heavy chain constant region. For example, the antibody heavy chain constant region may comprise a constant region derived from human IgG. For another example, the antibody heavy chain constant region may comprise a constant region derived from human IgG1, IgG2, IgG3, or IgG4.

In certain embodiments, the anti-FRα antibody comprises an antibody heavy chain, wherein the antibody heavy chain comprises an amino acid sequence set forth in SEQ ID NO: 51.

In certain embodiments, the anti-FRα antibody comprises VL, wherein the VL comprises LCDR1, LCDR2, and LCDR3, wherein the LCDR1 comprises an amino acid sequence set forth in SEQ ID NO: 39, the LCDR2 comprises an amino acid sequence set forth in SEQ ID NO: 40, and the LCDR3 comprises an amino acid sequence set forth in SEQ ID NO: 41.

In certain embodiments, the anti-FRα antibody comprises VH and VL, wherein the VH comprises HCDR1, HCDR2, and HCDR3, and the VL comprises LCDR1, LCDR2, and LCDR3; wherein the HCDR1 comprises an amino acid sequence set forth in SEQ ID NO: 36, the HCDR2 comprises an amino acid sequence set forth in SEQ ID NO: 37, the HCDR3 comprises an amino acid sequence set forth in SEQ ID NO: 38, the LCDR1 comprises an amino acid sequence set forth in SEQ ID NO: 39, the LCDR2 comprises an amino acid sequence set forth in SEQ ID NO: 40, and the LCDR3 comprises an amino acid sequence set forth in SEQ ID NO: 41.

In certain embodiments, the anti-FRα antibody comprises VL, wherein the VL comprises an amino acid sequence set forth in SEQ ID NO: 50.

In certain embodiments, the anti-FRα antibody comprises VH and VL, wherein the VH comprises an amino acid sequence set forth in SEQ ID NO: 49, and the VL comprises an amino acid sequence set forth in SEQ ID NO: 50.

In certain embodiments, the anti-FRα antibody comprises an antibody light chain constant region. For example, the antibody light chain constant region may comprise a human Igκ constant region or a human Igλ. constant region.

In certain embodiments, the anti-FRα antibody comprises an antibody light chain, wherein the antibody light chain comprises an amino acid sequence set forth in SEQ ID NO: 52.

In certain embodiments, the anti-FRα antibody comprises an antibody heavy chain and an antibody light chain, wherein the antibody heavy chain comprises an amino acid sequence set forth in SEQ ID NO: 51, and the antibody light chain comprises an amino acid sequence set forth in SEQ ID NO: 52.

Composition

In another aspect, the present application provides a pharmaceutical composition comprising the conjugate as described herein and optionally a pharmaceutically acceptable carrier.

The term "carrier" generally refers to a diluent, an adjuvant, or an excipient with which the compound is administered. Such pharmaceutical carriers may be liquids, such as water and oils, including those of petroleum, animal, vegetable or synthetic origin, such as peanut oil, soybean oil, mineral oil, and sesame oil. The carrier may be saline, gum arabic, gelatin, starch paste, talc, keratin, colloidal silica, or urea. In addition, an auxiliary agent, a stabilizer, a thickener, a lubricant, and a colorant may be used. In one embodiment, the compound or composition and the pharmaceutically acceptable carrier are sterile when administered to a patient.

The conjugate of the present application may be in any form that allows the administration of a compound (drug unit) to a patient to treat a condition associated with the expression of the antigen to which the ligand unit binds. For example, the conjugate may be in the form of a liquid or a solid. An exemplary route of administration is parenteral. Parenteral administration includes subcutaneous injection, intravenous, intramuscular, intrasternal injection or infusion techniques. In one aspect, the composition is administered parenterally. In one aspect, the conjugate is administered intravenously.

Administration may be performed by any convenient route, for example by infusion or bolus.

The materials used in the preparation of the pharmaceutical compositions may be non-toxic in the amounts used. It is apparent to those of ordinary skill in the art that the optimum doses of one or more active ingredients in the pharmaceutical composition depend on a variety of factors. Relevant factors include, but are not limited to, the type of animal (e.g., human), the specific form of compound, the administration route, and the composition adopted.

The composition may, for example, be in a liquid form. The liquid may be delivered by injection. The composition for administration by injection may further comprise one or more of a surfactant, a preservative, a wetting agent, a dispersant, a suspending agent, a buffering agent, a stabilizer, and an isotonic agent. Water is an exemplary carrier when the composition is administered intravenously. Saline solutions and aqueous dextrose and glycerol solutions may also be used as liquid carriers, particularly for injectable solutions. The suitable pharmaceutical carrier also includes an excipient, such as starch, glucose, lactose, sucrose, gelatin, malt, rice, flour, chalk, silica gel, sodium stearate, glycerol monostearate, talc, sodium chloride, skimmed milk powder, glycerol, propylene, ethylene glycol, water, or ethanol. The composition of the present invention may also comprise a small amount of wetting agent or emulsifying agent or pH buffering agent, if desired.

The pharmaceutical composition may be formulated such that the compound is bioavailable upon administration of the composition to a patient. The composition may be in the form of one or more dose units. For intravenous administration, the composition may comprise a conjugate from about 0.01 mg to about 100 mg per kg of subject body weight. Depending on the drug used, the dose may be even lower, e.g., from 1.0 μg/kg to 5.0 mg/kg, 4.0 mg/kg, 3.0 mg/kg, 2.0 mg/kg, or 1.0 mg/kg, or from 1.0 μg/kg to 500.0 μg/kg of subject body weight.

Typically, the dose of conjugate administered to a patient is typically from about 0.01 mg/kg to about 100 mg/kg of subject body weight or from 1.0 μg/kg to 5.0 mg/kg of subject body weight. In some embodiments, the dose administered to a patient is from about 0.01 mg/kg to about 15 mg/kg of subject body weight. In some embodiments, the dose administered to a patient is from about 0.1 mg/kg to about 15 mg/kg of subject body weight. In some embodiments, the dose administered to a patient is from about 0.1 mg/kg to about 20 mg/kg of subject body weight. In some embodiments, the dose administered is from about 0.1 mg/kg to about 5 mg/kg of subject body weight or from about 0.1 mg/kg to about 10 mg/kg of subject body weight.

## Use

In another aspect, the present application provides use of the conjugate as described herein or the pharmaceutical composition as described herein in preparing a medicament for treating a disease or a condition.

In another aspect, the present application provides a method for treating a disease or a condition in a subject in need thereof, comprising administering to the subject an effective amount of the conjugate as described herein.

In certain embodiments, the disease or condition comprises a tumor.

Cancers that may be treated with the ADCs of the present application include, but are not limited to, hematopoietic cancers such as, for example, lymphoma (Hodgkin lymphoma and non-Hodgkin lymphoma), leukemia and solid tumors. Examples of hematopoietic cancers include follicular lymphoma, anaplastic large cell lymphoma, mantle cell lymphoma, acute myeloid leukemia, chronic myeloid leukemia, chronic lymphocytic leukemia, diffuse large B-cell lymphoma, and multiple myeloma. Examples of solid tumors include fibrosarcoma, myxosarcoma, liposarcoma, chondrosarcoma, osteogenic sarcoma, chordoma, angiosarcoma, endothelial sarcoma, lymphangiosarcoma, lymphangioendotheliosarcoma, synovioma, mesothelioma, Ewing's sarcoma, leiomyosarcoma, rhabdomyosarcoma, colon cancer, colorectal cancer, kidney cancer, pancreatic cancer, bone cancer, breast cancer, ovarian cancer, prostate cancer, esophageal cancer, gastric cancer, oral cancer, nasal cancer, throat cancer, squamous cell carcinoma, basal cell carcinoma, adenocarcinoma, sweat gland carcinoma, sebaceous carcinoma, papillary carcinoma, papillary adenocarcinoma, cystadenocarcinoma, medullary cancer, bronchial cancer, renal cell carcinoma, liver cancer, bile duct cancer, choriocarcinoma, seminoma, embryonal carcinoma, Wilms tumor, cervical cancer, uterine cancer, testicular cancer, small-cell lung cancer,

bladder cancer, lung cancer, epithelial cancer, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, and retinoblastoma.

In certain embodiments, the tumor includes a tumor with abnormal HER3 expression.

In certain embodiments, the tumor includes a tumor with high HER3 expression.

In certain embodiments, the tumor includes a tumor with abnormal claudin18.2 expression.

In certain embodiments, the tumor includes a tumor with high claudin18.2 expression.

In certain embodiments, the tumor includes a tumor with abnormal FRα expression.

In certain embodiments, the tumor includes a tumor with high FRα expression.

Without wishing to be bound by any theory, the following examples are intended only to illustrate the linkers, linker-drugs, conjugates, preparation methods, uses, etc., of the present application, and are not intended to limit the scope of the invention to which the present application relates.

Examples

Example 1

**1.1 Construction Method of ADCs**

The anti-HER3 antibody, the anti-Claudin18.2 antibody, and the anti-FRα antibody were exchanged into a target buffer (citric acid buffer or histidine hydrochloride buffer, pH 6.0-6.5) using a buffer exchange device (desalting column, ultrafiltration tube, etc.), and the antibody concentrations were measured. With the antibody concentrations maintained at 5-20 mg/mL, the antibodies were mixed and incubated with a reducing agent (0.04-0.4 mM TCEP) and maintained at 10-37 °C for 1-5 h to completely break the inter-chain disulfide bonds of the antibodies. The antibody solutions obtained after reduction were subsequently adjusted to pH 6.0 with phosphoric acid, and cooled to room temperature. The linker-small molecule drugs were dissolved in DMSO, and their concentrations were calculated. According to a suitable drug-to-antibody ratio (DAR) (molar ratio > 10), the linker-drug solutions (04-Dxd, 07-Dxd, 08-Dxd, and 09-Dxd, wherein the amino acid unit in 04-Dxd was replaced by GGFG) were mixed with the antibody solutions obtained after complete reduction, with the DMSO proportion kept at no more than 10% (v/v), and the reaction was completed after 0.5-2 h. The ADC crude product solutions were purified with a purification device (desalting column, ultrafiltration tube, tangential flow system, etc.) after filtration, to remove small molecule organic impurities and excess DMSO from the solutions, and subsequently the ADC products were exchanged into storage buffers.

Table 1. Sequences of light chains and heavy chains of antibodies

| TYPE | NAMA | SEQUENCE ID NO. |
|---|---|---|
| Anti-HER3 antibody | RS-HT-002 | HC: SEQ ID NO: 17<br>LC: SEQ ID NO: 18 |
| Anti-Claudin18.2 antibody | hu782 | HC: SEQ ID NO: 34<br>LC: SEQ ID NO: 35 |
| Anti-FRα antibody | M9346A | HC: SEQ ID NO: 51<br>LC: SEQ ID NO: 52 |

Table 2. Linker-drug structures

| Linker-drug | Structure |
|---|---|
| 04-Dxd | 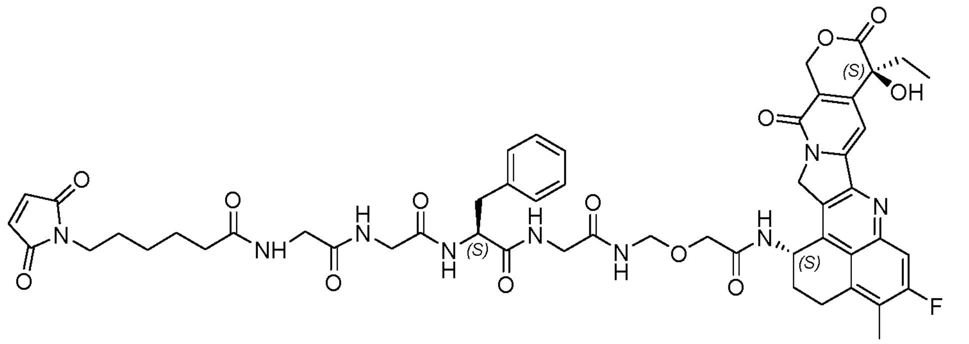<br>04Dxd |
| 07-Dxd | 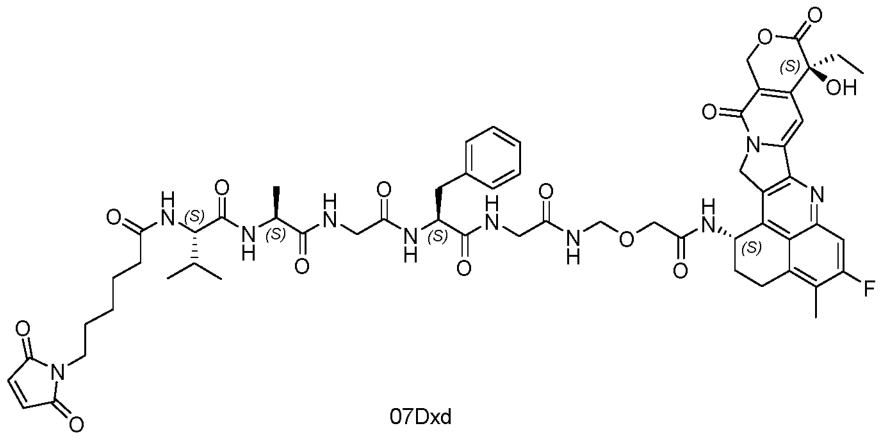<br>07Dxd |
| 08-Dxd | 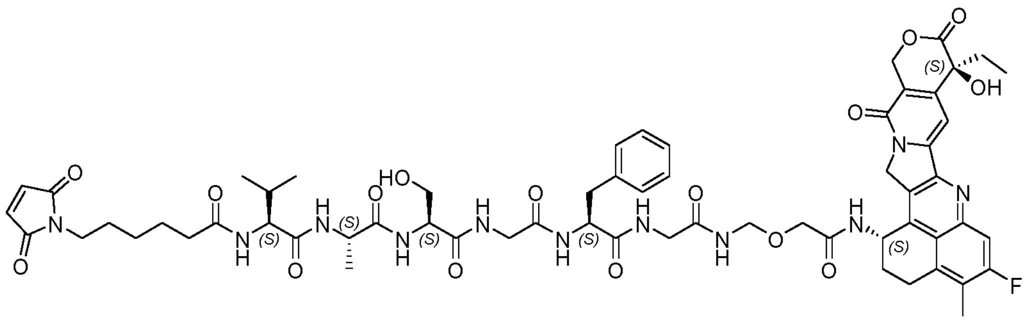<br>08Dxd |

(continued)

| Linker-drug | Structure |
|---|---|
| 09-Dxd | 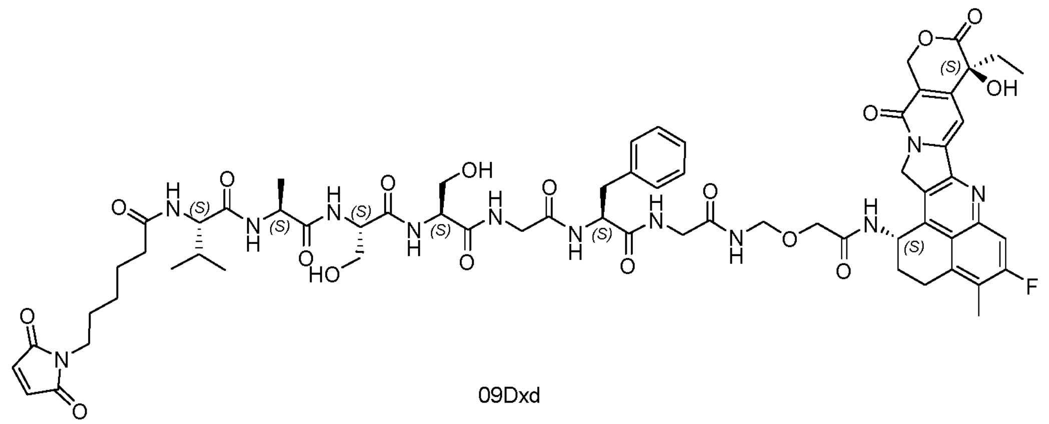 09Dxd |

### 1.2 Determination of DARs of ADC products

HIC-HPLC method was adopted in the determination. Sample loading and elution were carried out under the condition of pH 7.0 mobile phase. The areas of absorption peaks at specified wavelengths were measured. The specific determination method is shown in Table 3. The DARs of ADC products were in the range of 7-8.

Table 3. HIC-HPLC Method

| | |
|---|---|
| Column: | Hydrophobic interaction chromatography column |
| Flow Rate: | 0.5-1 ml/min |
| Stop Time: | 15-30 min |
| Injection Amount: | 10-20 ug |
| Mobile Phases: | Mobile Phase A: Phosphate buffer + high-concentration ammonium sulfate, pH = 7.0 ± 0.2<br>Mobile Phase B: Phosphate buffer + 15-25% v/v isopropanol, pH = 7.0 ± 0.2 |

### 1.3 Determination results for DAR values by HIC-HPLC

| | HER3-ADC | | | | Claudin18.2-ADC | | FRα-ADC | |
|---|---|---|---|---|---|---|---|---|
| | HER3-ADC04 | HER3-ADC07 | HER3-ADC08 | HER3-ADC09 | Claudin18.2-ADC08 | Claudin18.2-ADC09 | FRα-ADC08 | FRα-ADC09 |
| DAR | 8.05 | 7.97 | 7.93 | 7.97 | 7.67 | 7.42 | 8.00 | 8.00 |

Example 2

### 2.1 Evaluation *of In Vitro* Activity of HER3 ADCs

Human colon cancer SW620 cells in the logarithmic growth phase were digested with pancreatin, and subsequently resuspended in a corresponding culture medium, with the density adjusted to be 2 × $10^4$ cells /mL. The cells were seeded into a 96-well microplate at 100 μL/well, allowing for a density of 2 × $10^3$ cells/well, and 200 μL of the culture medium was added to each of the rest wells. After culturing for 48 h at 37 °C, 5% $CO_2$, the supernatant was pipetted from the cell culture plate. The linker-drugs (04-Dxd, 07-Dxd, 08-Dxd, and 09-Dxd), HER3 ADCs (HER3-ADC04, HER3-ADC07, HER3-ADC08, and HER3-ADC09), and a monoclonal antibody control were diluted in a 2-fold gradient with the culture medium, starting from the initial concentration of 400 nM, to obtain a total of 9 concentration gradients. The diluted samples were added at 100 μL/well. After further culturing for 4 days and 6 days, color development was

carried out. The cell plate was taken out and equilibrated for 15-20 min at room temperature, and subsequently 100 μL of CellTiter-Glo® Luminescent Cell Viability assay buffer (purchased from Promega) was added. The plate was shaken for 2 min on a shaker at 200 rpm, kept for 18 min in the dark, and read using an M5 microplate reader.

Data processing:

$$\text{Killing rate} = \frac{\textit{Fluorescence reading of culture medium control well-Fluorescence reading of dosing well}}{\textit{Fluorescence reading of culture medium control well}} \times 100\%.$$

followed by plotting through four-parameter fitting, with the logarithm of the concentration as the abscissa and the killing rate as the ordinate.

Results: FIG. 2A shows that the anti-HER3 monoclonal antibody conjugated to Dxd had a significant killing effect on SW620 target cells as compared to the naked antibody (RS-HT-002).

FIG. 2B shows that different linker-Dxd all had a killing effect on human colon cancer SW620 cells, and 07-Dxd, 08-Dxd, and 09-Dxd had a significantly better killing effect than 04-Dxd.

FIG. 3 shows that anti-HER3 ADCs containing different linkers all had a killing effect on human colon cancer SW620 cells, and HER3-ADC07, HER3-ADC08, and HER3-ADC09 had a significantly better killing effect than HER3-ADC04.

FIG. 4 shows that the anti-HER3 ADCs containing different linkers all had no significant killing effect on non-target cells CHO-K1.

### 2.2 Evaluation of Plasma Stability of HER3 ADCs

In order to determine the *in vivo* stability of the ADCs, in this study, human plasma *in vitro* was used for incubation to simulate the *in vivo* environment for detection. Specifically, HER3 ADCs were added to 10% human plasma to make the final antibody concentration of 1 mg/mL, and incubated at 37 °C for 3 days and 7 days, and subsequently cell killing assay was carried out. SW620 target cells in the logarithmic growth phase were digested, seeded at a certain density, and subsequently cultured for 48 h at 37 °C, 5% $CO_2$. Subsequently serial-diluted ADCs after incubation in plasma were added, and ADCs diluted with plasma in the same way and without incubation were used as controls. Results: FIG. 5 shows that HER3-ADC08 and HER3-ADC09 did not exhibit significant changes in cell killing activity after incubation in plasma for 3 days or 7 days.

### 2.3 Evaluation *of In Vivo* Activity of HER3 ADCs

Female SPF-grade BALB/c nude mice, 4-6 weeks old, were selected and adaptively raised for 3-4 days for tumor transplantation. The human colon cancer SW620 cells in the logarithmic growth phase were digested, washed twice with a serum-free 1640 culture medium, and diluted to $2.5 \times 10^7$ cells /mL. The dilution was subsequently inoculated subcutaneously into the right axilla of the mice at $5 \times 10^6$ cells/0.2 mL/mouse. The mice were grouped by randomized block design 1 week after the inoculation when tumors grew to 150 ± 50 $mm^3$, with 5 mice in each group. The blank model group was subjected to administration with a PBS control. The ADCs were administered at doses of 2 mg/kg, 6 mg/kg, and 10 mg/kg. The small molecule control group was subjected to administration according to the same molar dose of 10 mg/kg ADC. The administration was performed once weekly, wherein the 6 mg/kg and 10 mg/kg dose groups were subjected to administration for four times in total, and the 2 mg/kg dose group was subjected to administration for eight times in total. The administration was performed by intraperitoneal injection. The tumors were measured twice weekly, and the data of tumor volume and mouse body weight were recorded before each administration. Tumor volume calculation: $V (mm^3) = (W^2 \times L)/2$, where W is the short diameter of the tumor near the midline, and L is the long diameter of the tumor near the midline. The mice were sacrificed by cervical dislocation when tumor volume was greater than 2000 $mm^3$, or when weight loss was greater than 20%, or when interference with vital physiological function or neoplastic ulceration or necrosis was observed.

Results: FIG. 6 shows that HER3-ADC07, HER3-ADC08, and HER3-ADC09 were all able to significantly inhibit the growth of SW620 xenograft tumors. Complete tumor regression was observed in the mice in the 6 mg/kg and 10 mg/kg dose groups after four times of administration, and tumor growth was observed in individual mice three weeks after the administration was stopped. Inhibition on tumor growth was observed in the mice in the 2 mg/kg dose group. HER3-ADC08 had a better tumor inhibitory effect than HER3-ADC07 and HER3-ADC09. FIG. 7 shows that the ADC treatment groups did not exhibit significant changes in the body weight of the mice as compared to the PBS control group during the treatment.

Example 3

### 3.1 Evaluation *of In Vitro* Activity of Claudin18.2 ADCs

HEK293-hCLDN18.2, NUGC4, and NUGC4-hCLDN18.2 cells in the logarithmic growth phase were digested with pancreatin, and subsequently resuspended in a corresponding culture medium, with the density adjusted to be $2 \times 10^4$ cells/mL. The cells were seeded into a 96-well microplate at 100 μL/well, allowing for a density of $2 \times 10^3$ cells/well, and 200 μL of the culture medium was added to each of the rest wells. After cell culture for 48 h at 37 °C, 5% $CO_2$, the supernatant was pipetted from the cell culture plate. The linker-drugs (08-Dxd and 09-Dxd), ADCs (Claudin18.2-ADC08 and Claudin18.2-ADC09), and the monoclonal antibody control (hu782) were diluted in a 2-fold gradient with the culture medium, starting from the initial concentration of 400 nM, to obtain a total of 9 concentration gradients. The diluted samples were added at 100 μL/well. After further culturing for 4 days and 6 days, color development was carried out. The cell plate was taken out and equilibrated for 15-20 min at room temperature, and subsequently 100 μL of CellTiter-Glo® Luminescent Cell Viability assay buffer (purchased from Promega) was added. The plate was shaken for 2 min on a shaker at 200 rpm, kept for 18 min in the dark, and read using an M5 microplate reader.

Data processing:

$$\text{Killing rate} = \frac{\textit{Fluorescence reading of culture medium control well-Fluorescence reading of dosing well}}{\textit{Fluorescence reading of culture medium control well}} \times 100\%.$$

followed by plotting through four-parameter fitting, with the logarithm of the concentration as the abscissa and the killing rate as the ordinate.

Results: FIG. 8, FIG. 9, and FIG. 10 show that Claudin18.2-ADC08 and Claudin18.2-ADC09 had a significant killing effect on three Claudin18.2-positive target cells, namely NUGC4, NUGC4-hCLDn18.2, and HEK293-hCLDn18.2.

### 3.2 Evaluation of Plasma Stability of Claudin18.2 ADCs

In order to determine the *in vivo* stability of the ADCs, in this study, human plasma *in vitro* was used for incubation to simulate the *in vivo* environment for detection. Specifically, Claudin18.2-ADC08 was added to 10% human plasma to make the final antibody concentration of 1 mg/mL, and incubated at 37 °C for 3 days and 7 days, and subsequently cell killing assay was carried out. HEK293-hCLDN18.2 target cells in the logarithmic growth phase were digested, seeded at a certain density, and subsequently cultured for 48 h at 37 °C, 5% $CO_2$. Subsequently serial-diluted Claudin18.2-ADC08 after incubation in plasma was added, and Claudin18.2-ADC08 diluted with plasma in the same way and without incubation was used as a control.

Results: FIG. 11 shows that Claudin18.2-ADC08 did not exhibit significant changes in cell killing activity after incubation in plasma for 3 days or 7 days.

### 3.3 Evaluation *of In Vivo* Activity of Claudin18.2 ADCs

Female SPF-grade BALB/c nude mice, 4-6 weeks old, were selected and adaptively raised for 3-4 days for tumor transplantation. NUGC4 cells in the logarithmic growth phase were digested, washed twice with a serum-free 1640 culture medium, and diluted to $2.5 \times 10^7$ cells/mL. The dilution was subsequently inoculated subcutaneously into the right axilla of the mice at $5 \times 10^6$ cells/0.2 mL/mouse. The mice were grouped by randomized block design 1 week after the inoculation when tumors grew to $150 \pm 50$ mm$^3$, with 5 mice in each group. The blank model group was subjected to administration with a PBS control. The small molecule control group was subjected to administration according to the same molar dose of the 10 mg/kg ADC. Claudin18.2-ADC08 was administrated at three doses of 1 mg/kg, 3 mg/kg, and 10 mg/kg. The administration was performed once weekly, for four times in total. The measurement was performed twice weekly after the administration was stopped. The administration was performed by intraperitoneal injection. The data of tumor volume and mouse body weight were recorded before each administration.

Tumor volume calculation: V (mm$^3$) = ($W^2 \times L$)/2, where W is the short diameter of the tumor near the midline, and L is the long diameter of the tumor near the midline. The mice were sacrificed by cervical dislocation when tumor volume was greater than 2000 mm$^3$, or when weight loss was greater than 20%, or when interference with vital physiological function or neoplastic ulceration or necrosis was observed.

Results: FIG. 12 shows that Claudin18.2-ADC08 had tumor growth inhibitory activity with a certain dose effect in an NUGC4 subcutaneous xenograft mouse model.

Example 4

**4.1 Evaluation *of In Vitro* Activity of FRα ADCs**

Ovarian cancer OVCAR3 cells in the logarithmic growth phase were digested with pancreatin, and subsequently resuspended in a corresponding culture medium, with the density adjusted to be $2 \times 10^4$ cells/mL. The cells were seeded into a 96-well microplate at 100 μL/well, allowing for a density of $2 \times 10^3$ cells/well, and 200 μL of the culture medium was added to each of the rest wells. After cell culture for 48 h at 37 °C, 5% $CO_2$, the supernatant was pipetted from the cell culture plate. The linker-drugs (08-Dxd and 09-Dxd), ADCs (FRα-ADC08 and FRα-ADC09), and the monoclonal antibody control (M9346A) were diluted in a 2-fold gradient with the culture medium, starting from the initial concentration of 400 nM, to obtain a total of 9 concentration gradients. The diluted samples were added at 100 μL/well. After further culturing for 4 days and 6 days, color development was carried out. The cell plate was taken out and equilibrated for 15-20 min at room temperature, and subsequently 100 μL of CellTiter-Glo® Luminescent Cell Viability assay buffer (purchased from Promega) was added. The plate was shaken for 2 min on a shaker at 200 rpm, kept for 18 min in the dark, and read using an M5 microplate reader.

Data processing:

$$\text{Killing rate} = \frac{\textit{Fluorescence reading of culture medium control well-Fluorescence reading of dosing well}}{\textit{Fluorescence reading of culture medium control well}} \times 100\%.$$

followed by plotting through four-parameter fitting, with the logarithm of the concentration as the abscissa and the killing rate as the ordinate.

Result: FIG. 13 shows that FRα-ADC08 and FRα-ADC09 both had significant killing activity against OVCAR3 cells.

**4.2 Evaluation of Plasma Stability of FRα ADCs**

In order to determine the *in vivo* stability of the ADCs, in this study, human plasma *in vitro* was used for incubation to simulate the *in vivo* environment for detection. Specifically, FRα-ADC08 was added to 50% human plasma to make the final antibody concentration of 1 mg/mL, and incubated at 37 °C for 3 days and 7 days, and subsequently cell killing assay was carried out. OVCAR3 target cells in the logarithmic growth phase were digested, seeded at a certain density, and subsequently cultured for 48 h at 37 °C, 5% $CO_2$. Subsequently serial-diluted FRα-ADC08 after incubation in plasma was added, and FRα-ADC08 diluted with plasma in the same way and without incubation was used as a control.

Results: FIG. 14 shows that FRα-ADC08 did not exhibit significant changes in cell killing activity after incubation in plasma for 3 days or 7 days.

**4.3 Evaluation *of In Vivo* Activity of FRα ADCs**

Female SPF-grade BALB/c nude mice, 3-4 weeks old, were selected and adaptively raised for 3-4 days for tumor transplantation. The OVCAR3 cells in the logarithmic growth phase were digested, washed twice with serum-free 1640 culture medium, diluted to $3 \times 10^7$ cells/mL, and mixed with matrigel of the same volume. The dilution was subsequently inoculated subcutaneously into the right axilla of the mice at $3 \times 10^6$ cells/0.2 mL/mouse. The mice were grouped by randomized block design 1 week after the inoculation when tumors grew to $150 \pm 50$ $mm^3$, with 5 mice in each group. The blank model group was subjected to administration with a PBS control. The small molecule control group was subjected to administration according to the same molar dose of the ADC 5 mg/kg. FRα-ADC08 was administrated at two doses of 2 mg/kg and 5 mg/kg. The administration was performed once weekly. The measurement was performed twice weekly. The administration was performed by intraperitoneal injection. The data of tumor volume and mouse body weight were recorded before each administration.

Tumor volume calculation: $V (mm^3) = (W^2 \times L)/2$, where W is the short diameter of the tumor near the midline, and L is the long diameter of the tumor near the midline. The mice were sacrificed by cervical dislocation when tumor volume was greater than 2000 $mm^3$, or when weight loss was greater than 20%, or when interference with vital physiological function or neoplastic ulceration or necrosis was observed.

Results: FIG. 15 shows that FRα-ADC08 had a tumor growth inhibitory effect in an OVCAR3 subcutaneous xenograft mouse model.

## Claims

1. A linker, comprising an amino acid unit (RL), wherein RL comprises -Val-Ala-Q-Gly-Phe-Gly-, wherein Q is a bond, a single amino acid, or a dipeptide.

2. The linker according to claim 1, wherein RL is cleavable by an enzyme.

3. The linker according to any one of claims 1-2, wherein RL is cleavable by cathepsin B.

4. The linker according to any one of claims 1-3, wherein Q is a bond.

5. The linker according to any one of claims 1-3, wherein Q is -Ser- or -Ser-Ser-.

6. The linker according to any one of claims 1-5, wherein RL comprises -Val-Ala-Gly-Phe-Gly-(VAGFG), -Val-Ala-Ser-Gly-Phe-Gly- (VASGFG), or -Val-Ala-Ser-Ser-Gly-Phe-Gly- (VASSGFG).

7. The linker according to any one of claims 1-6, wherein the linker is selected from the following structures: -Z-A-S*-RL- and -Z-A-S*-RL-Y-; wherein, Z is an extender unit; A is a bond or an adapter unit; S* is a bond or a partitioning agent; Y is a spacer unit.

8. The linker according to claim 7, wherein Z comprises the following structure: -(succinimid-3-yl-N)-, $-CH_2-C(=O)-NH-$ or -C(=O)-; wherein, -(succinimid-3-yl-N)- has the following structure:

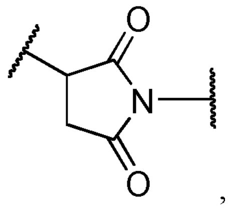

,

wherein the wavy line at position 3 of the structure represents an attachment position to a ligand unit, and the wavy line at the N atom of position 1 represents an attachment position to A.

9. The linker according to any one of claims 7-8, wherein A is selected from the following structures:

$-C_1-C_{10}$ alkylene-, $-C_1-C_{10}$ heteroalkylene-, $-C_3-C_8$ carbocyclyl-, $-O-(C_1-C_8$ alkylene)-, -arylene-, $-C_1-C_{10}$ alkylene-arylene-, -arylene-$C_1-C_{10}$ alkylene-, $-C_1-C_{10}$ alkylene-($C_3-C_8$ carbocyclyl)-, -($C_3-C_8$ carbocyclyl)-$C_1-C_{10}$ alkylene-, $-C_3-C_8$ heterocyclyl-, $-C_1-C_{10}$ alkylene-($C_3-C_8$ heterocyclyl)-, -($C_3-C_8$ heterocyclyl)-$C_1-C_{10}$ alkylene-, $-C_1-C_{10}$ alkylene-C(=O)-, $-C_1-C_{10}$ heteroalkylene-C(=O)-, $-C_3-C_8$ carbocyclyl-C(=O)-, -O-($C_1-C_8$ alkylene)-C(=O)-, -arylene-C(=O)-, $-C_1-C_{10}$ alkylene-arylene-C(=O)-, -arylene-$C_1-C_{10}$ alkylene-C(=O)-, $-C_1-C_{10}$ alkylene-($C_3-C_8$ carbocyclyl)-C(=O)-, -($C_3-C_8$ carbocyclyl)-$C_1-C_{10}$ alkylene-C(=O)-, $-C_3-C_8$ heterocyclyl-C(=O)-, $-C_1-C_{10}$ alkylene-($C_3-C_8$ heterocyclyl)-C(=O)-, -($C_3-C_8$ heterocyclyl)-$C_1-C_{10}$ alkylene-C(=O)-, $-C_1-C_{10}$ alkylene-NH-, $-C_1-C_{10}$ heteroalkylene-NH-, $-C_3-C_8$ carbocyclyl-NH-, -O-($C_1-C_8$ alkylene)-NH-, -arylene-NH-, $-C_1-C_{10}$ alkylene-arylene-NH-, -arylene-$C_1-C_{10}$ alkylene-NH-, $-C_1-C_{10}$ alkylene-($C_3-C_8$ carbocyclyl)-NH-, - ($C_3-C_8$ carbocyclyl)-$C_1-C_{10}$ alkylene-NH-, $-C_3-C_8$ heterocyclyl-NH-, $-C_1-C_{10}$ alkylene-($C_3-C_8$ heterocyclyl)-NH-, -($C_3-C_8$ heterocyclyl)-$C_1-C_{10}$ alkylene-NH-, $-C_1-C_{10}$ alkylene-S-, $-C_1-C_{10}$ heteroalkylene-S-, $-C_3-C_8$ carbocyclyl-S-, -O-($C_1-C_8$ alkylene)-S-, -arylene-S-, $-C_1-C_{10}$ alkylene-arylene-S-, -arylene-$C_1-C_{10}$ alkylene-S-, $-C_1-C_{10}$ alkylene-($C_3-C_8$ carbocyclyl)-S-, -($C_3-C_8$ carbocyclyl)-$C_1-C_{10}$ alkylene-S-, $-C_3-C_8$ heterocyclyl-S-, $-C_1-C_{10}$ alkylene-($C_3-C_8$ heterocyclyl)-S-, or -($C_3-C_8$ heterocyclyl)-$C_1-C_{10}$ alkylene-S-;

wherein, A is optionally substituted with a basic unit (BU), wherein the basic unit is $-(CH_2)_xNH_2$, - $(CH_2)_xNHR^a$, or $-(CH_2)_xNR^a_2$; wherein x is any integer from 1 to 4; each $R^a$ is independently selected from $C_1$-$C_6$ alkyl and $C_1$-$C_6$ haloalkyl, or two $R^a$ groups, together with nitrogen attached thereto, form a 4- to 6-membered heterocycloalkyl ring, or an azetidinyl, pyrrolidinyl or piperidinyl group.

10. The linker according to any one of claims 7-9, wherein Ais selected from the following structure: -($C_1-C_{10}$)alkylene-C(=O)-, wherein the alkylene moiety of A is optionally substituted with the basic unit (BU).

**11.** The linker according to any one of claims 7-10, wherein the linker comprises the following structure:

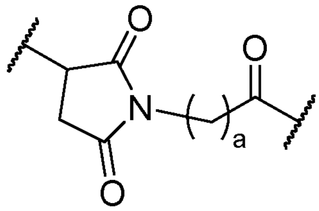

,

wherein a is any integer from 1 to 8.

**12.** The linker according to claim 11, wherein a is any integer from 2 to 5.

**13.** The linker according to any one of claims 7-12, wherein Y comprises a structure represented by -NH-$(CH_2)n^1$-La-Lb-Lc-, wherein La represents -C(=O)-NH-, -$NR^1$-$(CH_2)n^2$-, -O-, or a single bond, $R^1$ represents H, $C_1$-$C_6$ alkyl, $C_1$-$C_6$ alkylene-carboxyl, or $C_1$-$C_6$ alkylene-hydroxyl; Lb represents -$CR^2$(-$R^3$)- or a single bond, wherein $R^2$ and $R^3$ each independently represents H, -$NH_2$, $C_1$-$C_6$ alkylene, $C_1$-$C_6$ alkylene-amino, $C_1$-$C_6$ alkylene-carboxyl, or $C_1$-$C_6$ alkylene-hydroxyl, and $R^2$ and $R^3$ are not both -$NH_2$;

$n^1$ represents an integer from 0 to 6;
Lc represents -$CH_2$- or -C(=O)-.

**14.** The linker according to any one of claims 7-13, wherein Y comprises NH-$(CH_2)n^1$-O-$(CH_2)n^2$-C(=O)-, wherein $n^1$ or $n^2$ is each independently any integer from 0 to 5.

**15.** The linker according to any one of claims 7-14, wherein Y comprises -NH-$CH_2$-C(=O)-, -NH-$CH_2CH_2$-C(=O)-, -NH-$(CH_2)_3$-C(=O)-, -NH-$(CH_2)_4$-C(=O)-, -NH-$(CH_2)_5$-C(=O)-, - NH-$CH_2$-O-$CH_2$-C(=O)-, or -NH-$(CH_2)_2$-O-$CH_2$-C(=O)-.

**16.** The linker according to any one of claims 7-15, wherein S* is a bond, and the linker is -Z-A-RL- or -Z-A-RL-Y-.

**17.** The linker according to any one of claims 7-15, wherein S* is a partitioning agent, and the linker is -Z-A-S*-RL- or -Z-A-S*-RL-Y-.

**18.** The linker according to claim 17, wherein S* comprises a PEG unit.

**19.** The linker according to claim 18, wherein S* has the following formula:

-NH-$(CH_2CH_2O)_b$-$CH_2CH_2C$(=O)-;
-NH-$(CH_2CH_2O)_b$-$CH_2CH_2C$(=O)NH-$(CH_2CH_2O)$-$CH_2CH_2C$(=O)-; or
-NH-$(CH_2CH_2O)_b$-$CH_2CH_2NH$-$(CH_2CH_2O)$-$CH_2CH_2C$(=O)-;

wherein S* is connected to A on the left side and connected to RL on the right side, and b is any integer from 2 to 20.

**20.** The linker according to claim 19, wherein b is 2, 4, 8, or 12.

**21.** The linker according to any one of claims 1-20, wherein the linker is selected from the following structures:

-(succinimid-3-yl-N)-$CH_2CH_2$-C(=O)-RL-NH-$CH_2CH_2$-C(=O)-;
-(succinimid-3-yl-N)-$CH_2CH_2$-C(=O)-RL-NH-$CH_2CH_2CH_2$-C(=O)-;
-(succinimid-3-yl-N)-$CH_2CH_2CH_2CH_2CH_2$-C(=O)-RL-NH-$CH_2CH_2$-C(=O)-;
-(succinimid-3-yl-N)-$CH_2CH_2CH_2CH_2CH_2$-C(=O)-RL-NH-$CH_2CH_2CH_2$-C(=O)-;
-(succinimid-3-yl-N)-$CH_2CH_2CH_2CH_2CH_2$-C(=O)-RL-NH-$CH_2CH_2CH_2CH_2CH_2$-C(=O)-;
-(succinimid-3-yl-N)-$CH_2CH_2CH_2CH_2CH_2$-C(=O)-RL-NH-$CH_2$-O-$CH_2$-C(=O)-;
-(succinimid-3-yl-N)-$CH_2CH_2CH_2CH_2CH_2$-C(=O)-RL-NH-$CH_2CH_2$-O-$CH_2$-C(=O)-;
-(succinimid-3-yl-N)-$CH_2CH_2$-C(=O)-NH-$CH_2CH_2O$-$CH_2CH_2O$-$CH_2CH_2$-C(=O)-RL-NH-$CH_2CH_2CH_2$-C(=O)-;

-(succinimid-3-yl-N)-$CH_2CH_2$-C(=O)-NH-$CH_2CH_2O$-$CH_2CH_2O$-$CH_2CH_2$-C(=O)-RL-NH-$CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2$-C(=O)-;
-$CH_2$-C(=O)-NH-$CH_2CH_2$-C(=O)-RL-NH-$CH_2CH_2CH_2$-C(=O)-;
-C(=O)-$CH_2CH_2CH_2CH_2CH_2CH_2$-C(=O)-RL-NH-$CH_2CH_2CH_2$-C(=O)-;

wherein,

-(succinimid-3-yl-N)- has the following structure:

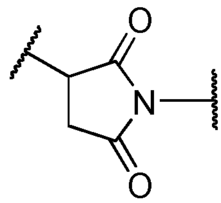
,

wherein the wavy line at position 3 of the structure represents an attachment position to a ligand unit, the wavy line at the N atom of position 1 represents an attachment position to A;
-RL- represents the following structure: -Val-Ala-Gly-Phe-Gly- (VAGFG), -Val-Ala-Ser-Gly-Phe-Gly- (VASGFG), or -Val-Ala-Ser-Ser-Gly-Phe-Gly- (VASSGFG).

**22.** The linker according to any one of claims 1-21, wherein the linker-drug is selected from the following structures:

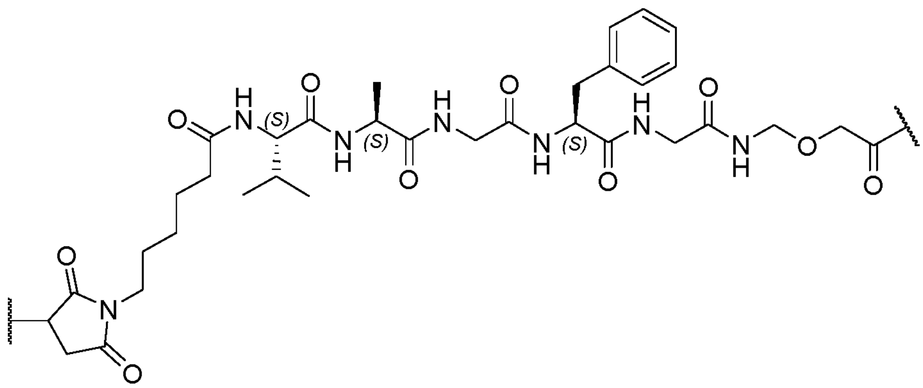
,

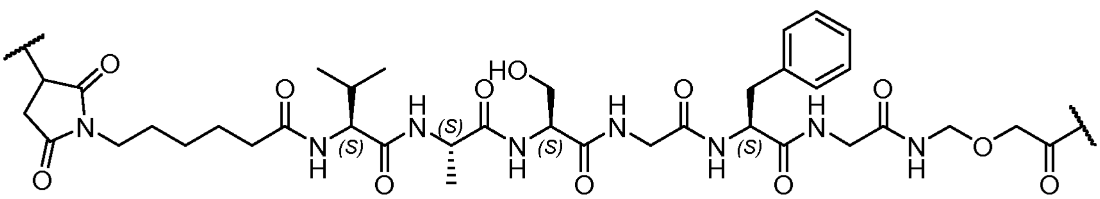
,

and

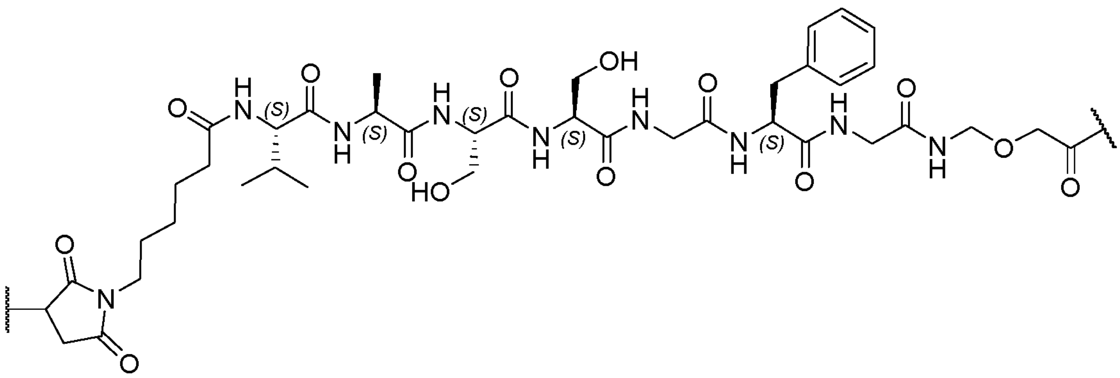

.

**23.** A linker-drug, comprising the linker according to any one of claims 1-22.

**24.** The linker-drug according to claim 23, wherein the linker-drug is selected from the following structures: Z'-A-S*-RL-D and Z'-A-S*-RL-Y-D;
wherein, Z' is an extender unit precursor; A is a bond or an adapter unit; S* is a bond or a partitioning agent; Y is a spacer unit; D is a drug unit.

**25.** The linker-drug according to any one of claims 23-24, wherein the extender unit precursor Z' is selected from (maleimid-N-yl)-, (pyrrolidine-2,5-dione-N-yl)-, $M-CH_2-C(=O)-NH-$, and $HS-(CH_2)_{1-5}-C(=O)-$, wherein M is halogen.

**26.** The linker-drug according to any one of claims 23-25, wherein the drug unit comprises a cytotoxic agent.

**27.** The linker-drug according to any one of claims 23-26, wherein the drug unit comprises: amanitins, anthracyclines, auristatins, baccatins, calicheamicins, camptothecins, cemadotins, colchicines, colcimids, combretastatins, cryptophycins, discodermolides, duocarmycins, docetaxel, doxorubicin, duocarmycins, echinomycins, eleutherobins, epothilones, estramustines, lexitropsins, maytansines, maytansinoids, methotrexate, netropsins, pyrrolo[2,1-c][1,4] benzodiazepines (PBDs), puromycins, rhizoxins, SN-38, taxanes, tubulysins, vinca alkaloids, or derivatives thereof.

**28.** The linker-drug according to any one of claims 23-27, wherein the drug unit comprises a DNA topoisomerase I inhibitor.

**29.** The linker-drug according to any one of claims 23-28, wherein the drug unit comprises camptothecin or a derivative thereof.

**30.** The linker-drug according to any one of claims 23-29, wherein the drug unit has the following structure:

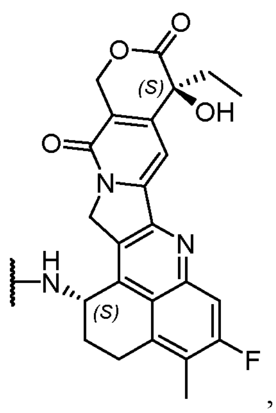

,

Dxd

wherein the nitrogen atom of the amino group at position 1 is a group at an attachment position.

**31.** The linker-drug according to claim 30, wherein the linker-drug unit has the following structure:

(maleimid-N-yl)-$CH_2CH_2$-C(=O)-RL-NH-$CH_2CH_2$-C(=O)-Dxd;
(maleimid-N-yl)-$CH_2CH_2$-C(=O)-RL-NH-$CH_2CH_2CH_2$-C(=O)-Dxd;
(maleimid-N-yl)-$CH_2CH_2CH_2CH_2CH_2$-C(=O)-RL-NH-$CH_2CH_2$-C(=O)-Dxd;
(maleimid-N-yl)-$CH_2CH_2CH_2CH_2CH_2$-C(=O)-RL-NH-$CH_2CH_2CH_2$-C(=O)-Dxd;
(maleimid-N-yl)-$CH_2CH_2CH_2CH_2CH_2$-C(=O)-RL-NH-$CH_2CH_2CH_2CH_2CH_2$-C(=O)-Dxd;
(maleimid-N-yl)-$CH_2CH_2CH_2CH_2CH_2$-C(=O)-RL-NH-$CH_2$-O-$CH_2$-C(=O)-Dxd;
(maleimid-N-yl)-$CH_2CH_2CH_2CH_2CH_2$-C(=O)-RL-NH-$CH_2CH_2$-O-$CH_2$-C(=O)-Dxd;
(maleimid-N-yl)-$CH_2CH_2$-C(=O)-NH-$CH_2CH_2O$-$CH_2CH_2O$-$CH_2CH_2$-C(=O)-RL-NH-$CH_2CH_2CH_2$-C(=O)-Dxd;
(maleimid-N-yl)-$CH_2CH_2$-C(=O)-NH-$CH_2CH_2O$-$CH_2CH_2O$-$CH_2CH_2$-C(=O)-RL-NH-$CH_2CH_2$-C(=O)-Dxd;
(maleimid-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-Dxd;
(maleimid-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2$-C(=O)-Dxd;
(pyrrolidine-2,5-dione-N-yl)-$CH_2CH_2$-C(=O)-RL-NH-$CH_2CH_2$-C(=O)-Dxd;
(pyrrolidine-2,5-dione-N-yl)-$CH_2CH_2$-C(=O)-RL-NH-$CH_2CH_2CH_2$-C(=O)-Dxd;
(pyrrolidine-2,5-dione-N-yl)-$CH_2CH_2CH_2CH_2CH_2$-C(=O)-RL-NH-$CH_2CH_2$-C(=O)-Dxd;
(pyrrolidine-2,5-dione-N-yl)-$CH_2CH_2CH_2CH_2CH_2$-C(=O)-RL-NH-$CH_2CH_2CH_2$-C(=O)-Dxd;
(pyrrolidine-2,5-dione-N-yl)-$CH_2CH_2CH_2CH_2CH_2$-C(=O)-RL-NH-$CH_2CH_2CH_2CH_2CH_2$-C(=O)-Dxd;
(pyrrolidine-2,5-dione-N-yl)-$CH_2CH_2CH_2CH_2CH_2$-C(=O)-RL-NH-$CH_2$-O-$CH_2$-C(=O)-Dxd;
(pyrrolidine-2,5-dione-N-yl)-$CH_2CH_2CH_2CH_2CH_2$-C(=O)-RL-NH-$CH_2CH_2$-O-$CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2$-C(=O)-RL-NH-$CH_2CH_2CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2$-C(=O)-RL-NH-$CH_2CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-Dxd;
(pyrrolidine-2,5-dione-N-yl)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2$-C(=O)-Dxd;
M-$CH_2$-C(=O)-NH-$CH_2CH_2$-C(=O)-RL-NH-$CH_2CH_2$-C(=O)-Dxd;
M-$CH_2$-C(=O)-NH-$CH_2CH_2$-C(=O)-RL-NH-$CH_2CH_2CH_2$-C(=O)-Dxd;
M-$CH_2$-C(=O)-NH-$CH_2CH_2$-C(=O)-RL-NH-$CH_2$-O-$CH_2$-C(=O)-Dxd;
M-$CH_2$-C(=O)-NH-$CH_2CH_2$-C(=O)-RL-NH-$CH_2CH_2$-O-$CH_2$-C(=O)-Dxd;
M-$CH_2$-C(=O)-NH-$CH_2CH_2CH_2$-C(=O)-RL-NH-$CH_2CH_2$-C(=O)-Dxd;
M-$CH_2$-C(=O)-NH-$CH_2CH_2CH_2$-C(=O)-RL-NH-$CH_2CH_2CH_2$-C(=O)-Dxd;
M-$CH_2$-C(=O)-NH-$CH_2CH_2CH_2$-C(=O)-RL-NH-$CH_2$-O-$CH_2$-C(=O)-Dxd;
M-$CH_2$-C(=O)-NH-$CH_2CH_2CH_2$-C(=O)-RL-NH-$CH_2CH_2$-O-$CH_2$-C(=O)-Dxd;
M-$CH_2$-C(=O)-NH-$CH_2CH_2CH_2CH_2$-C(=O)-RL-NH-$CH_2CH_2$-C(=O)-Dxd;
M-$CH_2$-C(=O)-NH-$CH_2CH_2CH_2CH_2$-C(=O)-RL-NH-$CH_2CH_2CH_2$-C(=O)-Dxd;
M-$CH_2$-C(=O)-NH-$CH_2CH_2CH_2CH_2$-C(=O)-RL-NH-$CH_2$-O-$CH_2$-C(=O)-Dxd;
M-$CH_2$-C(=O)-NH-$CH_2CH_2CH_2CH_2$-C(=O)-RL-NH-$CH_2CH_2$-O-$CH_2$-C(=O)-Dxd;
M-$CH_2$-C(=O)-NH-$CH_2CH_2CH_2CH_2CH_2$-C(=O)-RL-NH-$CH_2CH_2$-C(=O)-Dxd;
M-$CH_2$-C(=O)-NH-$CH_2CH_2CH_2CH_2CH_2$-C(=O)-RL-NH-$CH_2CH_2CH_2$-C(=O)-Dxd;
M-$CH_2$-C(=O)-NH-$CH_2CH_2CH_2CH_2CH_2$-C(=O)-RL-NH-$CH_2$-O-$CH_2$-C(=O)-Dxd;
M-$CH_2$-C(=O)-NH-$CH_2CH_2CH_2CH_2CH_2$-C(=O)-RL-NH-$CH_2CH_2$-O-$CH_2$-C(=O)-Dxd;
HS-$CH_2CH_2$-C(=O)-RL-NH-$CH_2CH_2$-C(=O)-Dxd;
HS-$CH_2CH_2CH_2$-C(=O)-RL-NH-$CH_2CH_2$-C(=O)-Dxd;
HS-$CH_2CH_2CH_2CH_2$-C(=O)-RL-NH-$CH_2CH_2$-C(=O)-Dxd;
HS-$CH_2CH_2CH_2CH_2CH_2$-C(=O)-RL-NH-$CH_2CH_2$-C(=O)-Dxd;
HS-$CH_2CH_2$-C(=O)-RL-NH-$CH_2CH_2CH_2$-C(=O)-Dxd;
HS-$CH_2CH_2CH_2$-C(=O)-RL-NH-$CH_2CH_2CH_2$-C(=O)-Dxd;
HS-$CH_2CH_2CH_2CH_2$-C(=O)-RL-NH-$CH_2CH_2CH_2$-C(=O)-Dxd;
HS-$CH_2CH_2CH_2CH_2CH_2$-C(=O)-RL-NH-$CH_2CH_2CH_2$-C(=O)-Dxd;
HS-$CH_2CH_2$-C(=O)-RL-NH-$CH_2$-O-$CH_2$-C(=O)-Dxd;
HS-$CH_2CH_2CH_2$-C(=O)-RL-NH-$CH_2$-O-$CH_2$-C(=O)-Dxd;
HS-$CH_2CH_2CH_2CH_2$-C(=O)-RL-NH-$CH_2$-O-$CH_2$-C(=O)-Dxd;
HS-$CH_2CH_2CH_2CH_2CH_2$-C(=O)-RL-NH-$CH_2$-O-$CH_2$-C(=O)-Dxd;

$HS-CH_2CH_2-C(=O)-RL-NH-CH_2CH_2-O-CH_2-C(=O)-Dxd$;
$HS-CH_2CH_2CH_2-C(=O)-RL-NH-CH_2CH_2-O-CH_2-C(=O)-Dxd$;
$HS-CH_2CH_2CH_2CH_2-C(=O)-RL-NH-CH_2CH_2-O-CH_2-C(=O)-Dxd$;
$HS-CH_2CH_2CH_2CH_2CH_2-C(=O)-RL-NH-CH_2CH_2-O-CH_2-C(=O)-Dxd$;
$HS-CH_2CH_2-C(=O)-NH-CH_2CH_2O-CH_2CH_2O-CH_2CH_2-C(=O)-RL-NH-CH_2CH_2CH_2-C(=O)-Dxd$;
$HS-CH_2CH_2-C(=O)-NH-CH_2CH_2O-CH_2CH_2O-CH_2CH_2O-CH_2CH_2-C(=O)-RL-NH-CH_2CH_2CH_2-C(=O)-Dxd$; or
$HS-CH_2CH_2-C(=O)-NH-CH_2CH_2O-CH_2CH_2O-CH_2CH_2O-CH_2CH_2O-CH_2CH_2-C(=O)-RL-NH-CH_2CH_2CH_2-C(=O)-Dxd$;

wherein,

(maleimid-N-yl)- has the following structure:

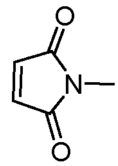

,

wherein the nitrogen atom is a group at an attachment position;
(pyrrolidine-2,5-dione-N-yl)- has the following structure:

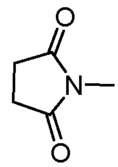

,

wherein the nitrogen atom is a group at an attachment position;
-RL- represents the following structure: -Val-Ala-Gly-Phe-Gly- (VAGFG), -Val-Ala-Ser-Gly-Phe-Gly- (VASGFG), or -Val-Ala-Ser-Ser-Gly-Phe-Gly- (VASSGFG);
-Dxd has the following structure:

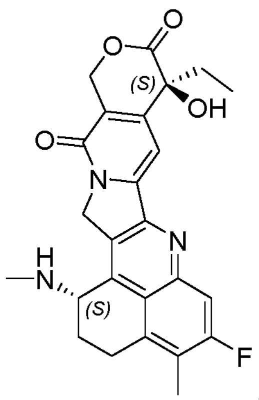

,

wherein the nitrogen atom of the amino group at position 1 is a group at an attachment position;
M represents a halogen atom.

**32.** The linker-drug according to any one of claims 23-31, wherein the linker-drug unit is selected from the following structures:

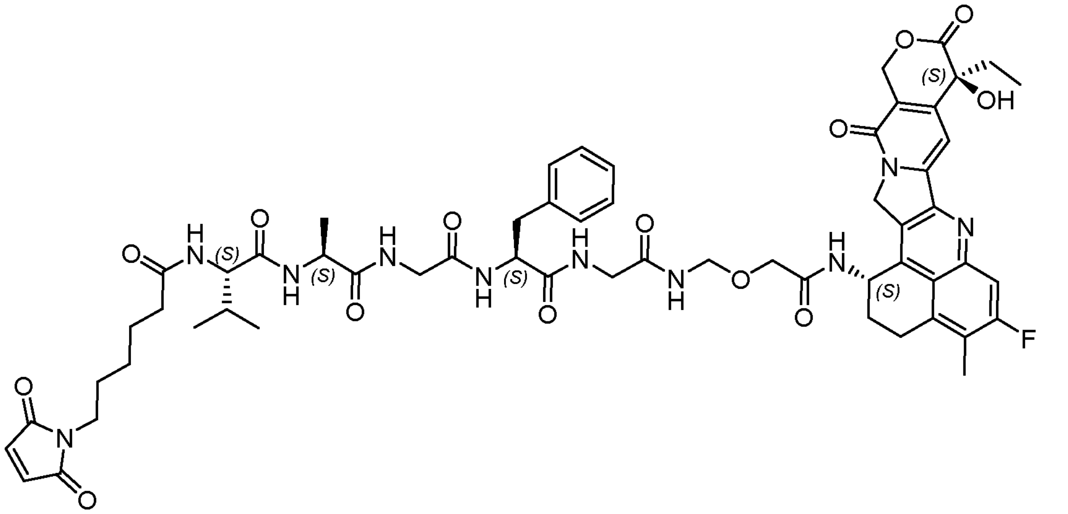

;

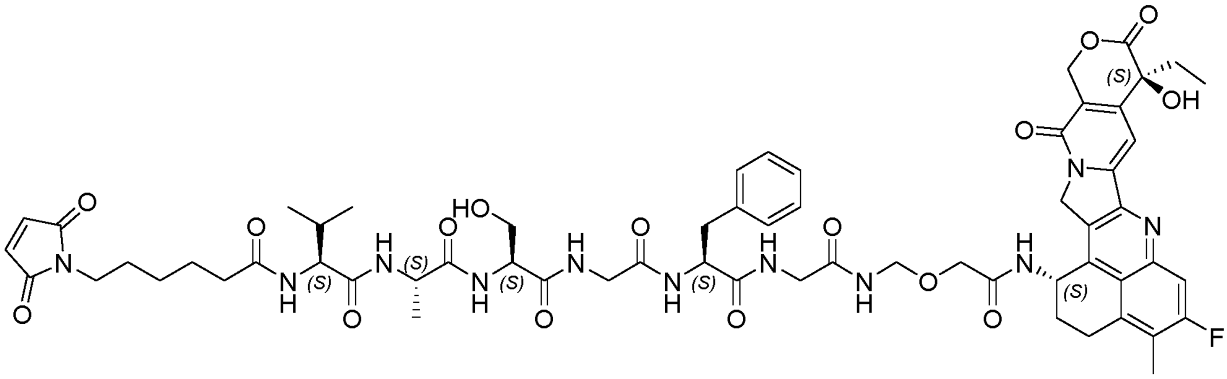

;

and

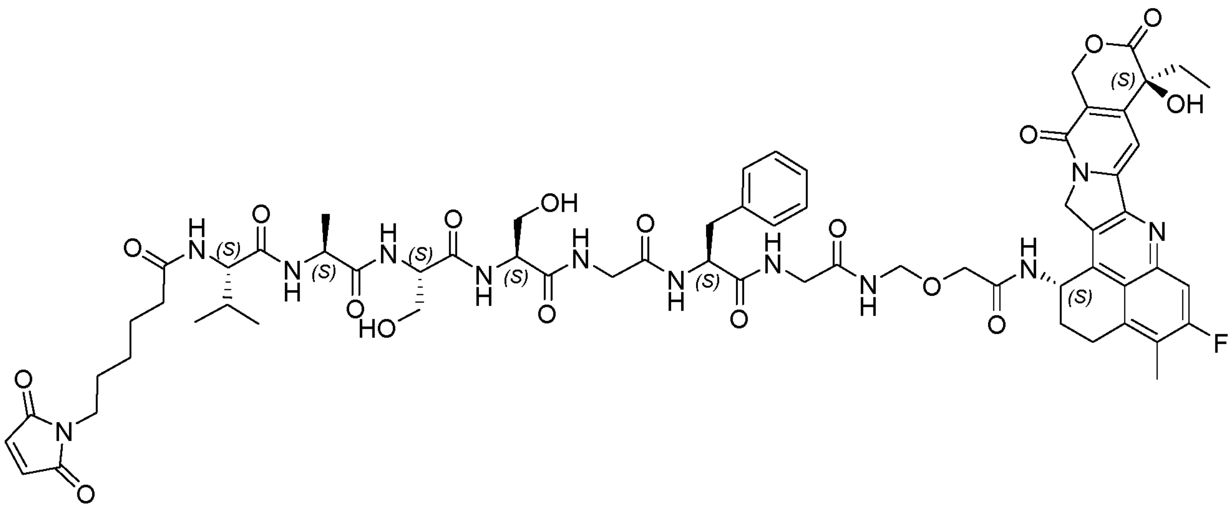

.

**33.** A conjugate, comprising the linker according to any one of claims 1-22.

**34.** The conjugate according to claim 33, wherein the conjugate comprises a ligand.

**35.** The conjugate according to claim 34, wherein the ligand targets a cell surface receptor or a tumor-associated antigen.

**36.** The conjugate according to claim 35, wherein the cell surface receptor or the tumor-associated antigen comprises: HER2, HER3, claudin18.2, folate receptor alpha (FRα), BCMA, PSMA, TROP-2, CD19, CD20, CD22, CD30, CD79b, EGFR, c-Met, or CEACAM5.

**37.** The conjugate according to any one of claims 34-36, wherein the ligand comprises an antibody or an antigen-binding fragment thereof.

**38.** The conjugate according to claim 37, wherein the antibody comprises a monoclonal antibody, a polyclonal antibody, a dimer, a multimer, a multispecific antibody, an intact antibody, an antibody fragment, a human antibody, a humanized antibody, or a chimeric antibody.

**39.** The conjugate according to claim 37, wherein the antigen-binding fragment comprises a Fab, a Fab', an Fv fragment, an F(ab')2, an scFv, a di-scFv, and/or a dAb.

**40.** The conjugate according to any one of claims 37-39, wherein the antibody is a monoclonal antibody.

**41.** The conjugate according to any one of claims 37-40, wherein the antibody is a human antibody, a humanized antibody, or a chimeric antibody.

**42.** The conjugate according to any one of claims 33-41, wherein the conjugate comprises a ligand-drug conjugate, and has the following structure: $C\text{-}(L\text{-}D)_m$, wherein, C represents a ligand unit, L represents a linker, D represents a drug unit, m represents any number from 1 to 10.

**43.** The conjugate according to any one of claims 33-42, wherein the ligand-drug conjugate is an antibody-drug conjugate (ADC), and has the following structure: $Ab\text{-}(L\text{-}D)_m$, wherein, Ab represents an antibody or an antigen-binding fragment thereof, L represents a linker, D represents a drug unit, m represents any number from 1 to 10.

**44.** The conjugate according to any one of claims 42-43, wherein the drug unit comprises a cytotoxic agent.

**45.** The conjugate according to any one of claims 42-44, wherein the drug unit comprises: amanitins, anthracyclines, auristatins, baccatins, calicheamicins, camptothecins, cemadotins, colchicines, colcimids, combretastatins, cryptophycins, discodermolides, duocarmycins, docetaxel, doxorubicin, duocarmycins, echinomycins, eleutherobins, epothilones, estramustines, lexitropsins, maytansines, maytansinoids, methotrexate, netropsins, pyrrolo[2,1-c][1,4] benzodiazepines (PBDs), puromycins, rhizoxins, SN-38, taxanes, tubulysins, vinca alkaloids, or derivatives thereof.

**46.** The conjugate according to any one of claims 42-45, wherein the drug unit comprises a DNA topoisomerase I inhibitor.

**47.** The conjugate according to any one of claims 42-46, wherein the drug unit comprises camptothecin or a derivative thereof.

**48.** The conjugate according to any one of claims 42-47, wherein the drug unit has the following structure:

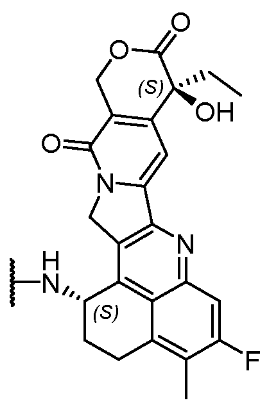

Dxd

wherein the nitrogen atom of the amino group at position 1 is a group at an attachment position.

**49.** The conjugate according to any one of claims 43-48, wherein the antibody-drug conjugate is selected from the following structures:

Ab-[-(succinimid-3-yl-N)-$CH_2CH_2$-C(=O)-RL-NH-$CH_2CH_2$-C(=O)-Dxd]$_m$;
Ab-[-(succinimid-3-yl-N)-$CH_2CH_2$-C(=O)-RL-NH-$CH_2CH_2CH_2$-C(=O)-Dxd]$_m$;
Ab-[-(succinimid-3-yl-N)-$CH_2CH_2CH_2CH_2CH_2$-C(=O)-RL-NH-$CH_2CH_2$-C(=O)-Dxd]$_m$;
Ab-[-(succinimid-3-yl-N)-$CH_2CH_2CH_2CH_2CH_2$-C(=O)-RL-NH-$CH_2CH_2CH_2$-C(=O)-Dxd]$_m$;
Ab-[-(succinimid-3-yl-N)-$CH_2CH_2CH_2CH_2CH_2$-C(=O)-RL-NH-$CH_2CH_2CH_2CH_2CH_2$-C(=O)-Dxd]$_m$;
Ab-[-(succinimid-3-yl-N)-$CH_2CH_2CH_2CH_2CH_2$-C(=O)-RL-NH-$CH_2$-O-$CH_2$-C(=O)-Dxd]$_m$;
Ab-[-(succinimid-3-yl-N)-$CH_2CH_2CH_2CH_2CH_2$-C(=O)-RL-NH-$CH_2CH_2$-O-$CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-RL-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-RL-NH-$CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-RL-NH-$CH_2CH_2$-C(=O)-Dxd]$_m$;
Ab-[-(succinimid-3-yl-N)-$CH_2CH_2$-C(=O)-VASGFG-NH-$CH_2CH_2$-C(=O)-Dxd]$_m$;
Ab-[-(succinimid-3-yl-N)-$CH_2CH_2$-C(=O)-VASGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2CH_2CH_2CH_2$-C(=O)-Dxd]$_m$;
Ab-[-(succinimid-3-yl-N)-$CH_2CH_2CH_2CH_2CH_2$-C(=O)-VASGFG-NH-$CH_2$-O-$CH_2$-C(=O)-Dxd]$_m$;
Ab-[-(succinimid-3-yl-N)-$CH_2CH_2CH_2CH_2CH_2$-C(=O)-VASGFG-NH-$CH_2CH_2$-O-$CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-VASGFG-NH-$CH_2CH_2CH_2$-C(=O)-Dxd]m;
Ab-[-(succinimid-3-yl-N)-$CH_2CH_2$-C(=O)-NH-$CH_2CH_2O$-$CH_2CH_2O$-$CH_2CH_2$-C(=O)-VASGFG-NH-$CH_2CH_2$-C(=O)-Dxd]m;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VASGFG-NH-$CH_2CH_2CH_2$-C(=O)-Dxd]m;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VASGFG-NH-$CH_2CH_2$-C(=O)-Dxd]$_m$;
Ab-[-(succinimid-3-yl-N)-$CH_2CH_2$-C(=O)-VASSGFG-NH-$CH_2CH_2$-C(=O)-Dxd]$_m$;
Ab-[-(succinimid-3-yl-N)-$CH_2CH_2$-C(=O)-VASSGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2CH_2CH_2CH_2$-C(=O)-Dxd]$_m$;
Ab-[-(succinimid-3-yl-N)-$CH_2CH_2CH_2CH_2CH_2$-C(=O)-VASSGFG-NH-$CH_2$-O-$CH_2$-C(=O)-Dxd]$_m$;
Ab-[-(succinimid-3-yl-N)-$CH_2CH_2CH_2CH_2CH_2$-C(=O)-VASSGFG-NH-$CH_2CH_2$-O-$CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-VASSGFG-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2$-C(=O)-VASSGFG-NH-$CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VASSGFG-NH-$CH_2CH_2CH_2$-C(=O)-Dxd]$_m$;
Ab-[-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VASSGFG-NH-$CH_2CH_2$-C(=O)-Dxd]$_m$;
Ab-[-$CH_2$-C(=O)-NH-$CH_2CH_2$-C(=O)-RL-NH-$CH_2CH_2$-C(=O)-Dxd]$_m$;
Ab-[-$CH_2$-C(=O)-NH-$CH_2CH_2$-C(=O)-RL-NH-$CH_2CH_2CH_2$-C(=O)-Dxd]$_m$;
Ab-[-$CH_2$-C(=O)-NH-$CH_2CH_2$-C(=O)-RL-NH-$CH_2$-O-$CH_2$-C(=O)-Dxd]$_m$;
Ab-[-$CH_2$-C(=O)-NH-$CH_2CH_2$-C(=O)-RL-NH-$CH_2CH_2$-O-$CH_2$-C(=O)-Dxd]$_m$;
Ab-[-$CH_2$-C(=O)-NH-$CH_2CH_2CH_2$-C(=O)-RL-NH-$CH_2CH_2$-C(=O)-Dxd]$_m$;
Ab-[-$CH_2$-C(=O)-NH-$CH_2CH_2CH_2$-C(=O)-RL-NH-$CH_2CH_2CH_2$-C(=O)-Dxd]$_m$;
Ab-[-$CH_2$-C(=O)-NH-$CH_2CH_2CH_2$-C(=O)-RL-NH-$CH_2$-O-$CH_2$-C(=O)-Dxd]$_m$;
Ab-[-$CH_2$-C(=O)-NH-$CH_2CH_2CH_2$-C(=O)-RL-NH-$CH_2CH_2$-O-$CH_2$-C(=O)-Dxd]$_m$;

$Ab\text{-}[\text{-}CH_2\text{-}C(=O)\text{-}NH\text{-}CH_2CH_2CH_2CH_2\text{-}C(=O)\text{-}RL\text{-}NH\text{-}CH_2CH_2\text{-}C(=O)\text{-}Dxd]_m$;
$Ab\text{-}[\text{-}CH_2\text{-}C(=O)\text{-}NH\text{-}CH_2CH_2CH_2CH_2\text{-}C(=O)\text{-}RL\text{-}NH\text{-}CH_2CH_2CH_2\text{-}C(=O)\text{-}Dxd]_m$;
$Ab\text{-}[\text{-}CH_2\text{-}C(=O)\text{-}NH\text{-}CH_2CH_2CH_2CH_2\text{-}C(=O)\text{-}RL\text{-}NH\text{-}CH_2\text{-}O\text{-}CH_2\text{-}C(=O)\text{-}Dxd]_m$;
$Ab\text{-}[\text{-}CH_2\text{-}C(=O)\text{-}NH\text{-}CH_2CH_2CH_2CH_2\text{-}C(=O)\text{-}RL\text{-}NH\text{-}CH_2CH_2\text{-}O\text{-}CH_2\text{-}C(=O)\text{-}Dxd]_m$;
$Ab\text{-}[\text{-}CH_2\text{-}C(=O)\text{-}NH\text{-}CH_2CH_2CH_2CH_2CH_2\text{-}C(=O)\text{-}RL\text{-}NH\text{-}CH_2CH_2\text{-}C(=O)\text{-}Dxd]_m$;
$Ab\text{-}[\text{-}CH_2\text{-}C(=O)\text{-}NH\text{-}CH_2CH_2CH_2CH_2CH_2\text{-}C(=O)\text{-}RL\text{-}NH\text{-}CH_2CH_2CH_2\text{-}C(=O)\text{-}Dxd]_m$;
$Ab\text{-}[\text{-}CH_2\text{-}C(=O)\text{-}NH\text{-}CH_2CH_2CH_2CH_2CH_2\text{-}C(=O)\text{-}RL\text{-}NH\text{-}CH_2\text{-}O\text{-}CH_2\text{-}C(=O)\text{-}Dxd]_m$;
$Ab\text{-}[\text{-}CH_2\text{-}C(=O)\text{-}NH\text{-}CH_2CH_2CH_2CH_2CH_2\text{-}C(=O)\text{-}RL\text{-}NH\text{-}CH_2CH_2\text{-}O\text{-}CH_2\text{-}C(=O)\text{-}Dxd]_m$;
$Ab\text{-}[\text{-}S\text{-}CH_2CH_2\text{-}C(=O)\text{-}RL\text{-}NH\text{-}CH_2CH_2\text{-}C(=O)\text{-}Dxd]_m$;
$Ab\text{-}[\text{-}S\text{-}CH_2CH_2CH_2\text{-}C(=O)\text{-}RL\text{-}NH\text{-}CH_2CH_2\text{-}C(=O)\text{-}Dxd]_m$;
$Ab\text{-}[\text{-}S\text{-}CH_2CH_2CH_2CH_2\text{-}C(=O)\text{-}RL\text{-}NH\text{-}CH_2CH_2\text{-}C(=O)\text{-}Dxd]_m$;
$Ab\text{-}[\text{-}S\text{-}CH_2CH_2CH_2CH_2CH_2\text{-}C(=O)\text{-}RL\text{-}NH\text{-}CH_2CH_2\text{-}C(=O)\text{-}Dxd]_m$;
$Ab\text{-}[\text{-}S\text{-}CH_2CH_2\text{-}C(=O)\text{-}RL\text{-}NH\text{-}CH_2CH_2CH_2\text{-}C(=O)\text{-}Dxd]_m$;
$Ab\text{-}[\text{-}S\text{-}CH_2CH_2CH_2\text{-}C(=O)\text{-}RL\text{-}NH\text{-}CH_2CH_2CH_2\text{-}C(=O)\text{-}Dxd]_m$;
$Ab\text{-}[\text{-}S\text{-}CH_2CH_2CH_2CH_2\text{-}C(=O)\text{-}RL\text{-}NH\text{-}CH_2CH_2CH_2\text{-}C(=O)\text{-}Dxd]_m$;
$Ab\text{-}[\text{-}S\text{-}CH_2CH_2CH_2CH_2CH_2\text{-}C(=O)\text{-}RL\text{-}NH\text{-}CH_2CH_2CH_2\text{-}C(=O)\text{-}Dxd]_m$;
$Ab\text{-}[\text{-}S\text{-}CH_2CH_2\text{-}C(=O)\text{-}RL\text{-}NH\text{-}CH_2\text{-}O\text{-}CH_2\text{-}C(=O)\text{-}Dxd]_m$;
$Ab\text{-}[\text{-}S\text{-}CH_2CH_2CH_2\text{-}C(=O)\text{-}RL\text{-}NH\text{-}CH_2\text{-}O\text{-}CH_2\text{-}C(=O)\text{-}Dxd]_m$;
$Ab\text{-}[\text{-}S\text{-}CH_2CH_2CH_2CH_2\text{-}C(=O)\text{-}RL\text{-}NH\text{-}CH_2\text{-}O\text{-}CH_2\text{-}C(=O)\text{-}Dxd]_m$;
$Ab\text{-}[\text{-}S\text{-}CH_2CH_2CH_2CH_2CH_2\text{-}C(=O)\text{-}RL\text{-}NH\text{-}CH_2\text{-}O\text{-}CH_2\text{-}C(=O)\text{-}Dxd]_m$;
$Ab\text{-}[\text{-}S\text{-}CH_2CH_2\text{-}C(=O)\text{-}RL\text{-}NH\text{-}CH_2CH_2\text{-}O\text{-}CH_2\text{-}C(=O)\text{-}Dxd]_m$;
$Ab\text{-}[\text{-}S\text{-}CH_2CH_2CH_2\text{-}C(=O)\text{-}RL\text{-}NH\text{-}CH_2CH_2\text{-}O\text{-}CH_2\text{-}C(=O)\text{-}Dxd]_m$;
$Ab\text{-}[\text{-}S\text{-}CH_2CH_2CH_2CH_2\text{-}C(=O)\text{-}RL\text{-}NH\text{-}CH_2CH_2\text{-}O\text{-}CH_2\text{-}C(=O)\text{-}Dxd]_m$;
$Ab\text{-}[\text{-}S\text{-}CH_2CH_2CH_2CH_2CH_2\text{-}C(=O)\text{-}RL\text{-}NH\text{-}CH_2CH_2\text{-}O\text{-}CH_2\text{-}C(=O)\text{-}Dxd]_m$;
$Ab\text{-}[\text{-}S\text{-}CH_2CH_2\text{-}C(=O)\text{-}NH\text{-}CH_2CH_2O\text{-}CH_2CH_2O\text{-}CH_2CH_2\text{-}C(=O)\text{-}RL\text{-}NH\text{-}CH_2CH_2CH_2\text{-}C(=O)\text{-}Dxd]_m$;
$Ab\text{-}[\text{-}S\text{-}CH_2CH_2\text{-}C(=O)\text{-}NH\text{-}CH_2CH_2O\text{-}CH_2CH_2O\text{-}CH_2CH_2O\text{-}CH_2CH_2\text{-}C(=O)\text{-}RL\text{-}NH\text{-}CH_2CH_2CH_2\text{-}C(=O)\text{-}Dxd]_m$; and
$Ab\text{-}[\text{-}S\text{-}CH_2CH_2\text{-}C(=O)\text{-}NH\text{-}CH_2CH_2O\text{-}CH_2CH_2O\text{-}CH_2CH_2O\text{-}CH_2CH_2O\text{-}CH_2CH_2\text{-}C(=O)\text{-}RL\text{-}NH\text{-}CH_2CH_2CH_2\text{-}C(=O)\text{-}Dxd]_m$;

wherein,

-(succinimid-3-yl-N)- has the following structure:

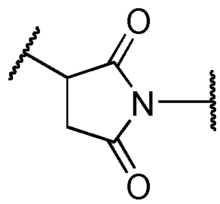
,

wherein the wavy line at position 3 of the structure represents an attachment position to a ligand unit, the wavy line at the N atom of position 1 represents an attachment position to A;
-RL- represents the following structure: -Val-Ala-Gly-Phe-Gly- (VAGFG), -Val-Ala-Ser-Gly-Phe-Gly- (VASGFG), or -Val-Ala-Ser-Ser-Gly-Phe-Gly- (VASSGFG);
-Dxd has the following structure:

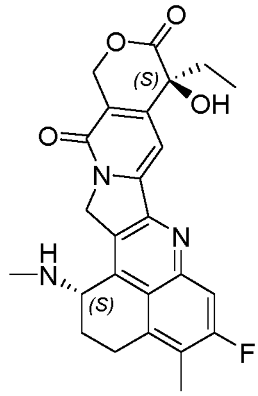

,

wherein the nitrogen atom of the amino group at position 1 is a group at an attachment position;
Ab represents an antibody or an antigen-binding fragment thereof, m represents any number from 1 to 10.

**50.** The conjugate according to any one of claims 43-49, wherein the antibody-drug conjugate is selected from the following structures:

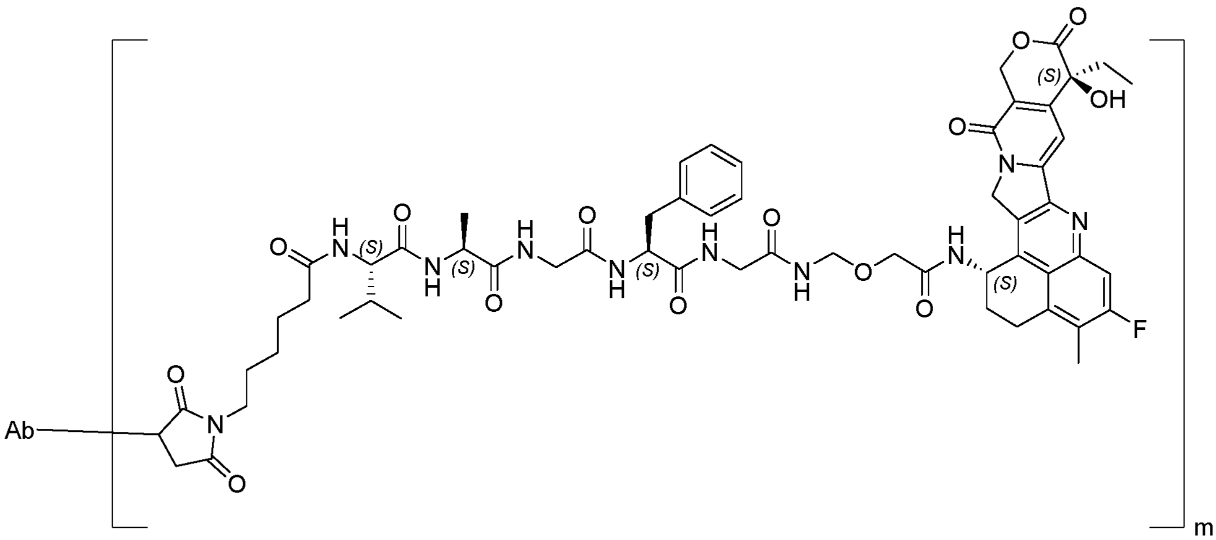

,

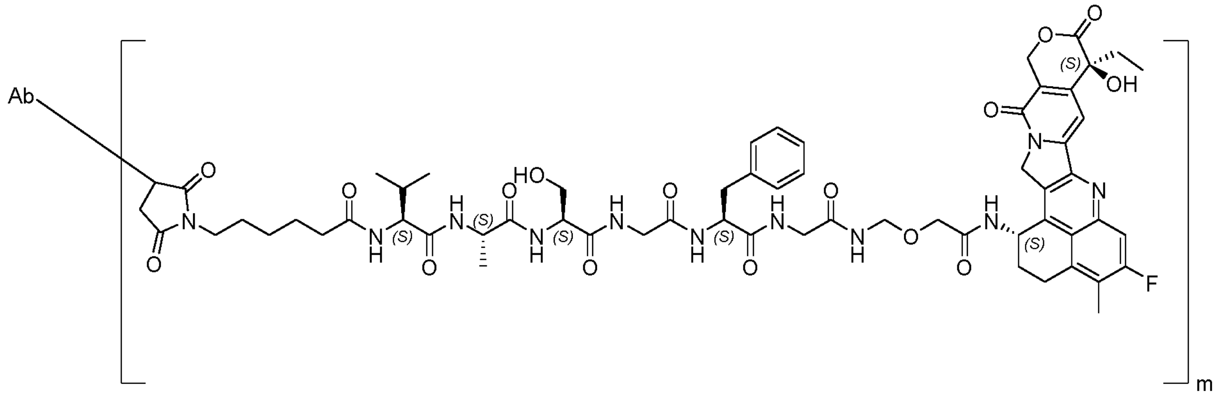

,

and

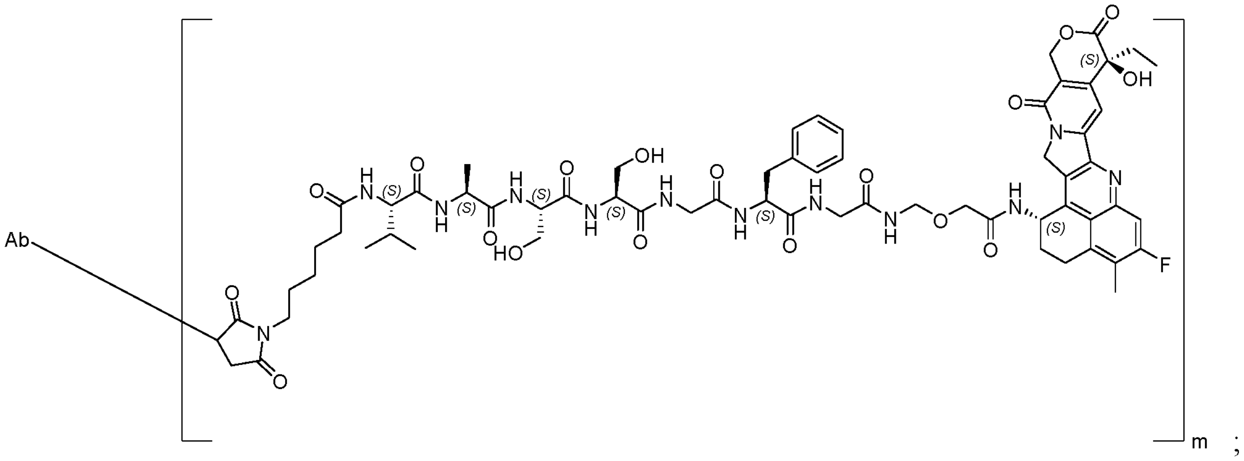

wherein, Ab represents an antibody or an antigen-binding fragment thereof, m is any number from 1 to 10.

**51.** The conjugate according to any one of claims 37-50, wherein the antibody or the antigen-binding fragment thereof comprises: an anti-HER2 antibody, an anti-HER3 antibody, an anti-claudin18.2 antibody, an anti-FRα antibody, or an antigen-binding fragment thereof.

**52.** A pharmaceutical composition, comprising the conjugate according to any one of preceding claims 33-51 and optionally a pharmaceutically acceptable carrier.

**53.** Use of the linker according to any one of claims 1-22, the linker-drug according to any one of claims 23-32, the conjugate according to any one of claims 33-51, or the pharmaceutical composition according to claim 52 in preparing a medicament for treating a disease or a condition.

**54.** The use according to claim 53, wherein the disease or condition comprises a tumor.

**55.** The use according to claim 54, wherein the disease or condition comprises a solid tumor and a hematologic tumor.

**56.** A method for treating a disease or a condition in a subject in need thereof, comprising administering to the subject an effective amount of the conjugate according to any one of claims 33-51.

**57.** The method according to claim 56, wherein the disease or condition comprises a tumor.

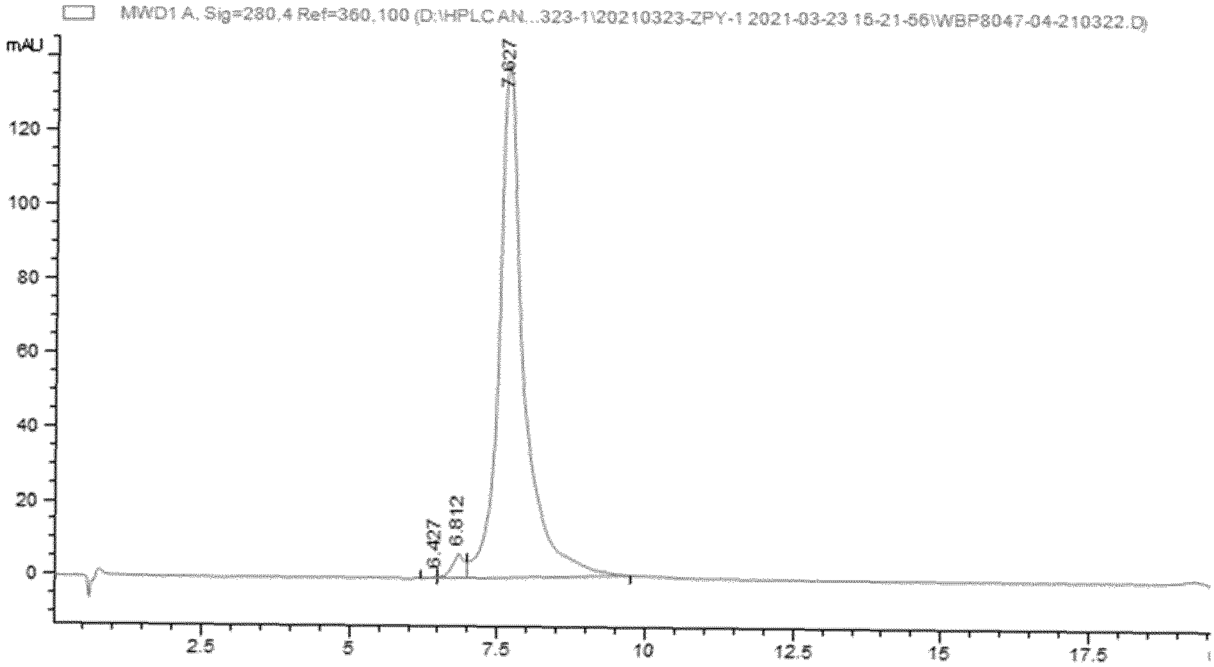
MWD1 A, Sig=280,4 Ref=360,100 (D:\HPLC AN...323-1\20210323-ZPY-1 2021-03-23 15-21-56\WBP8047-04-210322.D)
mAU
120
100
80
60
40
20
0
2.5
5
7.5
10
12.5
15
17.5
6.427
6.812
7.627

FIG. 1A

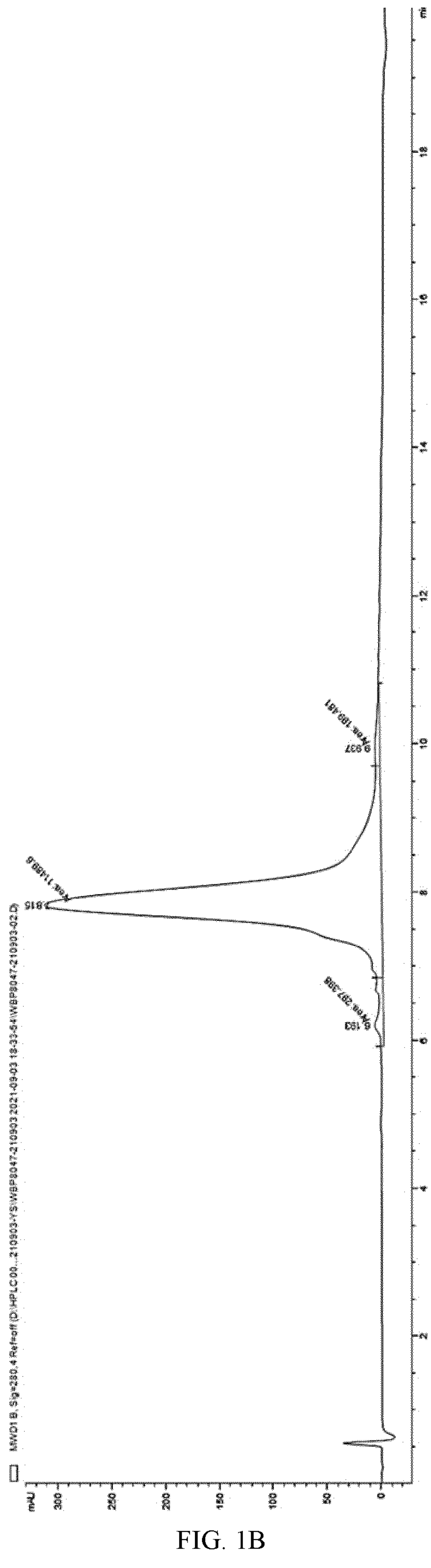

FIG. 1B

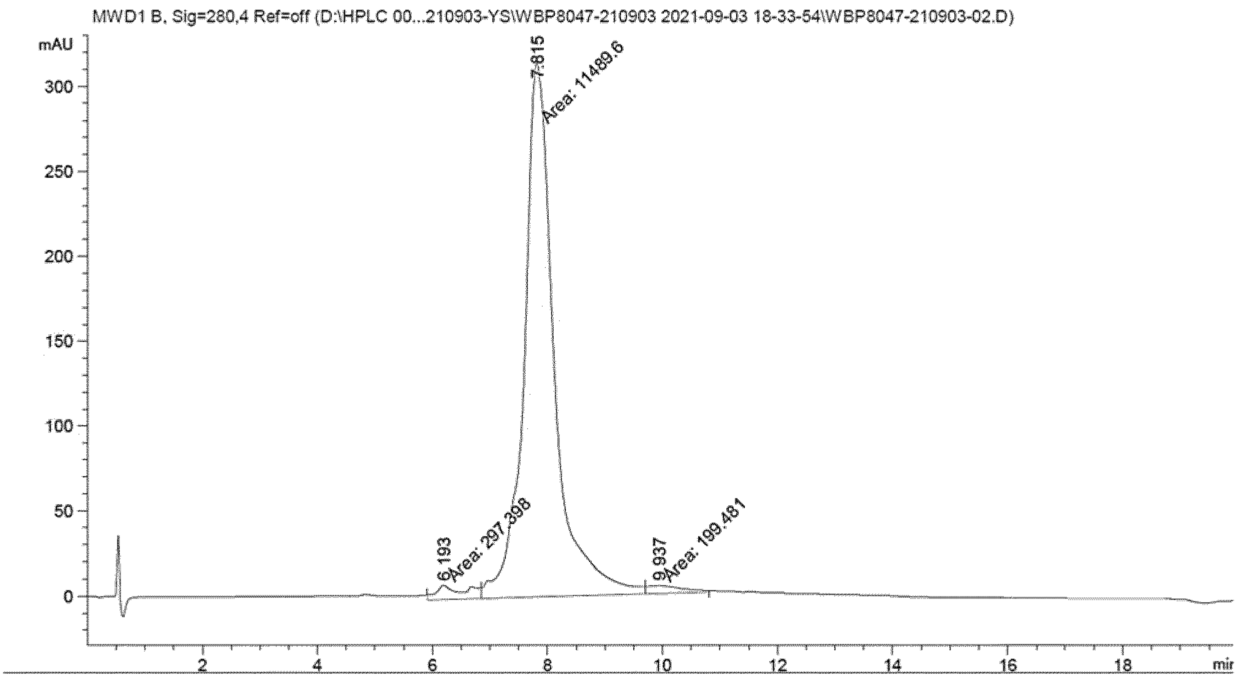
MWD1 B, Sig=280,4 Ref=off (D:\HPLC 00...210903-YS\WBP8047-210903 2021-09-03 18-33-54\WBP8047-210903-02.D)
mAU
300
250
200
150
100
50
0
7.815
Area: 11489.6
6.193
Area: 297.398
9.937
Area: 199.481
2
4
6
8
10
12
14
16
18
min

FIG. 1C

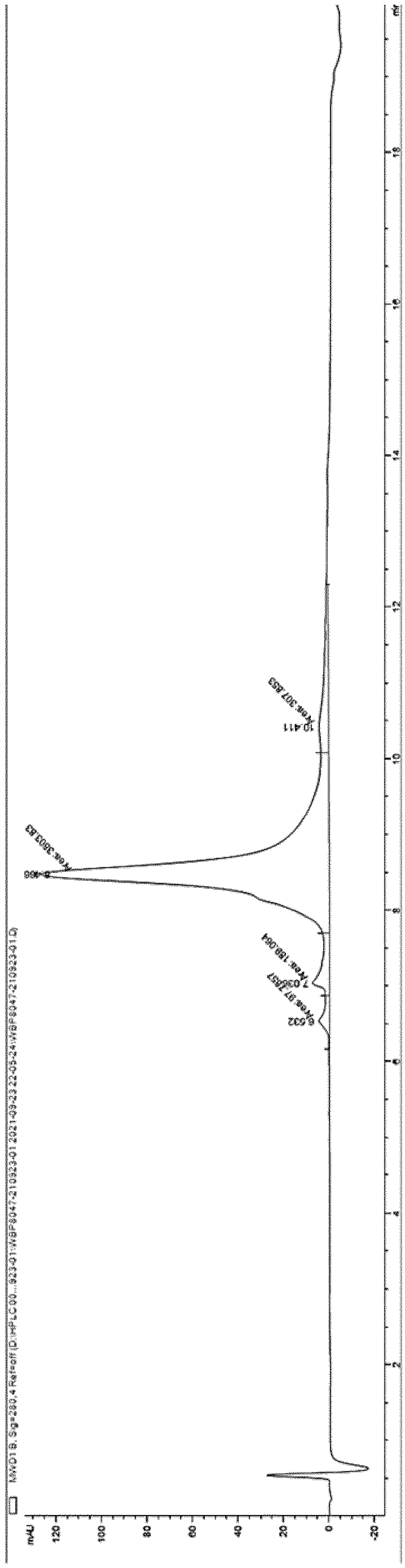

FIG. 1D

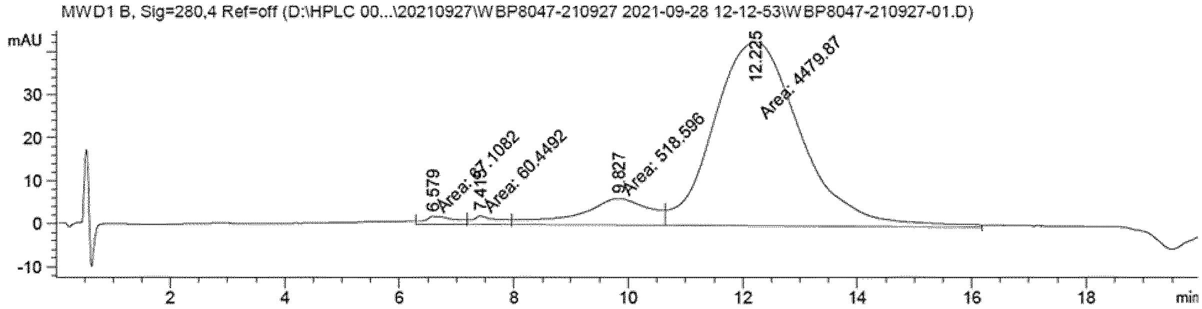


FIG. 1E

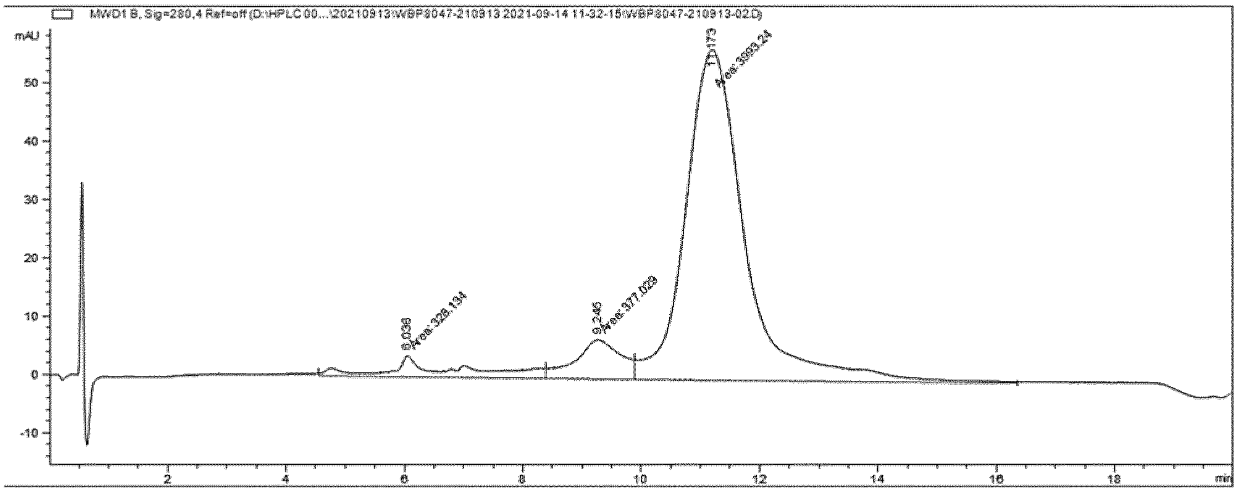


FIG. 1F

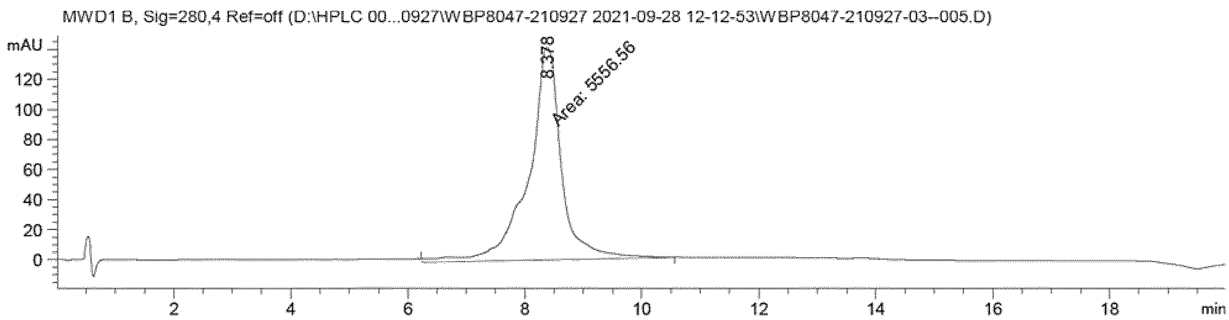


FIG. 1G

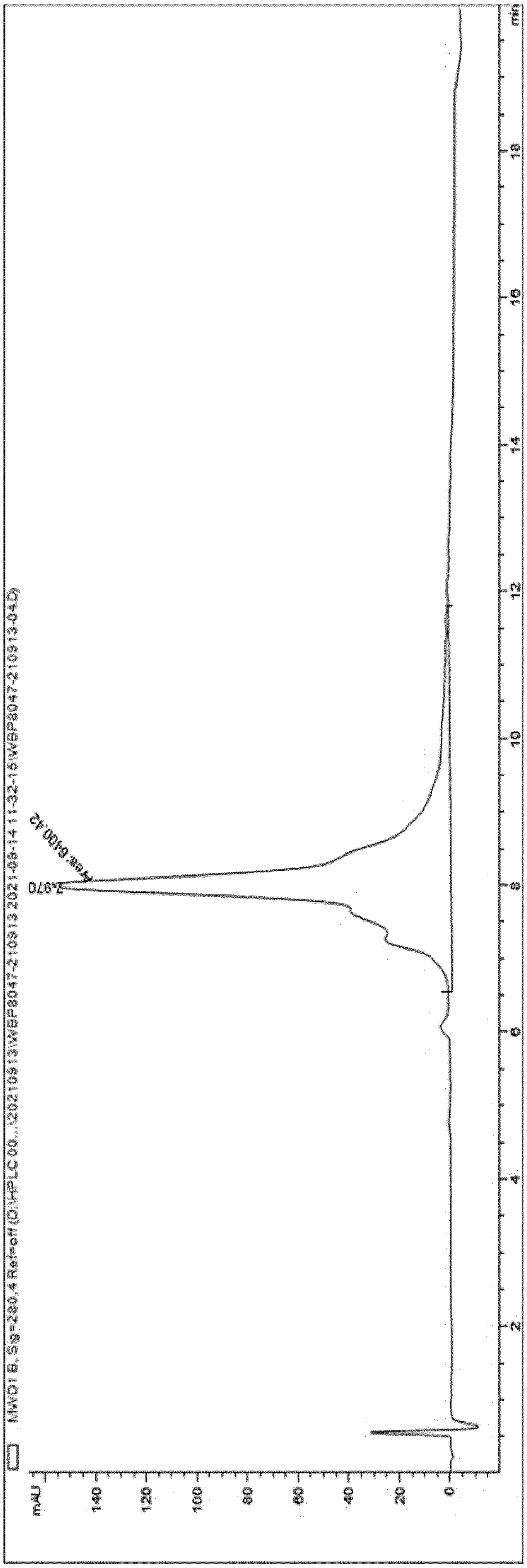
MWD1 B, Sig=280,4 Ref=off (D:\HPLC00...\20210913\WBP8047-210913 2021-09-14 11-32-15\WBP8047-210913-04.D)
mAU
140
120
100
80
60
40
20
0
7.970
Area: 6400.42
2
4
6
8
10
12
14
16
18
min

FIG. 1H

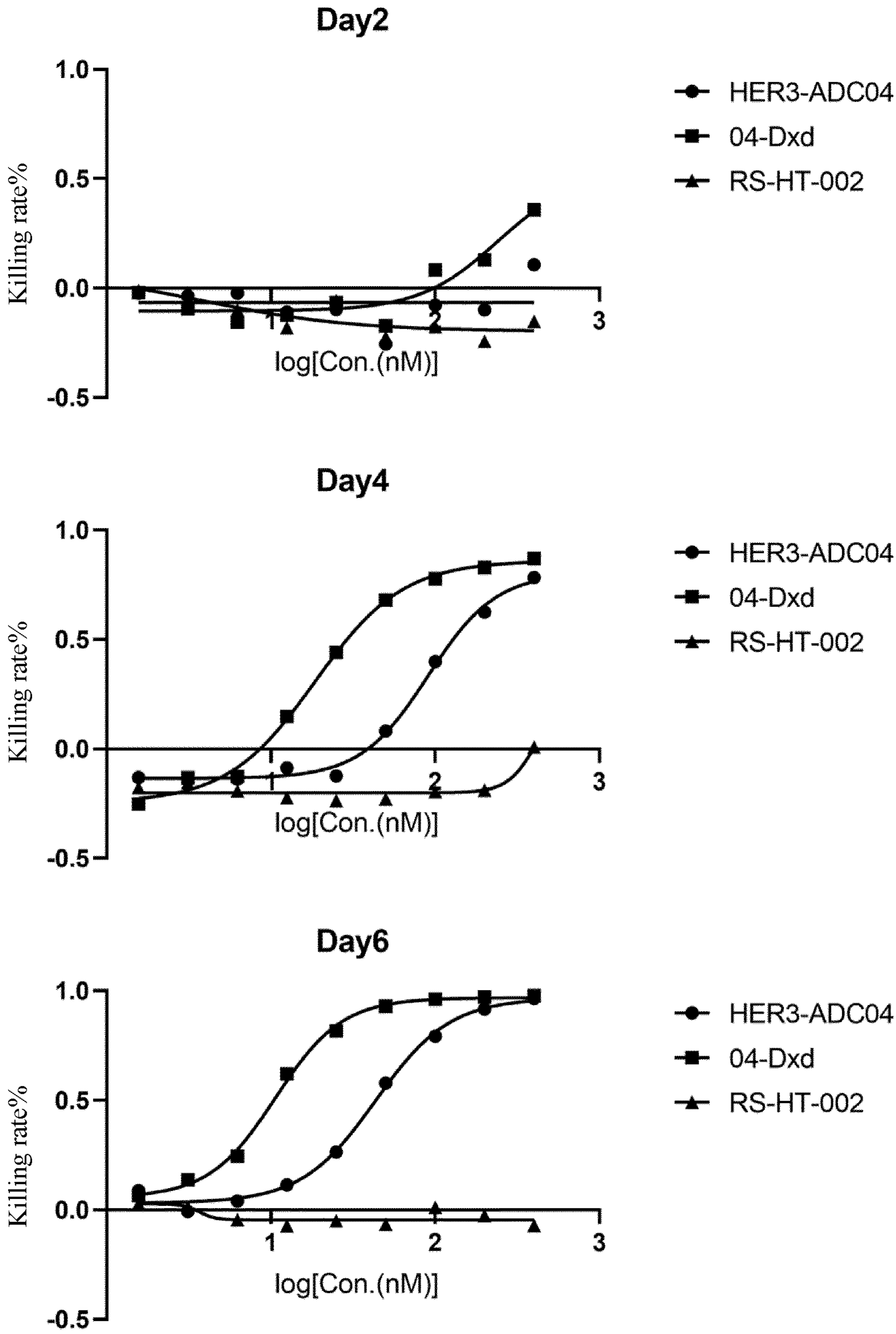
Day2
Killing rate%
1.0
0.5
0.0
-0.5
1
2
3
log[Con.(nM)]
HER3-ADC04
04-Dxd
RS-HT-002
Day4
Killing rate%
1.0
0.5
0.0
-0.5
1
2
3
log[Con.(nM)]
HER3-ADC04
04-Dxd
RS-HT-002
Day6
Killing rate%
1.0
0.5
0.0
-0.5
1
2
3
log[Con.(nM)]
HER3-ADC04
04-Dxd
RS-HT-002

FIG. 2A

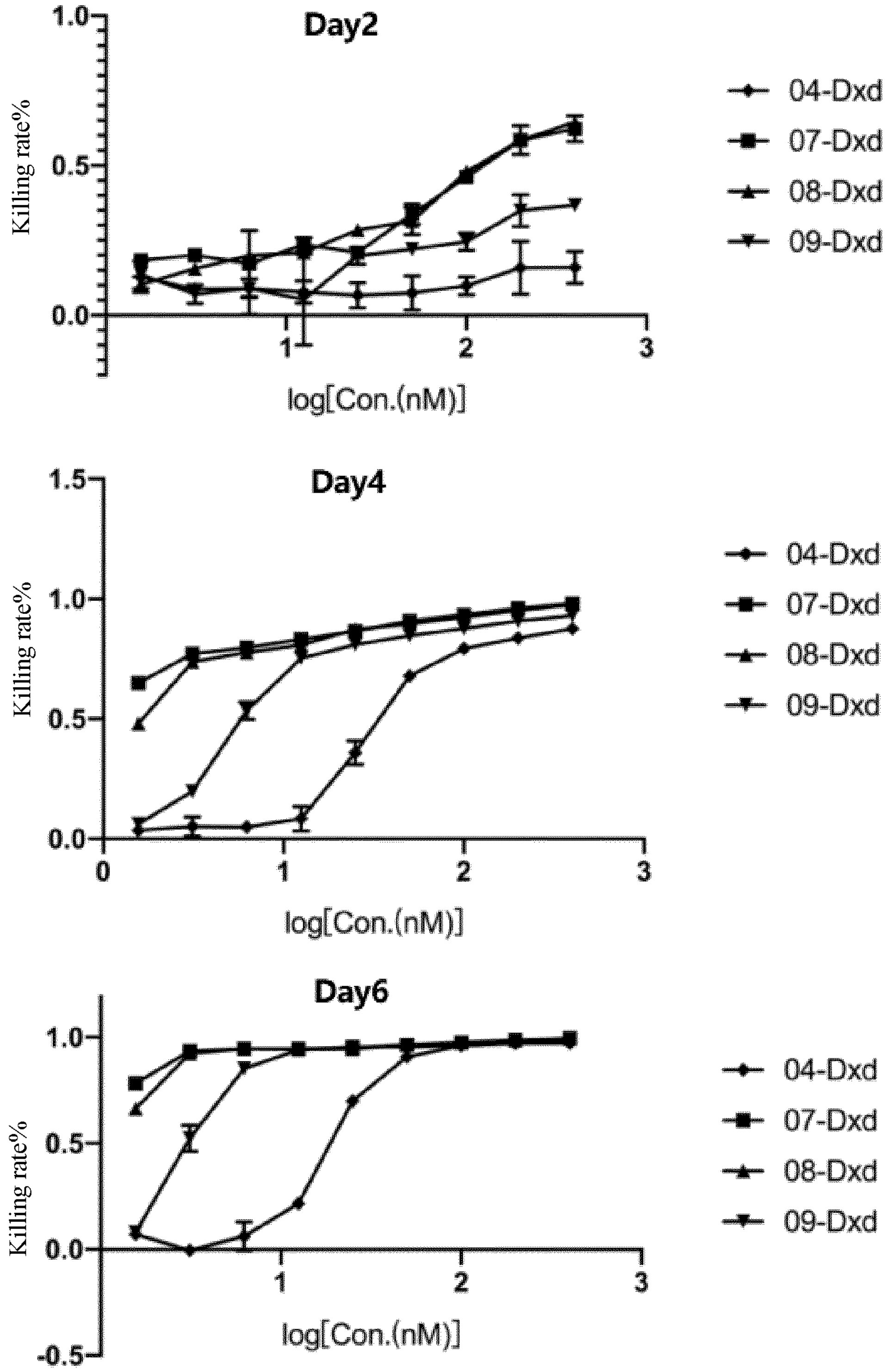
Day2
Killing rate%
1.0
0.5
0.0
1
2
3
log[Con.(nM)]
04-Dxd
07-Dxd
08-Dxd
09-Dxd
Day4
Killing rate%
1.5
1.0
0.5
0.0
0
1
2
3
log[Con.(nM)]
04-Dxd
07-Dxd
08-Dxd
09-Dxd
Day6
Killing rate%
1.0
0.5
0.0
-0.5
1
2
3
log[Con.(nM)]
04-Dxd
07-Dxd
08-Dxd
09-Dxd

FIG. 2B

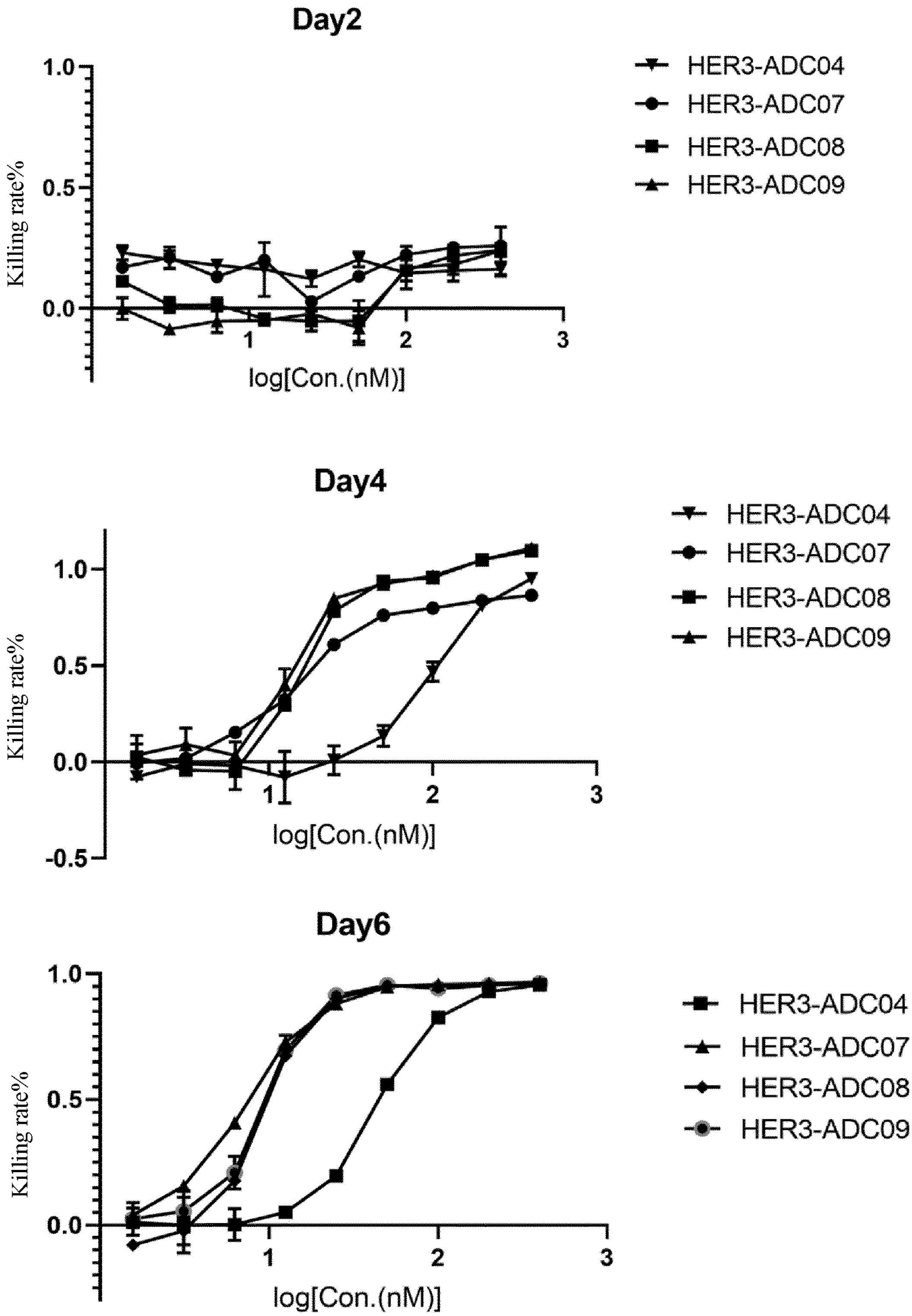
Day2
1.0
0.5
0.0
Killing rate%
1
2
3
log[Con.(nM)]
HER3-ADC04
HER3-ADC07
HER3-ADC08
HER3-ADC09
Day4
1.0
0.5
0.0
-0.5
Killing rate%
1
2
3
log[Con.(nM)]
HER3-ADC04
HER3-ADC07
HER3-ADC08
HER3-ADC09
Day6
1.0
0.5
0.0
Killing rate%
1
2
3
log[Con.(nM)]
HER3-ADC04
HER3-ADC07
HER3-ADC08
HER3-ADC09

FIG. 3

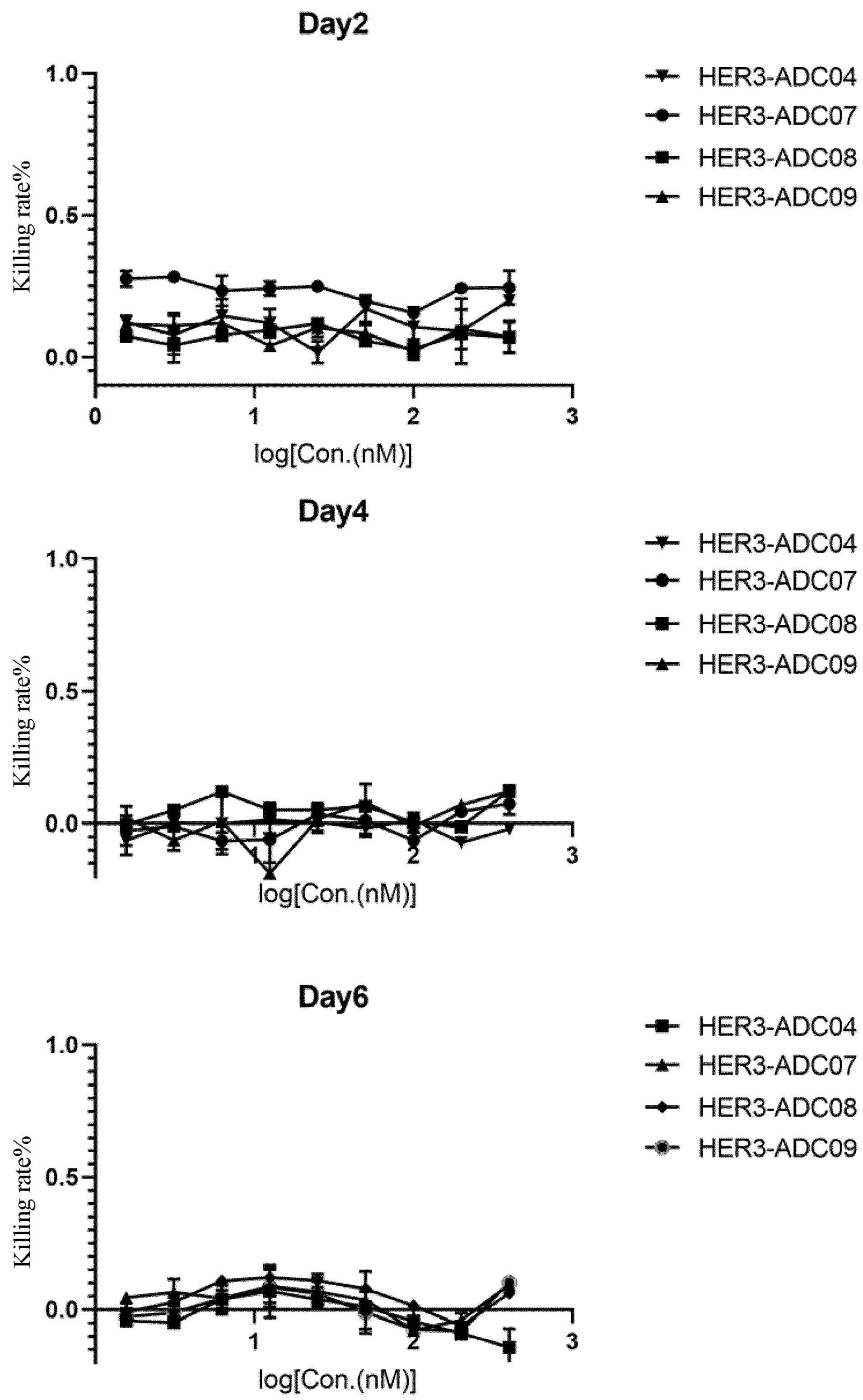
Day2
Killing rate%
1.0
0.5
0.0
0
1
2
3
log[Con.(nM)]
HER3-ADC04
HER3-ADC07
HER3-ADC08
HER3-ADC09
Day4
Killing rate%
1.0
0.5
0.0
1
2
3
log[Con.(nM)]
HER3-ADC04
HER3-ADC07
HER3-ADC08
HER3-ADC09
Day6
Killing rate%
1.0
0.5
0.0
1
2
3
log[Con.(nM)]
HER3-ADC04
HER3-ADC07
HER3-ADC08
HER3-ADC09

FIG. 4

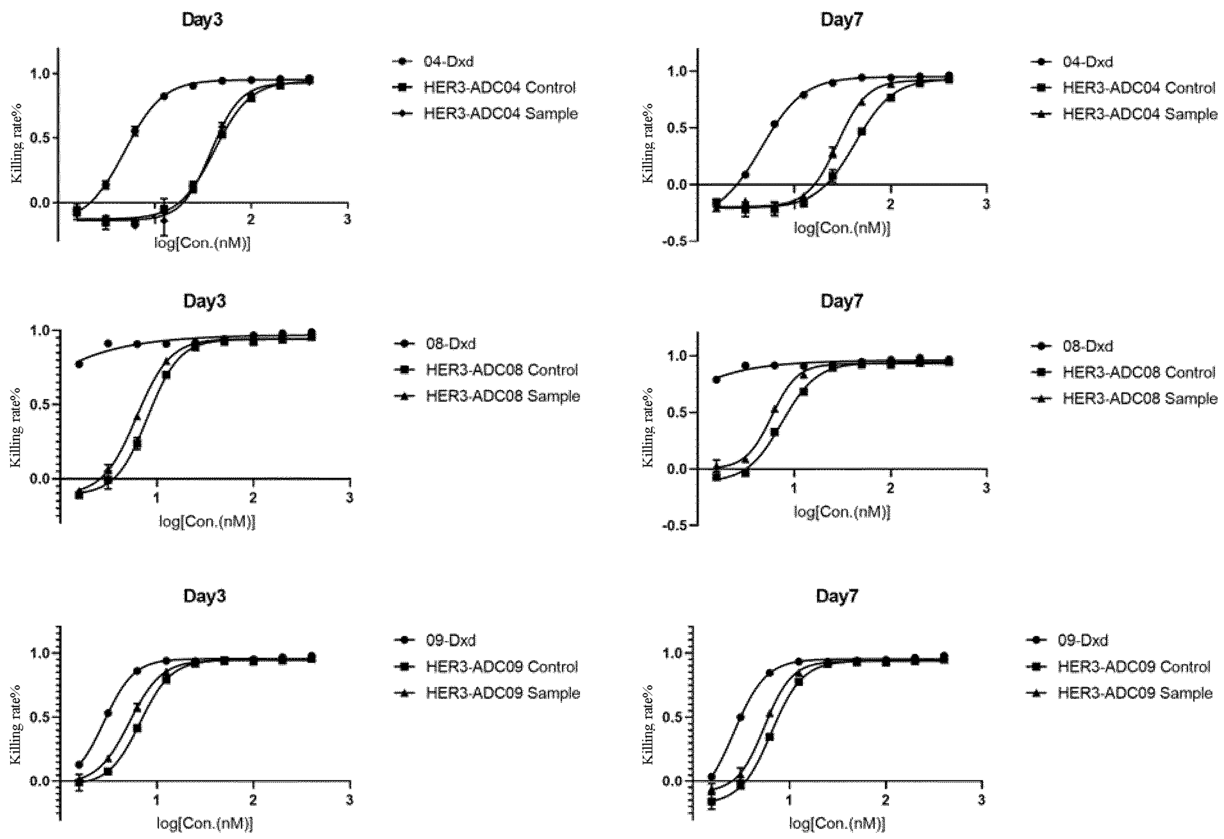
Day3
Day7
Killing rate%
log[Con.(nM)]
04-Dxd
HER3-ADC04 Control
HER3-ADC04 Sample
08-Dxd
HER3-ADC08 Control
HER3-ADC08 Sample
09-Dxd
HER3-ADC09 Control
HER3-ADC09 Sample

FIG. 5

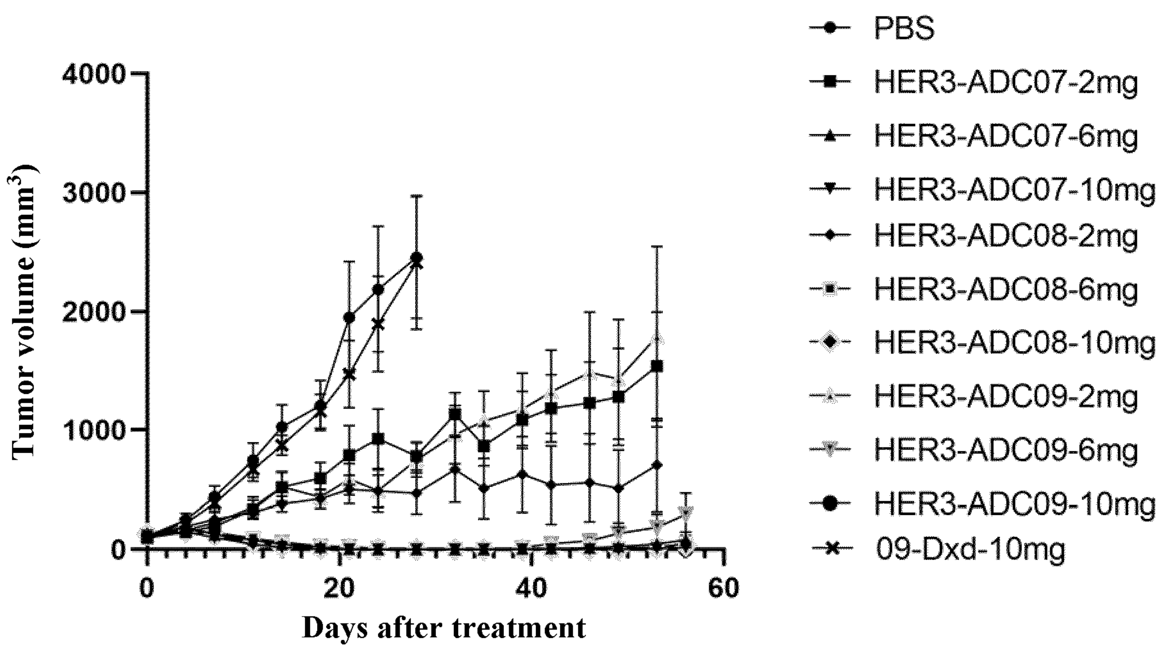
Tumor volume (mm3)
Days after treatment
PBS
HER3-ADC07-2mg
HER3-ADC07-6mg
HER3-ADC07-10mg
HER3-ADC08-2mg
HER3-ADC08-6mg
HER3-ADC08-10mg
HER3-ADC09-2mg
HER3-ADC09-6mg
HER3-ADC09-10mg
09-Dxd-10mg

FIG. 6

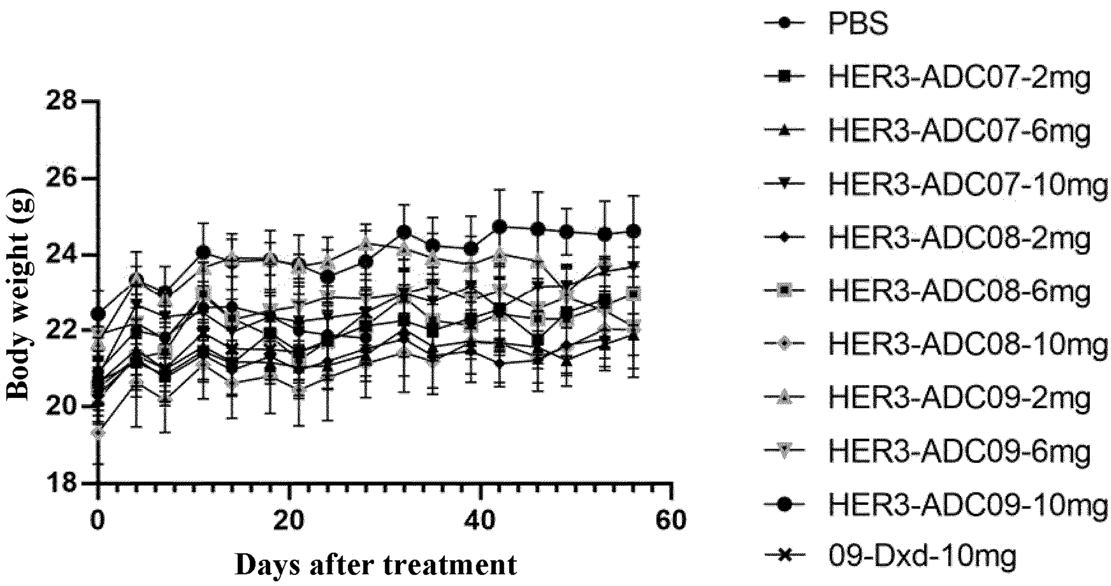
Body weight (g)
Days after treatment
PBS
HER3-ADC07-2mg
HER3-ADC07-6mg
HER3-ADC07-10mg
HER3-ADC08-2mg
HER3-ADC08-6mg
HER3-ADC08-10mg
HER3-ADC09-2mg
HER3-ADC09-6mg
HER3-ADC09-10mg
09-Dxd-10mg

FIG. 7

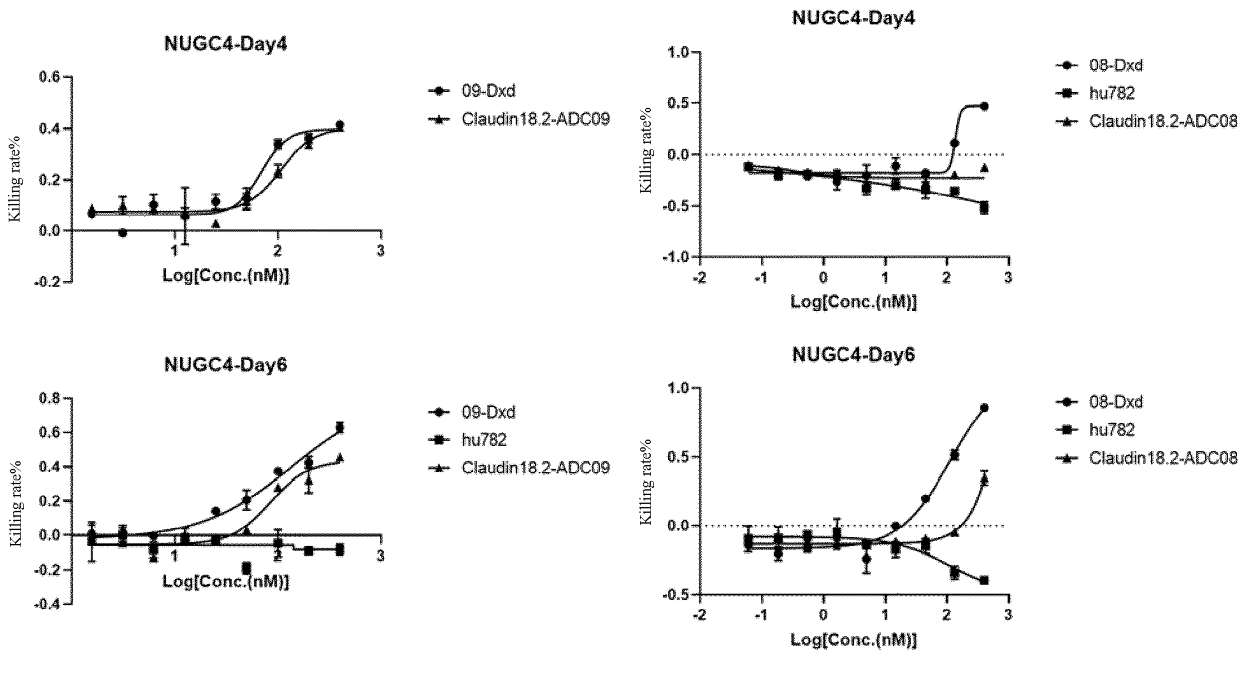
NUGC4-Day4
Killing rate%
Log[Conc.(nM)]
09-Dxd
Claudin18.2-ADC09
NUGC4-Day4
08-Dxd
hu782
Claudin18.2-ADC08
NUGC4-Day6
09-Dxd
hu782
Claudin18.2-ADC09
NUGC4-Day6
08-Dxd
hu782
Claudin18.2-ADC08

FIG. 8

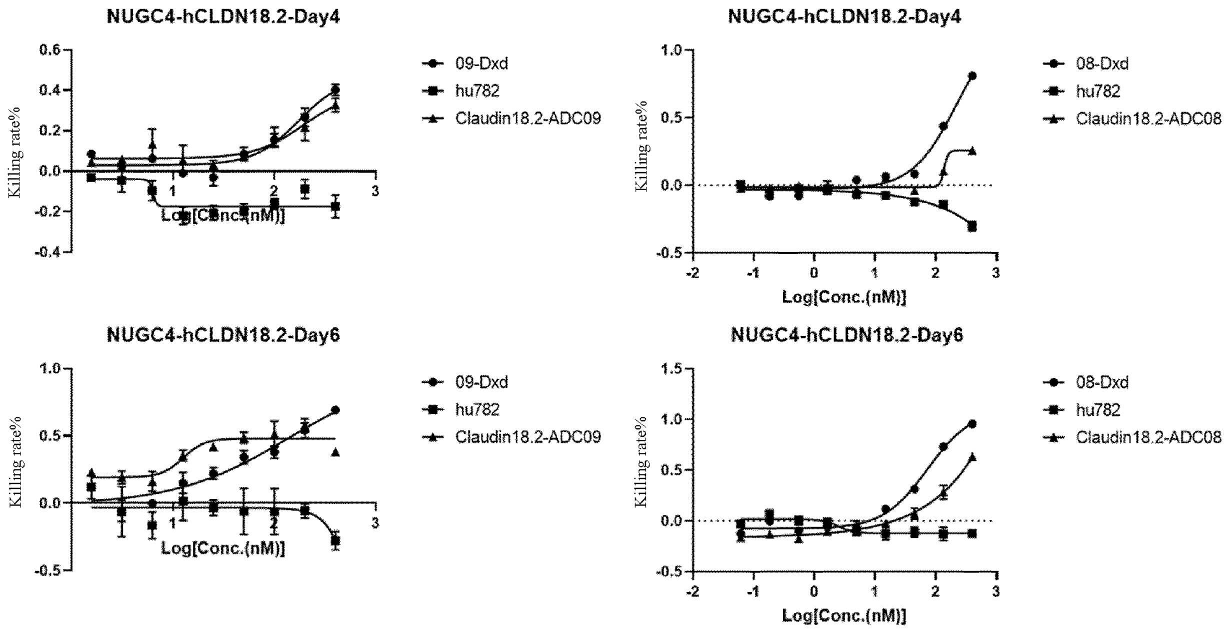
NUGC4-hCLDN18.2-Day4
Killing rate%
Log[Conc.(nM)]
09-Dxd
hu782
Claudin18.2-ADC09
NUGC4-hCLDN18.2-Day4
Killing rate%
Log[Conc.(nM)]
08-Dxd
hu782
Claudin18.2-ADC08
NUGC4-hCLDN18.2-Day6
Killing rate%
Log[Conc.(nM)]
09-Dxd
hu782
Claudin18.2-ADC09
NUGC4-hCLDN18.2-Day6
Killing rate%
Log[Conc.(nM)]
08-Dxd
hu782
Claudin18.2-ADC08

FIG. 9

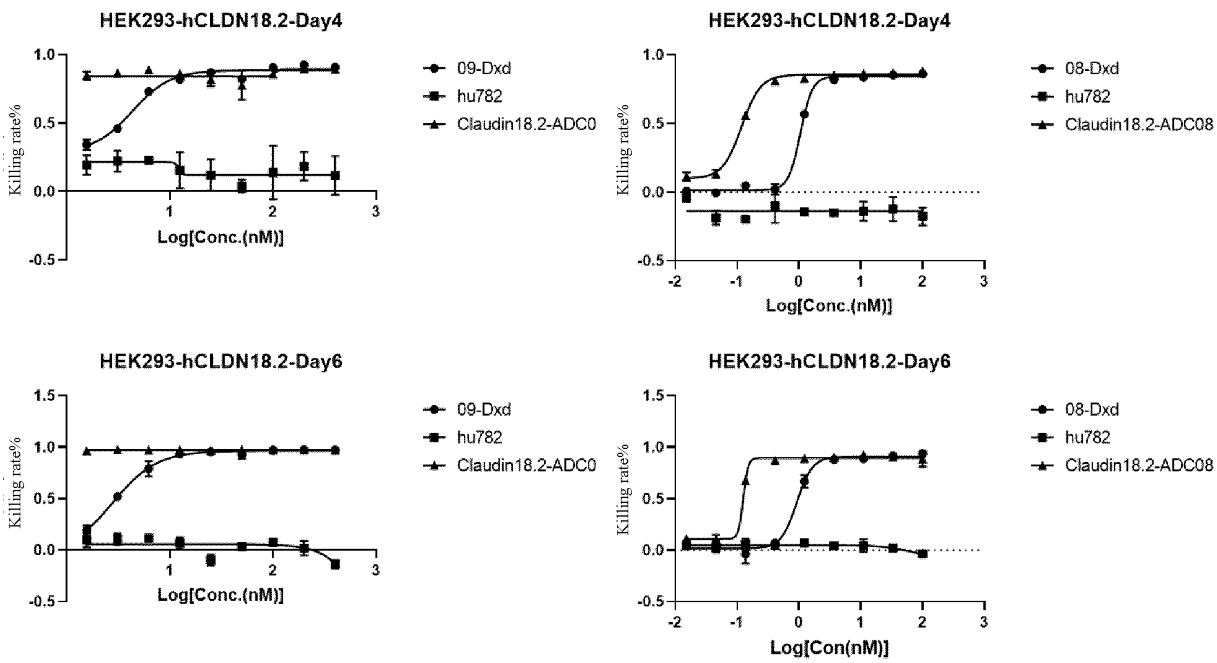
HEK293-hCLDN18.2-Day4
Killing rate%
Log[Conc.(nM)]
09-Dxd
hu782
Claudin18.2-ADC0
HEK293-hCLDN18.2-Day4
Killing rate%
Log[Conc.(nM)]
08-Dxd
hu782
Claudin18.2-ADC08
HEK293-hCLDN18.2-Day6
Killing rate%
Log[Conc.(nM)]
09-Dxd
hu782
Claudin18.2-ADC0
HEK293-hCLDN18.2-Day6
Killing rate%
Log[Con(nM)]
08-Dxd
hu782
Claudin18.2-ADC08

FIG. 10

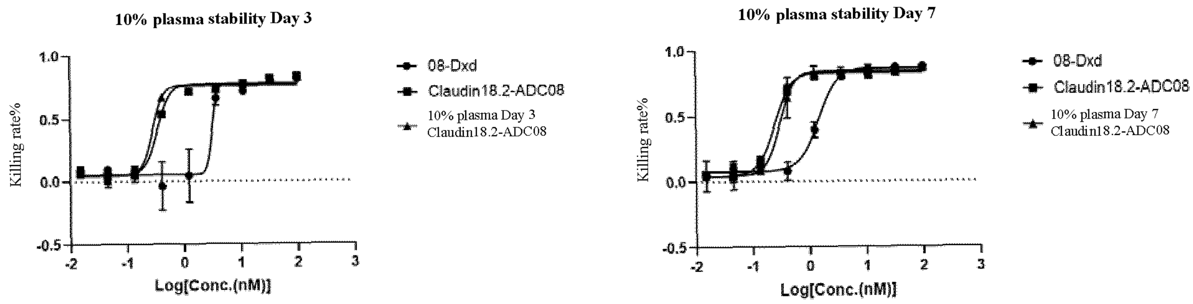
10% plasma stability Day 3
Killing rate%
1.0
0.5
0.0
-0.5
-2
-1
0
1
2
3
Log[Conc.(nM)]
08-Dxd
Claudin18.2-ADC08
10% plasma Day 3 Claudin18.2-ADC08
10% plasma stability Day 7
Killing rate%
1.0
0.5
0.0
-0.5
-2
-1
0
1
2
3
Log[Conc.(nM)]
08-Dxd
Claudin18.2-ADC08
10% plasma Day 7 Claudin18.2-ADC08

FIG. 11

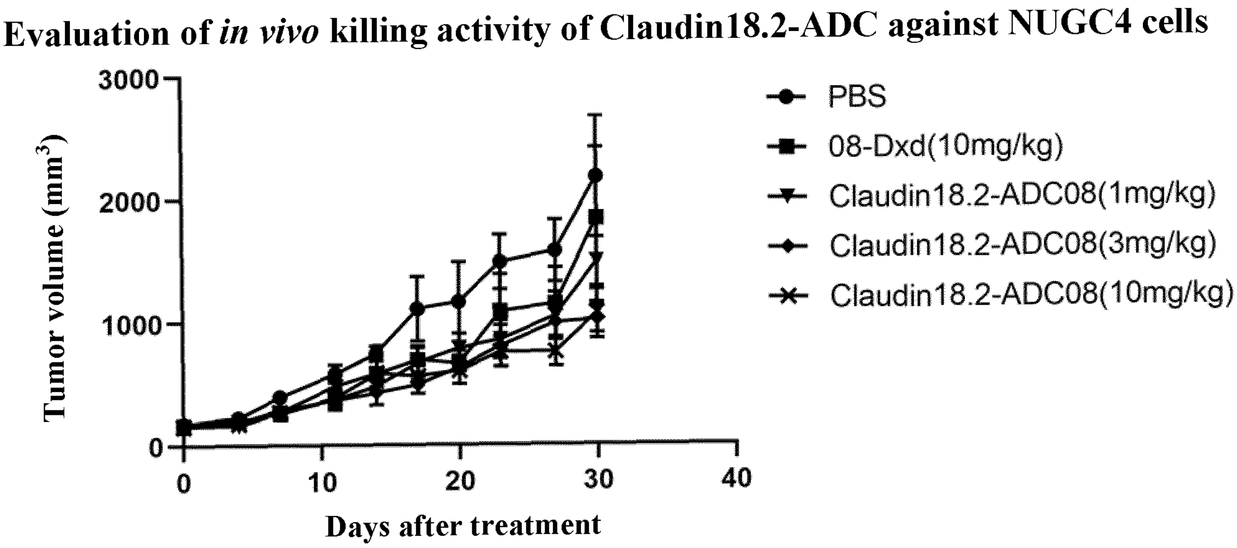
Evaluation of *in vivo* killing activity of Claudin18.2-ADC against NUGC4 cells
Tumor volume (mm³)
3000
2000
1000
0
0
10
20
30
40
Days after treatment
PBS
08-Dxd(10mg/kg)
Claudin18.2-ADC08(1mg/kg)
Claudin18.2-ADC08(3mg/kg)
Claudin18.2-ADC08(10mg/kg)

FIG. 12

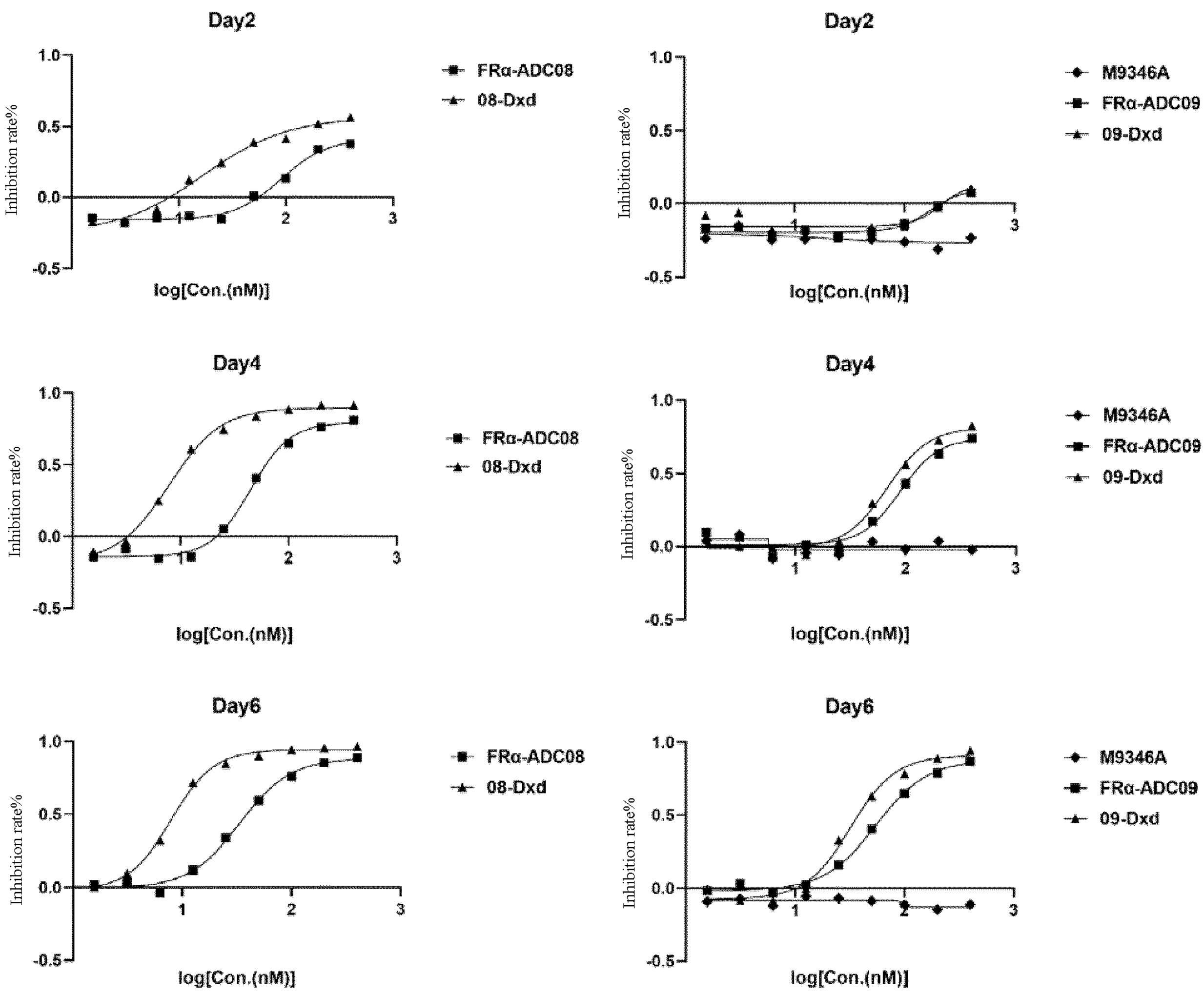
Day2
Inhibition rate%
log[Con.(nM)]
FRα-ADC08
08-Dxd
Day2
M9346A
FRα-ADC09
09-Dxd
Day4
Day6

FIG. 13

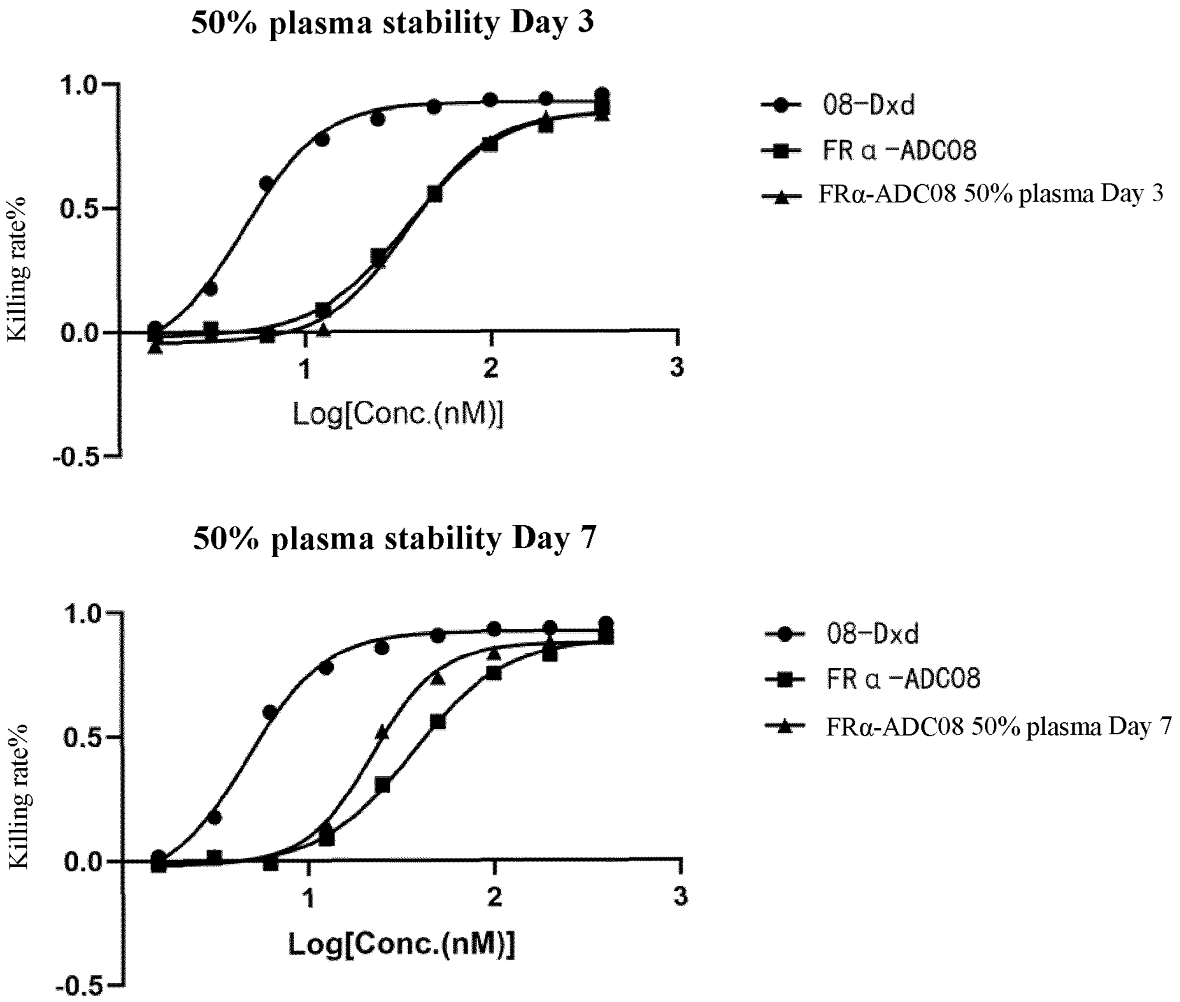
50% plasma stability Day 3
Killing rate%
1.0
0.5
0.0
-0.5
1
2
3
Log[Conc.(nM)]
08-Dxd
FRα-ADC08
FRα-ADC08 50% plasma Day 3
50% plasma stability Day 7
Killing rate%
1.0
0.5
0.0
-0.5
1
2
3
Log[Conc.(nM)]
08-Dxd
FRα-ADC08
FRα-ADC08 50% plasma Day 7

FIG. 14

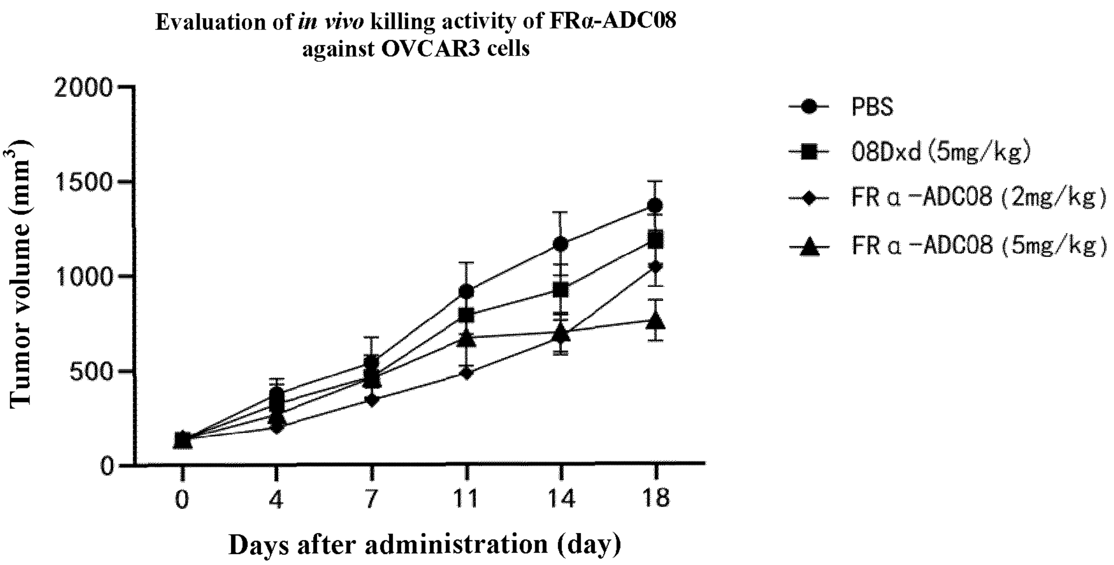
Evaluation of *in vivo* killing activity of FRα-ADC08 against OVCAR3 cells
Tumor volume (mm³)
2000
1500
1000
500
0
0
4
7
11
14
18
Days after administration (day)
PBS
08Dxd (5mg/kg)
FRα-ADC08 (2mg/kg)
FRα-ADC08 (5mg/kg)

FIG. 15

## INTERNATIONAL SEARCH REPORT

International application No.
**PCT/CN2022/135216**

**A. CLASSIFICATION OF SUBJECT MATTER**

C07K7/02(2006.01)i;A61K47/68(2017.01)i;A61P35/00(2006.01)i

According to International Patent Classification (IPC) or to both national classification and IPC

**B. FIELDS SEARCHED**

Minimum documentation searched (classification system followed by classification symbols)

C07K，A61K，A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pudmed, STN on web: 上海生物制品研究所有限责任公司, 瞿爱东, Val-Ala-Gly-Phe-Gly, Val-Ala-Ser-Gly-Phe-Gly, Val-Ala-Ser-Ser-Gly-Phe-Gly, VAGFG, VASGFG, VASSGFG, 组织蛋白酶B, cathepsin B, 偶联物, conjugate, peptide linker

**C. DOCUMENTS CONSIDERED TO BE RELEVANT**

| Category* | Citation of document, with indication, where appropriate, of the relevant passages | Relevant to claim No. |
|---|---|---|
| X | WO 2021148003 A1 (SHANGHAI SENHUI MEDICINE CO., LTD. et al.) 29 July 2021 (2021-07-29)<br>page 4, paragraphs 1 and 2, page 6, paragraph 5-page 8, last paragraph, pages 14-15, and page 18, last paragraph | 1-57 |
| A | CN 112604004 A (INSTITUTE OF MEDICINAL BIOTECHNOLOGY, CHINESE ACADEMY OF MEDICAL SCIENCES) 06 April 2021 (2021-04-06)<br>claims 1-10 | 1-57 |
| A | WO 2017151979 A1 (EISAI INC. et al.) 08 September 2017 (2017-09-08)<br>entire document | 1-57 |
| A | WO 2019096867 A1 (DEBIOPHARM RESEARCH & MANUFACTURING S.A.) 23 May 2019 (2019-05-23)<br>entire document | 1-57 |
| A | WO 2019231879 A1 (BRISTOL-MYERS SQUIBB COMPANY) 05 December 2019 (2019-12-05)<br>entire document | 1-57 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 February 2023** | **15 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/135216**

**C. DOCUMENTS CONSIDERED TO BE RELEVANT**

| Category* | Citation of document, with indication, where appropriate, of the relevant passages | Relevant to claim No. |
|---|---|---|
| A | TSUCHIKAMA, K. et al. "Antibody-drug conjugates: recent advances in conjugation and linker chemistries." *Protein Cell.*, Vol. 9, No. 1, 31 December 2016 (2016-12-31), page 41, right-hand column, and paragraph 1 | 1-57 |

Form PCT/ISA/210 (second sheet) (July 2022)

**INTERNATIONAL SEARCH REPORT**

International application No.
**PCT/CN2022/135216**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2022/135216**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **56-57**
because they relate to subject matter not required to be searched by this Authority, namely:

Claims 56-57 relate to a method for treating a disease or condition of a subject in need, and belong to a method for treatment of a disease defined in PCT Rule 39.1(iv).; however, a search was still conducted on the basis of a pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/135216**

| Patent document cited in search report | Publication date (day/month/year) | Patent family member(s) | Publication date (day/month/year) |
|---|---|---|---|
| WO 2021148003 A1 | 29 July 2021 | AU 2021210079 A1 | 25 August 2022 |
| | | BR 112022014398 A2 | 13 September 2022 |
| | | EP 4094779 A1 | 30 November 2022 |
| | | TW 202140078 A | 01 November 2021 |
| | | KR 20220130160 A | 26 September 2022 |
| | | CA 3168654 A1 | 29 July 2021 |
| CN 112604004 A | 06 April 2021 | None | |
| WO 2017151979 A1 | 08 September 2017 | UA 125024 C2 | 29 December 2021 |
| | | JP 2020019787 A | 06 February 2020 |
| | | JP 6870051 B2 | 12 May 2021 |
| | | CA 3013791 A1 | 08 September 2017 |
| | | US 2017252458 A1 | 07 September 2017 |
| | | US 10548986 B2 | 04 February 2020 |
| | | EP 3824909 A1 | 26 May 2021 |
| | | US 2020297860 A1 | 24 September 2020 |
| | | SG 10202007520 WA | 29 September 2020 |
| | | AR 121302 A2 | 04 May 2022 |
| | | LT 3423105 T | 10 September 2021 |
| | | IL 261428 A | 31 October 2018 |
| | | CO 2018008667 A2 | 31 August 2018 |
| | | SG 11201806515 RA | 27 September 2018 |
| | | KR 20220101204 A | 19 July 2022 |
| | | BR 112018067379 A2 | 15 January 2019 |
| | | RS 62108 B1 | 31 August 2021 |
| | | HUE 054726 T2 | 28 September 2021 |
| | | KR 20180115330 A | 22 October 2018 |
| | | KR 102445255 B1 | 22 September 2022 |
| | | TW 201800110 A | 01 January 2018 |
| | | TWI 772288 B | 01 August 2022 |
| | | JP 2020128413 A | 27 August 2020 |
| | | CL 2021000048 A1 | 28 May 2021 |
| | | MD 3423105 T2 | 31 October 2021 |
| | | ES 2880402 T3 | 24 November 2021 |
| | | HRP 20211125 T1 | 15 October 2021 |
| | | PL 3423105 T3 | 25 October 2021 |
| | | JP 2019516664 A | 20 June 2019 |
| | | JP 6599019 B2 | 30 October 2019 |
| | | EP 3423105 A1 | 09 January 2019 |
| | | EP 3423105 B1 | 05 May 2021 |
| | | IL 292946 A | 01 July 2022 |
| | | US 2018193478 A1 | 12 July 2018 |
| | | US 10322192 B2 | 18 June 2019 |
| | | AR 107787 A1 | 06 June 2018 |
| | | SI 3423105 T1 | 31 December 2021 |
| | | PE 20181953 A1 | 17 December 2018 |
| | | PH 12018501847 A1 | 15 May 2019 |
| | | AU 2017225982 A1 | 16 August 2018 |
| | | AR 121301 A2 | 04 May 2022 |
| | | MX 2018010562 A | 20 February 2019 |
| | | DK 3423105 T3 | 26 July 2021 |

Form PCT/ISA/210 (patent family annex) (July 2022)

**INTERNATIONAL SEARCH REPORT**
**Information on patent family members**

International application No.
**PCT/CN2022/135216**

| Patent document cited in search report | Publication date (day/month/year) | Patent family member(s) | Publication date (day/month/year) |
|---|---|---|---|
| | | KR 20220101203 A | 19 July 2022 |
| | | KR 102456433 B1 | 19 October 2022 |
| | | CL 2018002456 A1 | 21 December 2018 |
| | | RU 2018134331 A | 02 April 2020 |
| | | RU 2018134331 A3 | 14 August 2020 |
| | | RU 2754369 C2 | 01 September 2021 |
| | | PT 3423105 T | 19 July 2021 |
| | | RU 2021125492 A | 05 April 2022 |
| | | MA 45280 A | 09 January 2019 |
| | | MA 45280 B1 | 31 August 2021 |
| | | CL 2021000049 A1 | 28 May 2021 |
| | | JP 2021185176 A | 09 December 2021 |
| WO 2019096867 A1 | 23 May 2019 | AU 2018368520 A1 | 14 May 2020 |
| | | US 2022062371 A1 | 03 March 2022 |
| | | SG 11202003995 PA | 28 May 2020 |
| | | EA 202091203 A1 | 03 August 2020 |
| | | IL 274549 A | 30 June 2020 |
| | | CA 3082271 A1 | 23 May 2019 |
| | | KR 20200088402 A | 22 July 2020 |
| | | BR 112020008974 A2 | 17 November 2020 |
| | | EP 3710064 A1 | 23 September 2020 |
| WO 2019231879 A1 | 05 December 2019 | EA 202092747 A1 | 16 March 2021 |
| | | AU 2019277094 A1 | 21 January 2021 |
| | | IL 278938 A | 31 January 2021 |
| | | JP 2021525724 A | 27 September 2021 |
| | | US 2019365915 A1 | 05 December 2019 |
| | | US 10898578 B2 | 26 January 2021 |
| | | KR 20210015923 A | 10 February 2021 |
| | | SG 11202011739 SA | 30 December 2020 |
| | | US 2021113706 A1 | 22 April 2021 |
| | | BR 112020024022 A2 | 23 February 2021 |
| | | EP 3801629 A1 | 14 April 2021 |
| | | CA 3101601 A1 | 05 December 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MILLER et al.** *Jour. of Immunology,* 2003, vol. 170, 4854-4861
- **WARD et al.** Binding Activities of a Repertoire of Single Immunoglobulin Variable Domains Secreted From Escherichia coli. *Nature,* 1989, vol. 341, 544-546
- **HUSTON et al.** Protein Engineering of Antibody Binding Sites: Recovery of Specific Activity in an Anti-Digoxin Single-Chain Fv Analogue Produced in Escherichia coli. *Proc. Natl. Acad. Sci. USA,* 1988, vol. 85, 5879-5883
- **NGUYEN VK. et al.** *The EMBO Journal,* 2000, vol. 19, 921-930
- **MUYLDERMANS S.** *J Biotechnol.,* 2001, vol. 74, 277-302
- **VANLANDSCHOOT P. et al.** *Antiviral Research,* 2011, vol. 92, 389-407
- **KABAT et al.** Sequences of Proteins of Immunological Interest. National Institutes of Health, 1991
- **A1-LAZIKANI et al.** *J Mol Biol,* 1997, vol. 273, 927-48
- **ENGLISH et al.** *Mol Diagn Ther. [Molecular Diagnosis & Therapy,* 2013, vol. 17, 85-99